# EUROPEAN PATENT APPLICATION

(11) **EP 3 925 667 A1**
(43) Date of publication of application: **22.12.2021**
(21) Application number: 20180647.8
(22) Date of filing: 17.06.2020
(51) Int. Cl.: A61P 31/12, A61K 38/05, A61K 38/06, A61K 38/07, A61P 31/16, C07K 5/072, C07K 5/078, C07K 5/083, C07K 5/09, C07K 5/093, C07K 5/103, C07K 5/11, C07K 5/113

(54) **SELECTIVE TMPRSS2 INHIBITORS AND MEDICAL USE THEREOF**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The invention relates to the compound of general formula (I) as novel inhibitors of TMPRSS2 and their use for the prophylaxis and treatment of influenza virus infection or coronavirus infection, in particular, SARS-CoV2 (COVID-19) infection.

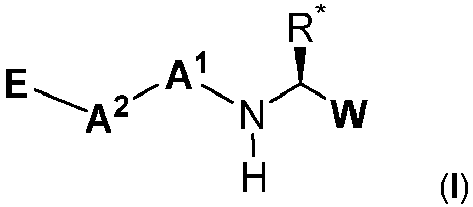

## Description

The invention relates to novel inhibitors of TMPRSS2 and their use for the prophylaxis and treatment of cancer, influenza virus infection or coronavirus infection, in particular, SARS-CoV2 infection.

### Background of the invention

To enter into airway epithelial cells, influenza, parainfluenza and coronaviruses rely on host cell proteases for activation of the viral protein involved in membrane fusion. Transmembrane protease serine 2 (TMPRSS2) was recently proven to be crucial for hemagglutinin cleavage of some human influenza viruses (W-J Shin et al., Expert Opinion on Drug Discovery, 2017, 12(11), 1139-1152). Since the catalytic sites of the diverse serine proteases linked to influenza, parainfluenza and coronavirus activation are structurally similar, active site inhibitors of these airway proteases could have broad therapeutic applicability against multiple respiratory viruses.
A role is now well established for transmembrane protease serine 2 (TMPRSS2), a member of the TTSP family, which are integral membrane proteins with an extracellular C-terminal serine protease domain and an N-terminal cytoplasmic domain.

One unique benefit of blocking TMPRSS2 and related airway proteases is that, besides influenza virus, several other respiratory viruses could be targeted. TMPRSS2 was shown to activate the fusion proteins of some paramyxoviruses (i.e. metapneumovirus, trypsin-dependent parainfluenza subtypes and Sendai virus).
TMPRSS2 is supported by recent proof, from mouse or clinical studies, that this protease is essential for replication of influenza viruses such as H1N1 and H7N9 viruses (Kouji Sakai et al., Journal of Virology, 2014, 88(10), 5608-5616).

In December 2019, Wuhan, in Hubei province, China, became the center of an outbreak of a new coronavirus. This new coronavirus, i.e. SARS-CoV2 causing COVID-19 and previous experiences with SARS and MERS-CoV, highlight the need for therapeutics for human coronavirus infections that can improve clinical outcomes, reduce risk of disease progression, speed recovery, and reduce the requirements for intensive supportive care and prolonged hospitalisation.
Likewise, for some human coronaviruses including the deadly SARS, COVID-19 (SARS-Cov-2) and MERS variants, TMPRSS2 cleavage activates the viral spike (S)-protein at the cell surface enabling cathepsin-independent host cell entry. SARS and COVID-19 uses the SARS-CoV receptor ACE2 for attachement and the serine protease TMPRSS2 for S protein priming and fusion events with the cell membrane. Thus, SARS, MERS and COVID-19 can be inhibited by TMPRSS2 inhibitors.

Recently it was shown that camostat could be used effectively in protecting mice against a lethal infection by SARS coronavirus (Hoffmann et al., Cell, 2020, 181, 1-10). Besides this selectivity issue, the safety of serine protease inhibitors largely depends on whether or not the binding is reversible. The classical inhibitors show covalent binding to the catalytic serine, which is in most cases (pseudo-) irreversible, for example with camostat. Such irreversible protease inhibitors may pose a safety issue, as such non-selective serine protease inhibitors also produce strong inhibition of off-tagets such as the related type II transmembrane serine proteases (TTSPs i) ncluding, thrombin, plasmin and factor Xa.

Since an antiviral medication for these viruses, in particular coronaviruses is currently lacking, it is neceesary to develop the antiviral drugs.

The objective of the present invention is to provide novel and preferably selective inhibitors of TMPRSS2 and methods for the synthesis of said inhibitors as well as their use for the prophylaxis and treatment of viral infections, especially corona virus infections, and in particular COVID-19 (SARS-CoV-2) infections.

Said objective is solved by the technical teachings of the independent claims. Further advantageous embodiments, aspects and details of the invention are evident from the dependent claims, the description, the figures and the examples.

Surprisingly, it has been found that tetrapeptidomimetics having a benzothiazole warhead as disclosed herein inhibit selectively TMPRSS2 over thrombin and coagulation factor Xa and also inhibit effectively the replication of coronavirus, in particular SARS-CoV2.

Thus, the present invention relates to a compound of the general formula (I): 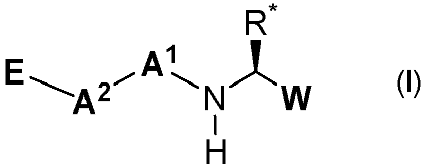 wherein
**W** represents -CN, -B(OH)₂, 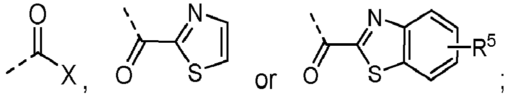
**X** represents -H, -CO-OR⁶, or -CO-NR⁷R⁸;
**R*** represents 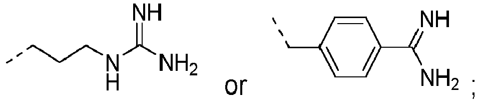
**E** represents -H, -R⁴, -CO**R⁴**, -**A³**-H, -**A³**-R⁴, or -**A³**-CO**R⁴**;
**A¹** represents 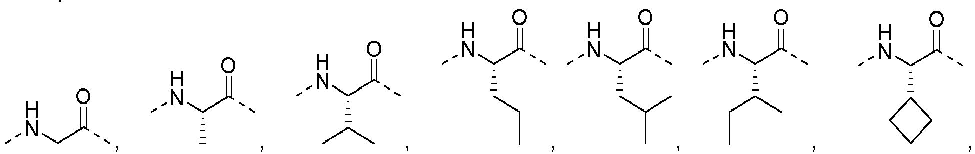 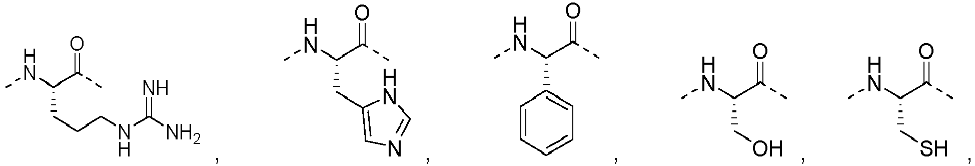 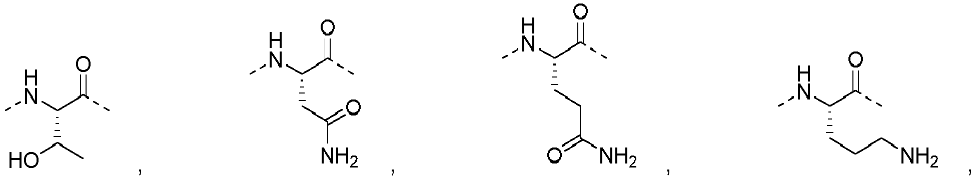 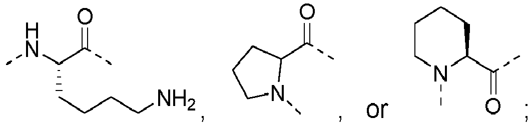
**A²** represents 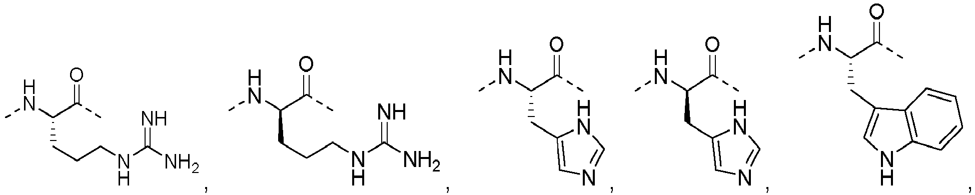 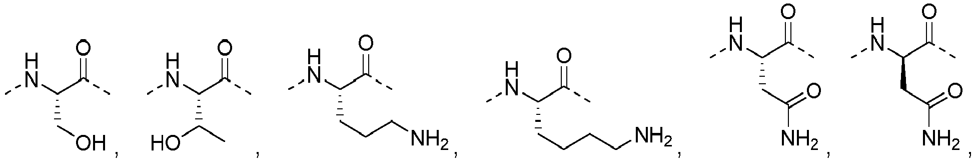 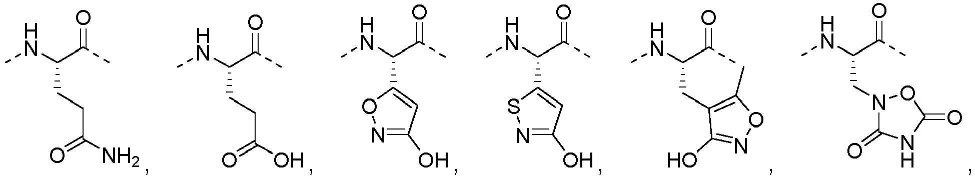 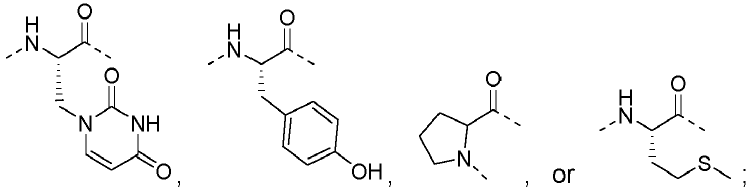
**A³** represents 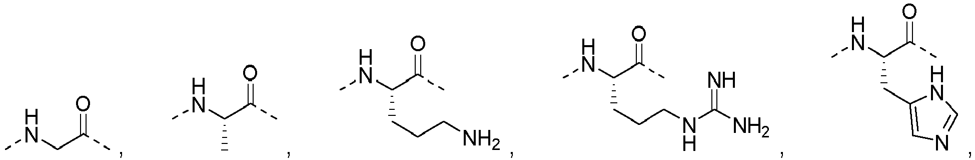 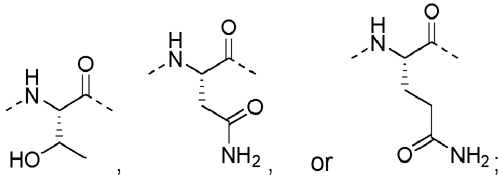
**R⁴** represent -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
**R⁶**, **R⁷**, and **R⁸** represent independently of each other -H, -CH₃, -CH₂CH₃, -CH₂CH₂CH₃, -CH(CH₃)₂, -C(CH₃)₃, or -cyclo-C₃H₅;
or a diastereomer, an enantiomer, a solvate, a hydrate, a prodrug or a pharmaceutically acceptable salt thereof.

Preferably, **W** represent -CHO, -CN, -B(OH)₂,

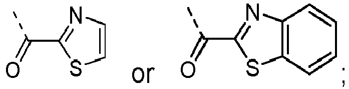

The pharmaceutically acceptable salts of the compounds of the present invention may be formed with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner.
In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Preferred are the following compounds of formula (I-1): 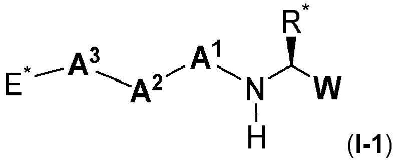
wherein **E*** represents -H, -R⁴, or -CO**R⁴**; and
**W**, R⁴, **R***, **A¹**, **A²**, and **A³** have the same meanings as defined in the formula (I).

Preferred are compounds of the general formula (**II-1**): 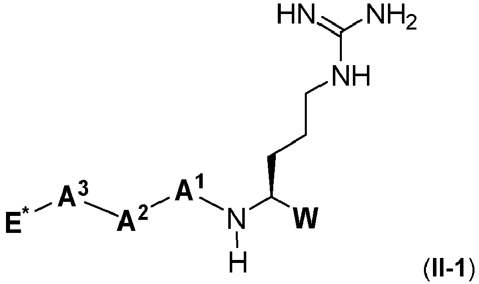 wherein
W represents -CN, -B(OH)₂, 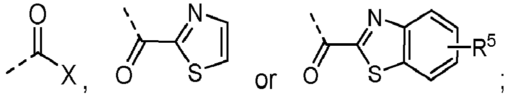
**X** represents -H, -CO-OR⁶, or -CO-NR⁷R⁸;
**E*** represents -H, -R⁴, or -CO**R⁴**;
**A¹** represents 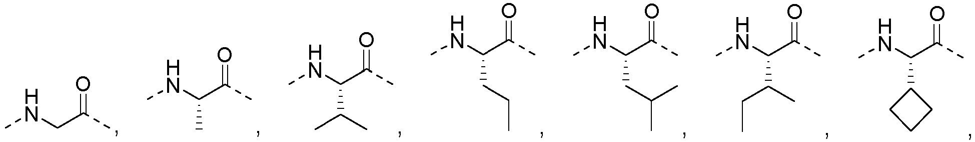 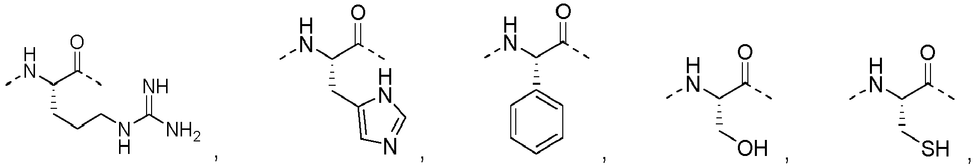 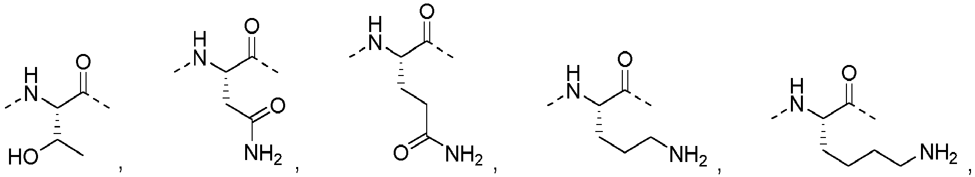 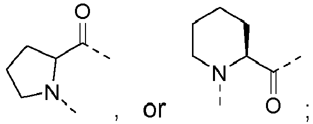
**A²** represents 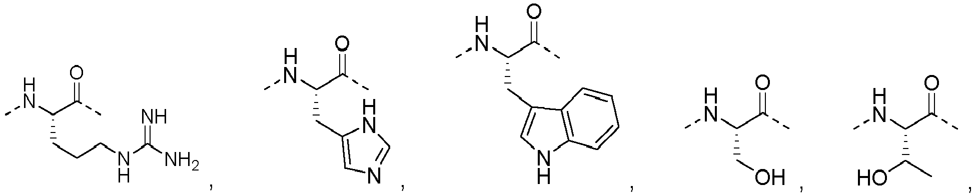 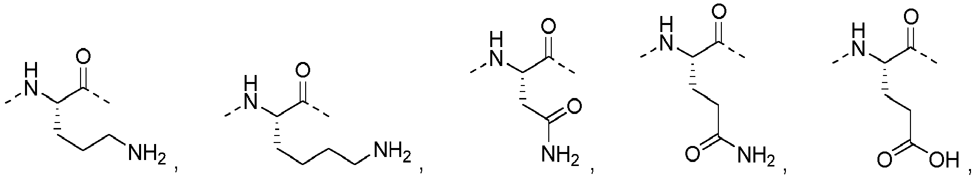 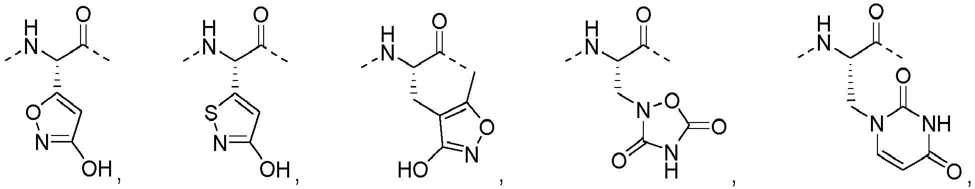 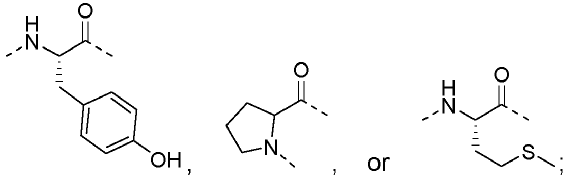
**A³** represents 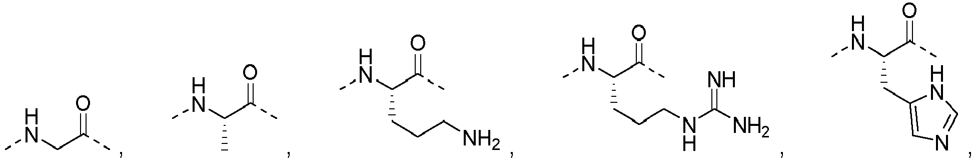 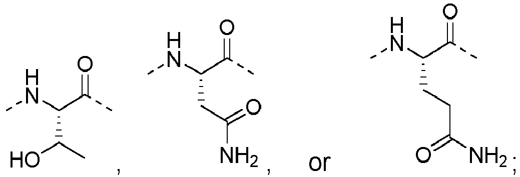
**R⁴** represent -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
**R⁶**, **R⁷**, and **R⁸** represents independently of each other -H, -CH₃, -CH₂CH₃, -CH₂CH₂CH₃, -CH(CH₃)₂, -C(CH₃)₃, or -cyclo-C₃H₅;
or a diastereomer, an enantiomer, a solvate, a hydrate, a prodrug or a pharmaceutically acceptable salt thereof.

More preferred are compound of the general formula (**II-1**): 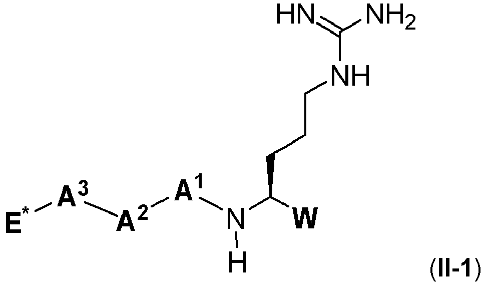 wherein
**A¹** represents 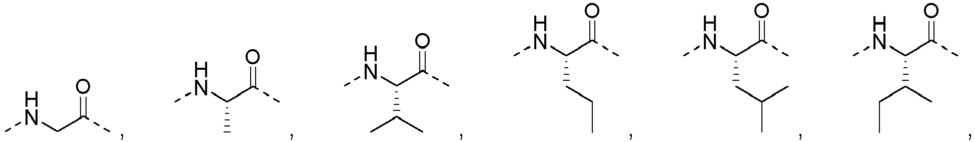 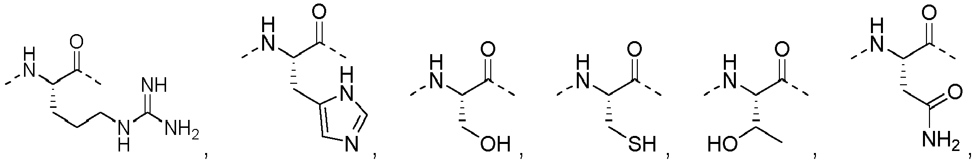 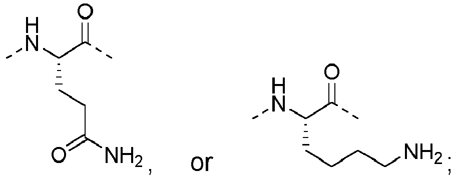
**A²** represents 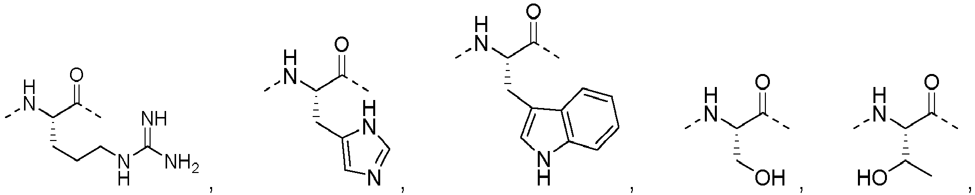 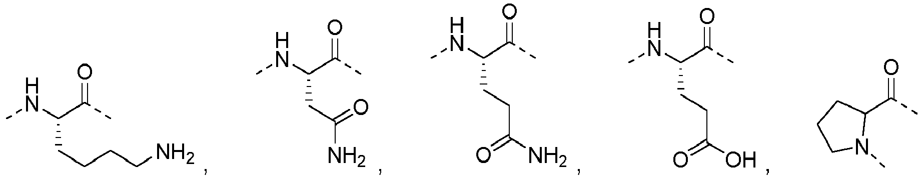 or 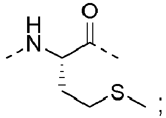
**A³** represents 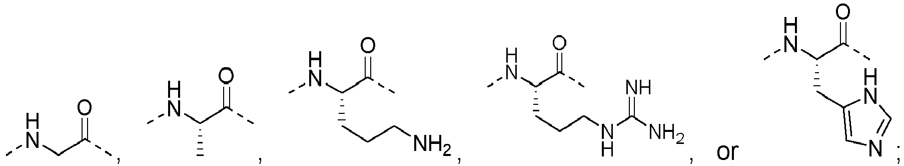
**E*** represents -H, -R⁴, or -CO**R⁴**;
wherein **W** has the same meanings as defined in the formula (I);
or a diastereomer, an enantiomer, a solvate, a hydrate, a prodrug or a pharmaceutically acceptable salt thereof.

Also preferred are compounds of the formula (**II-2a**) or (**II-2b**): 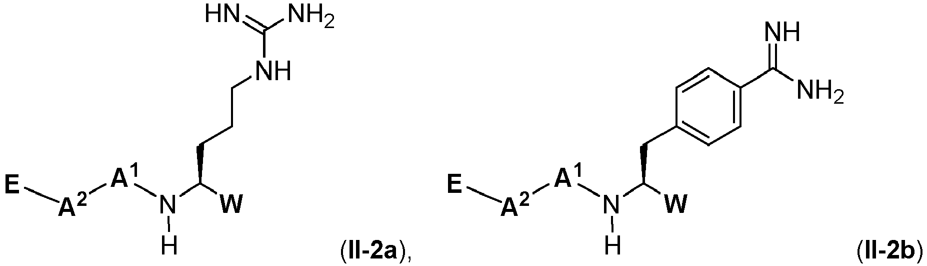 wherein
**W** represents -CN, -B(OH)₂, 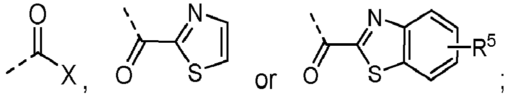
**X** represents -H, -CO-OR⁶, or -CO-NR⁷R⁸;
**E** represents -H, -R⁴, -CO**R⁴**, -**A³**-H, -**A³**-R⁴, or -**A³**-CO**R⁴**; and preferably -H, -R⁴, or -CO**R⁴**;
**A¹** represents 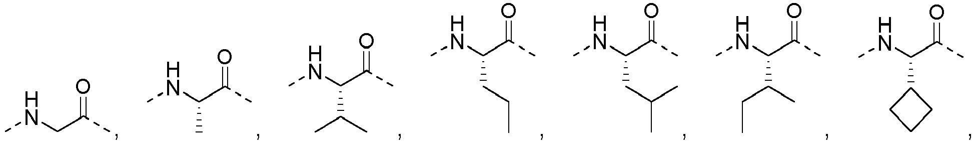 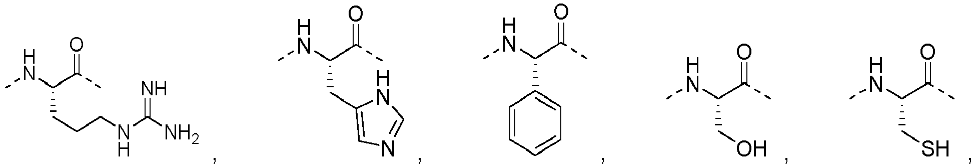 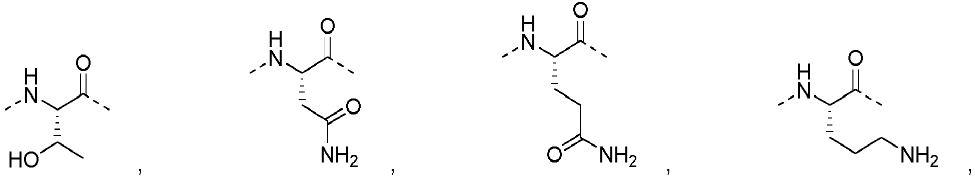 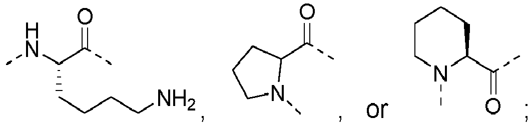
**A²** represents 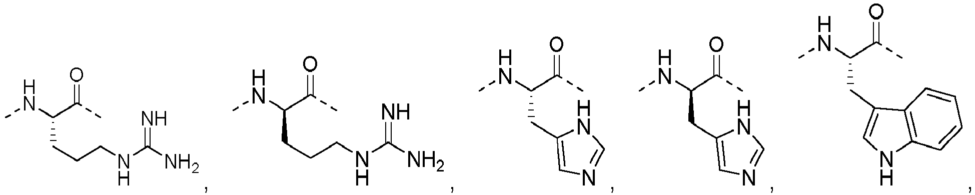 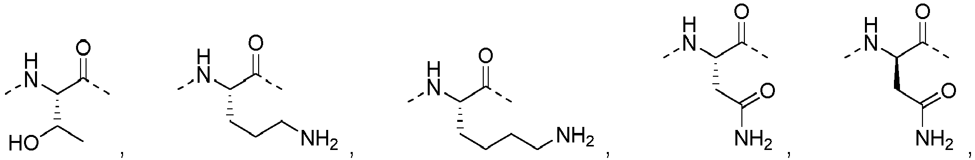 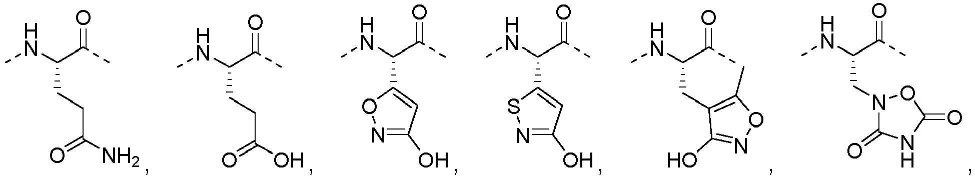 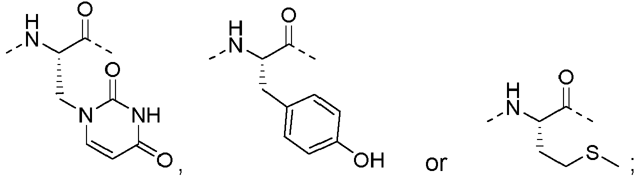
**R⁴** represent -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
**R⁶**, **R⁷**, and **R⁸** represents independently -H, -CH₃, -CH₂CH₃, -CH₂CH₂CH₃, -CH(CH₃)₂, -C(CH₃)₃, or -cyclo-C₃H₅;
or a diastereomer, an enantiomer, a solvate, a hydrate, a prodrug or a pharmaceutically acceptable salt thereof.

More preferred, the compound has the formula (**II-2a**) or (**II-2b**): 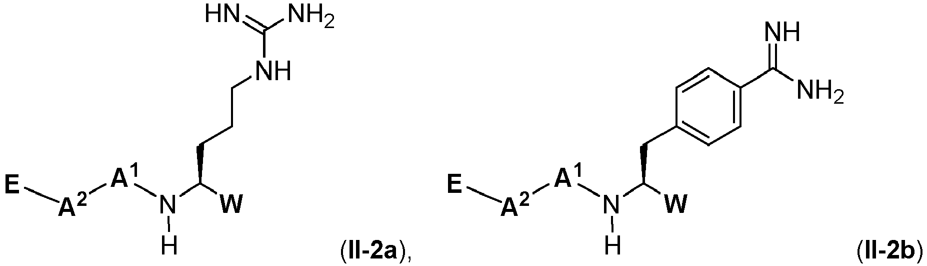 wherein
**A¹** represents 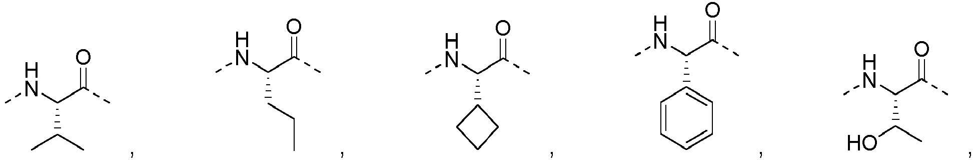 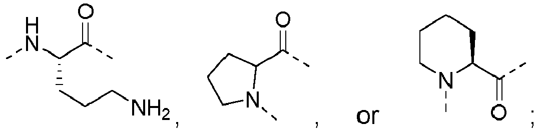
**A²** represents 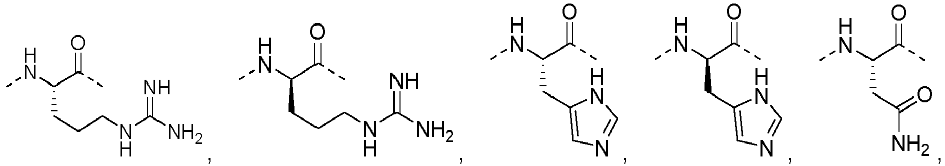 or 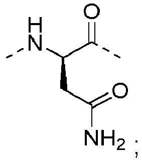
**E** represents -H, -R⁴, -CO**R⁴**, -**A³**-H, -**A³**-R⁴, or -**A³**-CO**R⁴**; and preferably -H, -R⁴, or -CO**R⁴**;
R⁴ and **W** have the same meanings as defined in the formula (I),
or a diastereomer, an enantiomer, a solvate, a hydrate, a prodrug or a pharmaceutically acceptable salt thereof.

Also preferred are the compounds of the formulae (**III-1**) - (**III-5**): 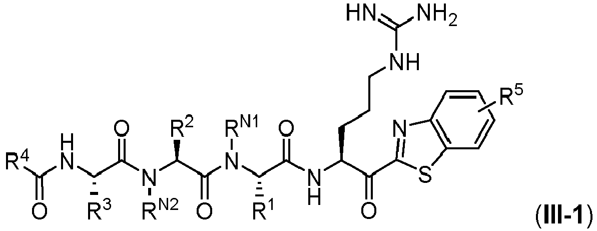 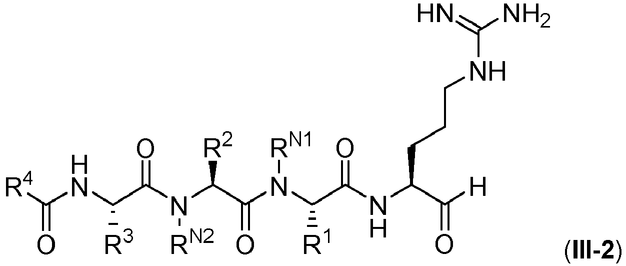 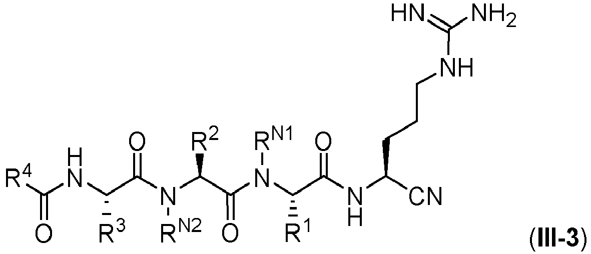 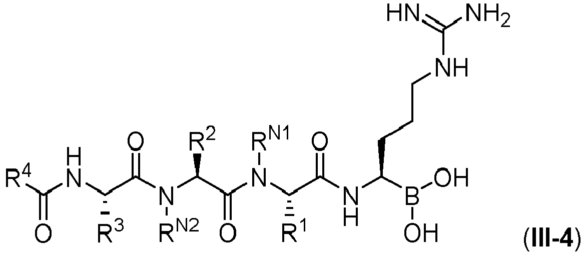 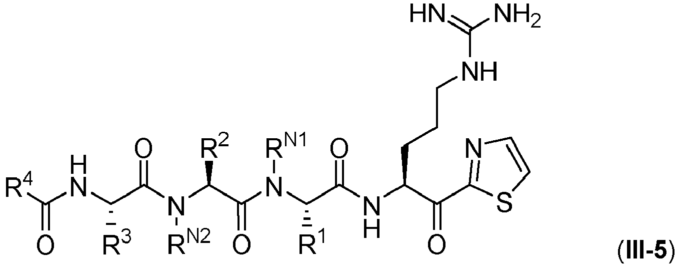 wherein
**R^{N1}** represents -H, or **R¹** and **R^{N1}** form -C₂H₄- (i.e. 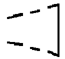 );
**R^{N2}** represents -H, or **R²** and **R^{N2}** form -C₂H₄- (i.e. 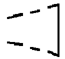 );
**R¹** represents 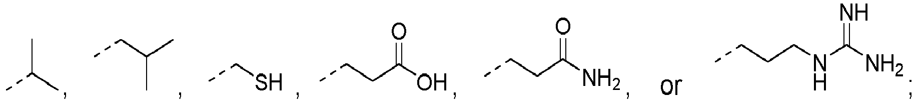
**R²** represents 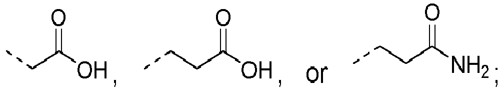
**R³** represents -H, -CH₃, 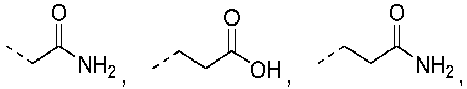 or 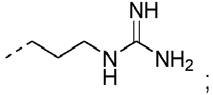
**R⁴** represents -CH₃, -CH₂CH₃, -CH₂CH₂CH₃, -CH(CH₃)₂, -O-C(CH₃)₃, or -cyclo-C₃H₅;
**R⁵** represents -H, -CO₂H, or -CONH₂;
or a diastereomer, an enantiomer, a solvate, a hydrate, a prodrug or a pharmaceutically acceptable salt thereof.

Moreover, in all general formulae disclosed herein it is preferred if **A¹** is a cyclic amino acid residue such as 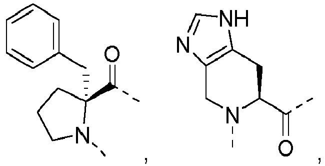 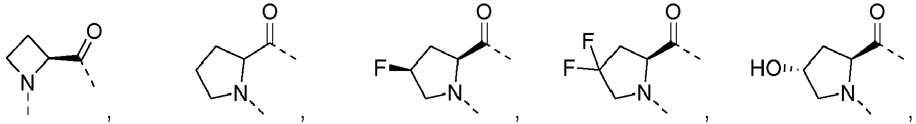 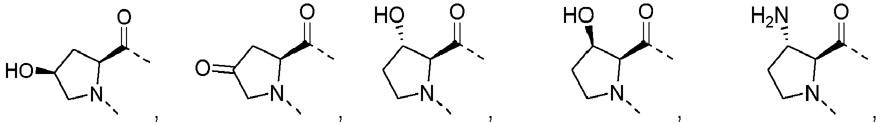 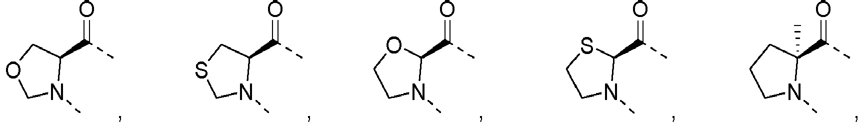 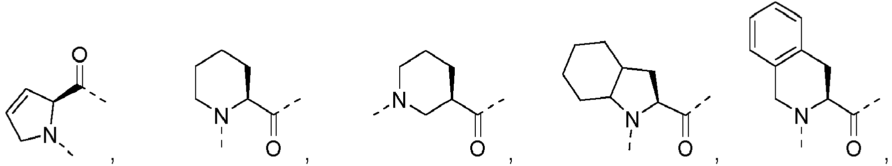 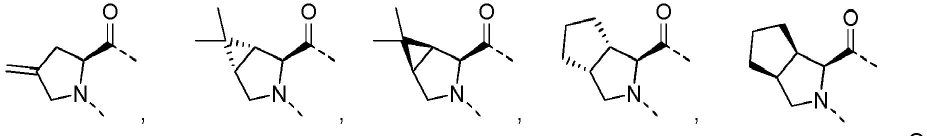 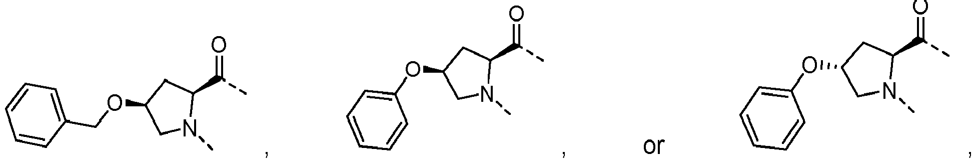 preferably 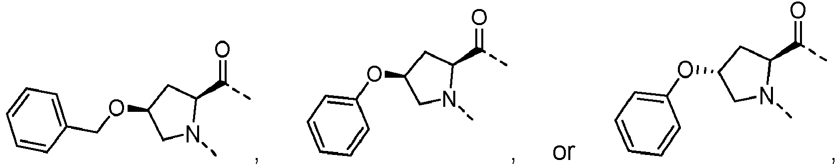 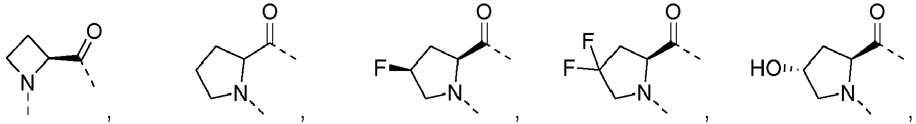 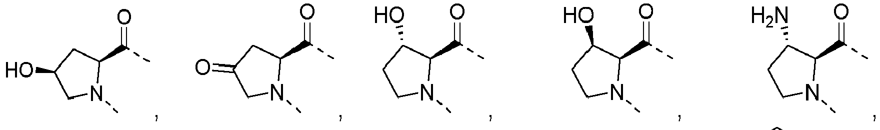 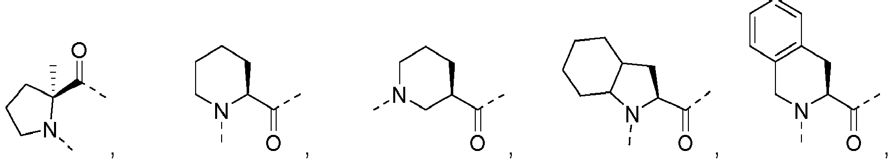 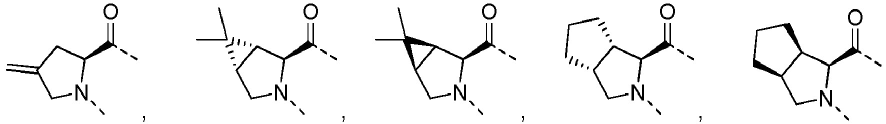 or contains a cyclic moiety such as in the amino acid residues 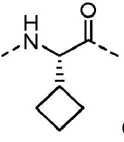 or 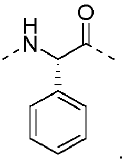 .

Moreover, in all general formulae disclosed herein it is preferred if the cyclic amino acid residue **A¹** or the cyclus-containing amino acid residue **A¹** as disclosed directly above is connected to an amino acid residue **A²** selected from the group consisting of: 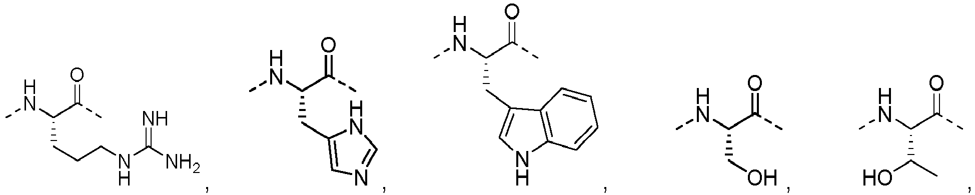 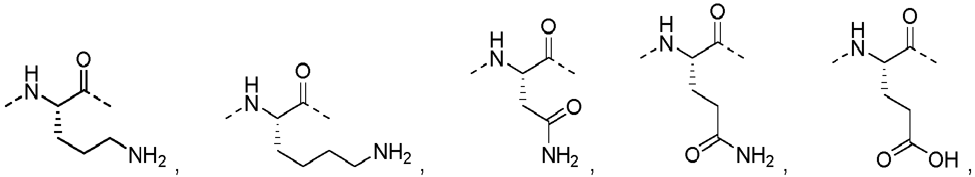 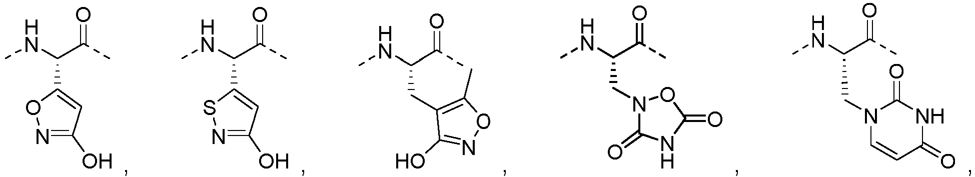 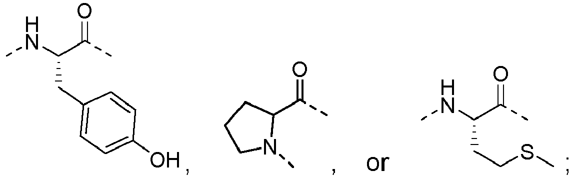 preferably from the group consisting of: 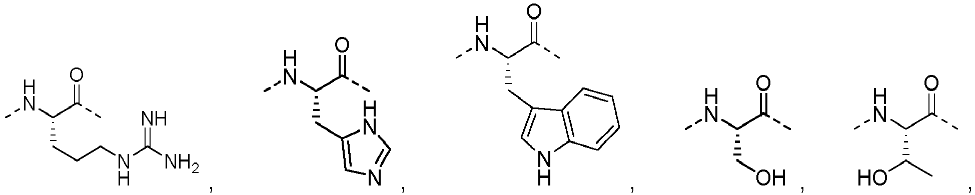 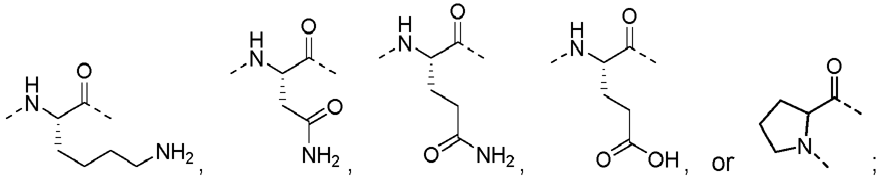 more preferably from the group consisting of:

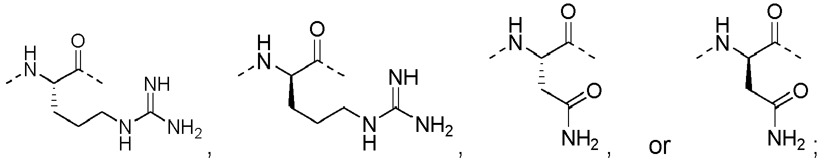

Moreover, it is preferred if **A²** or **A³** represent 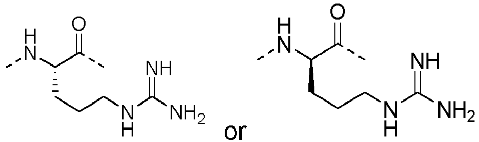 and more preferably 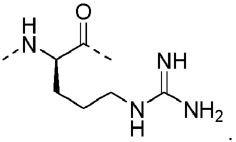 Still more preferred is if **A²** represent 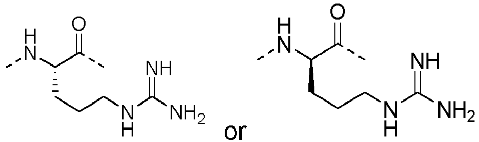 and more preferably

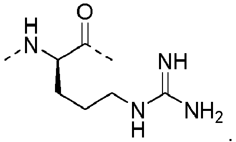

Especially preferred is if **A²** represent 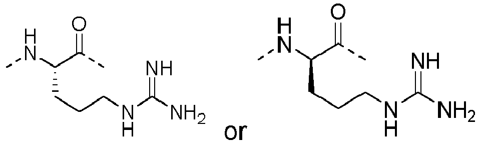 and more preferably 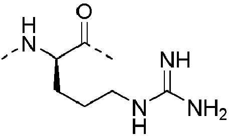 and **A¹** represents one of the cyclic amino acid residues or the cyclus-containing amino acid residues as directly disclosed above.

Thus, a very preferred diamino acid residue **-A²-A¹-** is

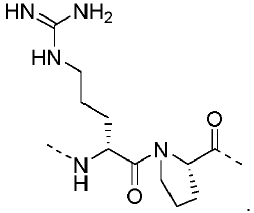

The term "prodrug" describes compounds according to the present invention, wherein the compounds comprises at least one carboxylate group which is modified with a rest that is generally known by a person skilled in the art in that way that the carboxylate group of the compound is released under physiological conditions and/or at least one modified hydroxyl group which is modified with a rest that is generally known by a person skilled in the art in that way that the hydroxyl group of the inventive compond is released under physiological conditions, The prodrug includes also amidoxime group of arginine and benzamidine of the compound as follows:

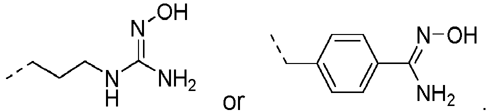

Suprisingly, it was found that the inventive compounds bound to TMPRSS2 reversibly and inhibit TMPRSS2 effectively. The electrophilic warheads can react with highly nucleophilic thiols in the active site of the TMPRSS2. Therefore, it was found that potential unspecific reactions with off-targets such as the related TTSPs including thrombin, plasmin and factor Xa are reduced. It would be expected that the inventive compounds as reversible TMPRSS2 inhibitor may be less toxic than the non selective transmembrane serine protease inhibitors.

Furthermore, the compounds of the present invention also inhibit the replication of coronavirus effectively and thus are useful for the prophylaxis and treatment of virus infection, preferred, wherein virus is influenza virus, parainfluenza virus or coronavirus, in particular SARS-CoV2 infection.

Surprisingly, it has been found that tetrapeptidomimetics having a benzothiazole warhead as disclosed herein inhibit selectively TMPRSS2 over thrombin, and coagulation factor Xa and also inhibit effectively virus infections, preferred influenza virus infection, parainfluenza virus infection, coronavirus infection, in particular COVID-19.

More preferred are the compounds of the formulae (**IV-1a**) - (**IV-1f**): 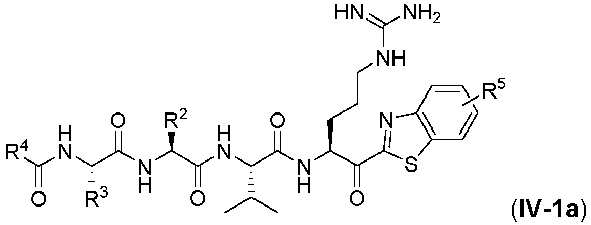 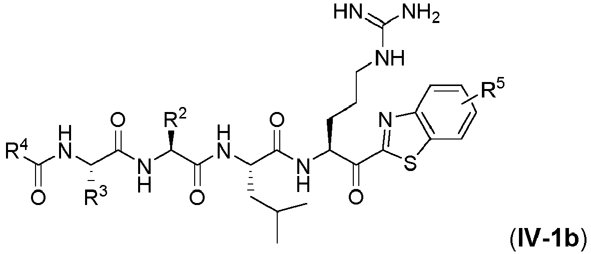 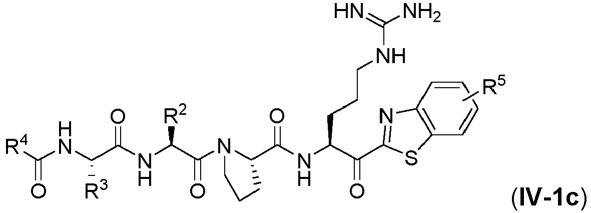 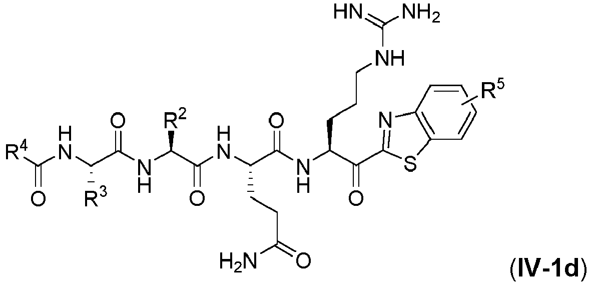 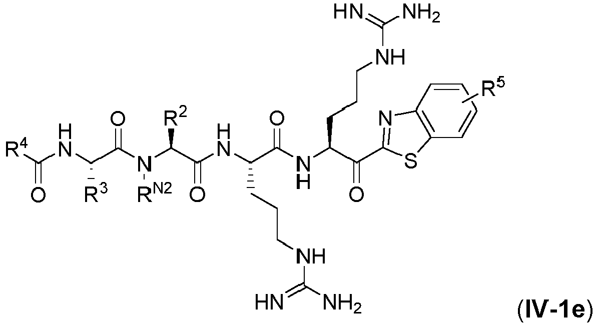 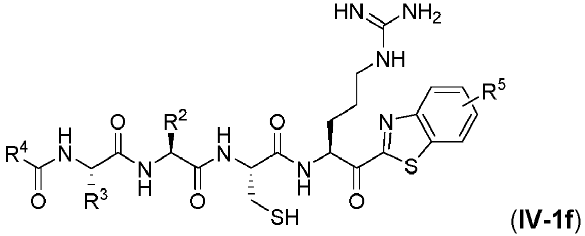 wherein **R²** - **R⁵** and **R^{N2}** have the same meansings as defined in the formula (**III-1**).

More preferred are the compounds of the formulae (**IV-2a**) - (**IV-2f**)**:** 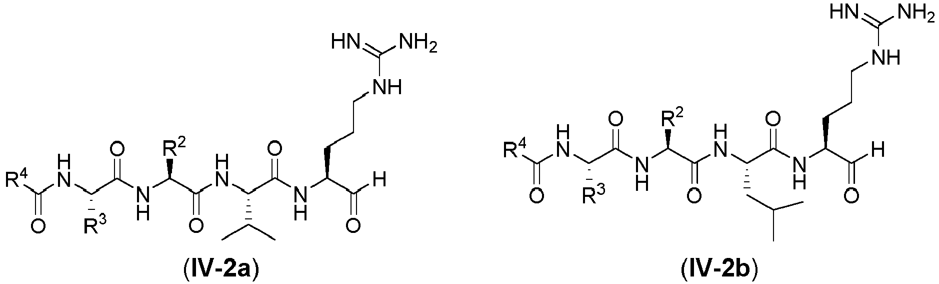 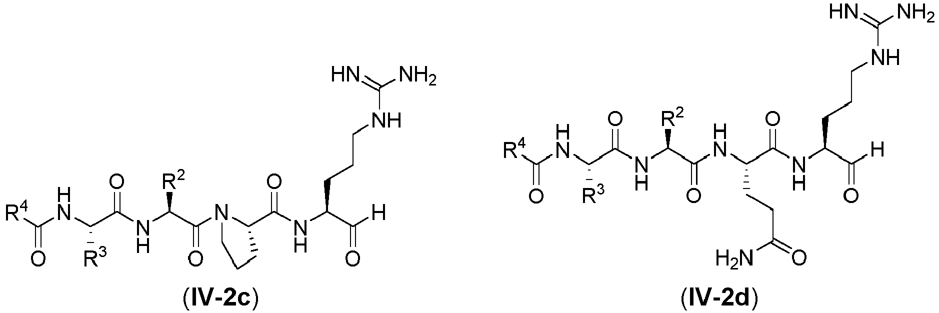 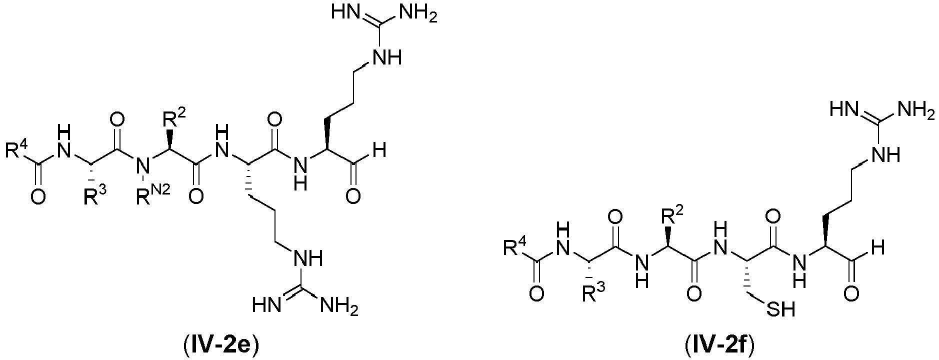 wherein **R²** - **R⁴** and **R^{N2}** have the same meansings as defined in the formula (**III-2**).

More preferred are the compounds of the formulae (**IV-3a**) - (**IV-3f**): 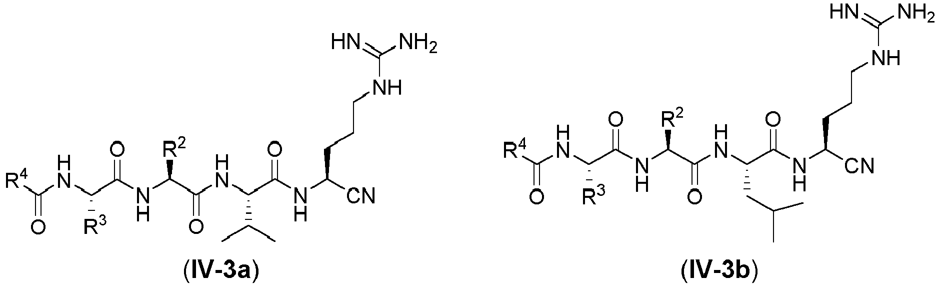 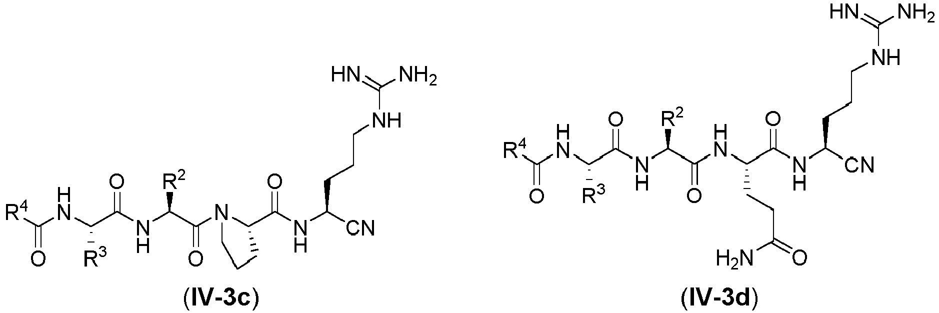 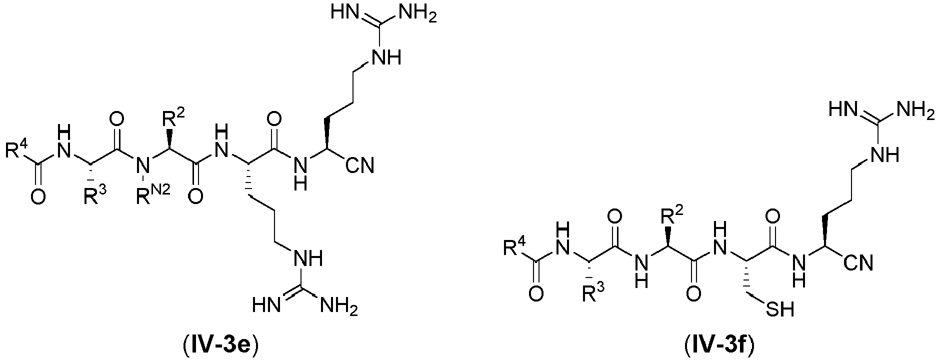 wherein **R²** - **R⁴** and **R^{N2}** have the same meansings as defined in the formula (**III-3**).

More preferred are the compounds of the formulae (**IV-4a**) - (**IV-4f**)**:** 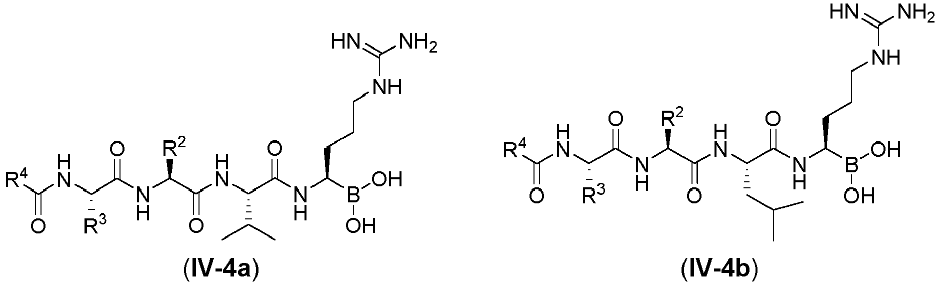 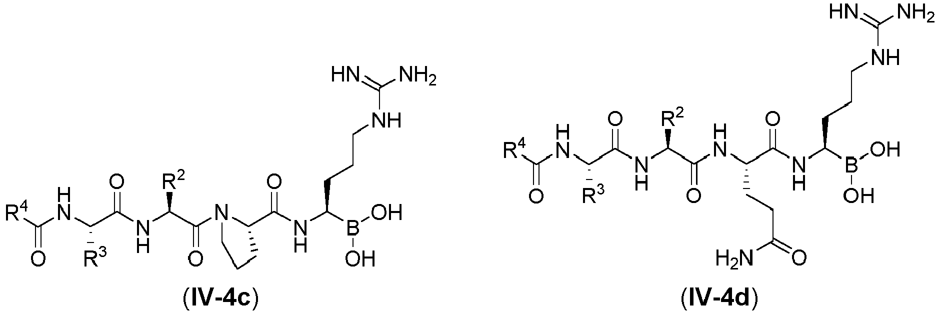 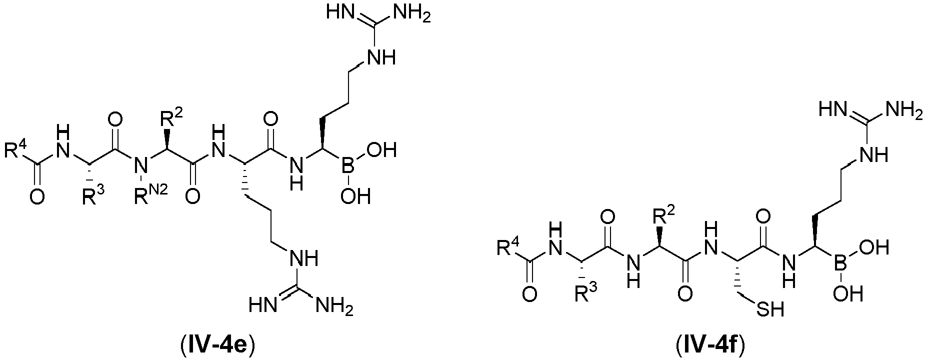 wherein **R²** - **R⁴** and **R^{N2}** have the same meansings as defined in the formula (**III-4**).

More preferred are the compounds of the formulae (**IV-5a**) - (**IV-5f**): 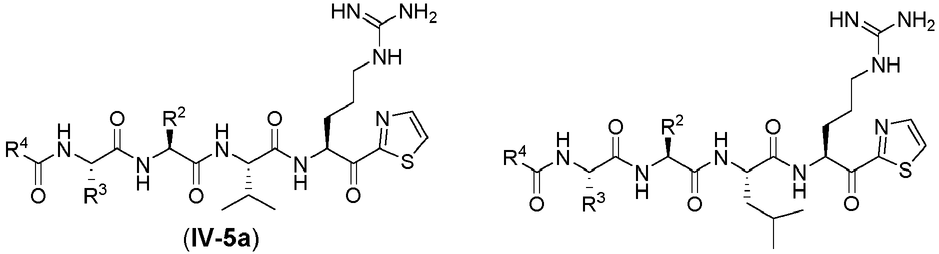 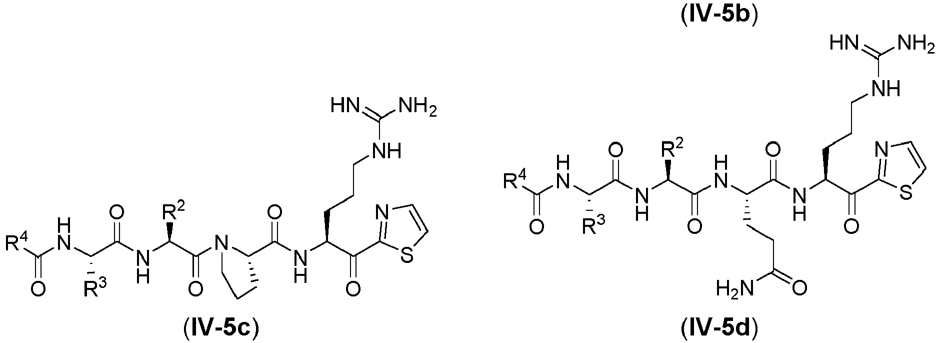 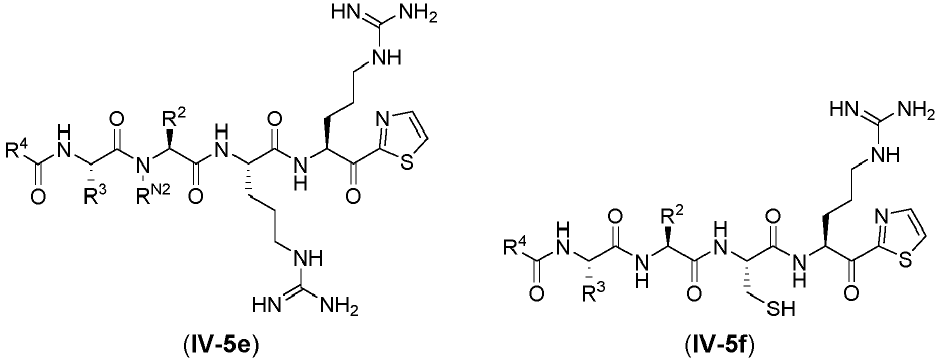 wherein **R²** - **R⁵** and **R^{N2}** have the same meansings as defined in the formula (**III-5**).

Still more preferred is the compound having any one of the formulae (**V-1a**) - (**V-1b**): 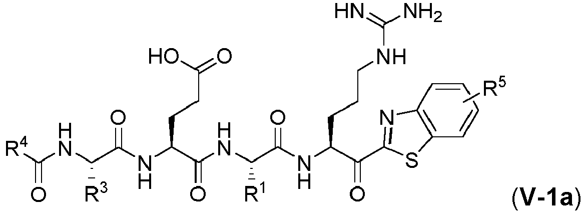 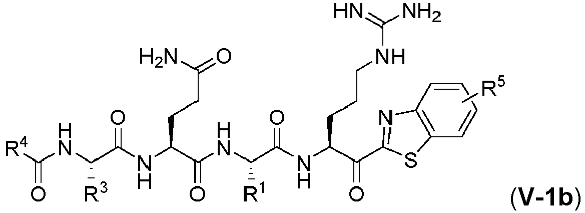 wherein **R¹**, **R³**, **R⁴**, and **R⁵** have the same meanings as defined in the formula (**III-1**).

Still more preferred is the compound having any one of the formulae (**V-2a**) - (**V-2b**): 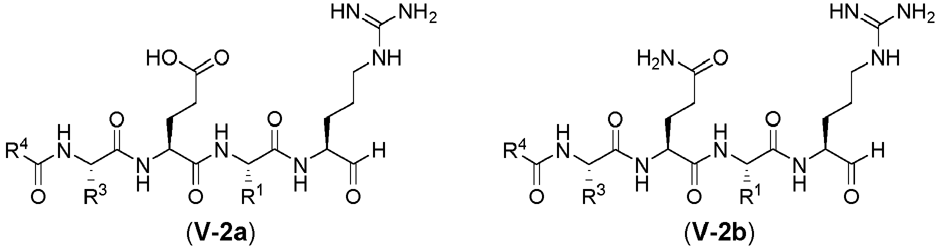 wherein **R¹**, **R³**, and **R⁴** have the same meanings as defined in the formula (**III-2**).

Still more preferred is the compound having any one of the formulae (**V-3a**) - (**V-3b**): 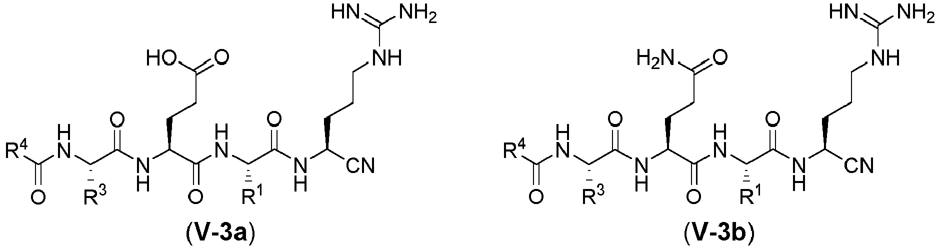 wherein **R¹**, **R³**, and **R⁴** have the same meanings as defined in the formula (**III-3**).

Still more preferred is the compound having any one of the formulae (**V-4a**) - (**V-4b**): 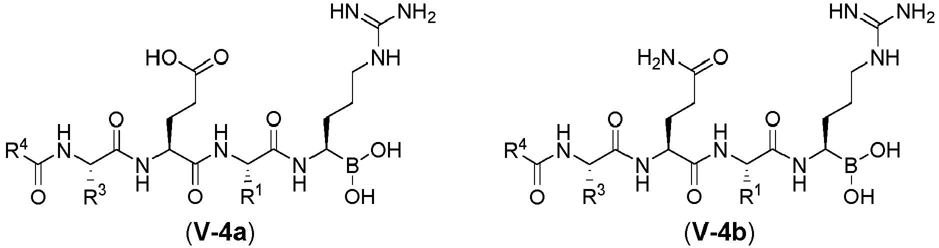 wherein **R¹**, **R³**, and **R⁴** have the same meanings as defined in the formula (**III-4**).

Still more preferred is the compound having any one of the formulae (V-1a) - (V-1b): 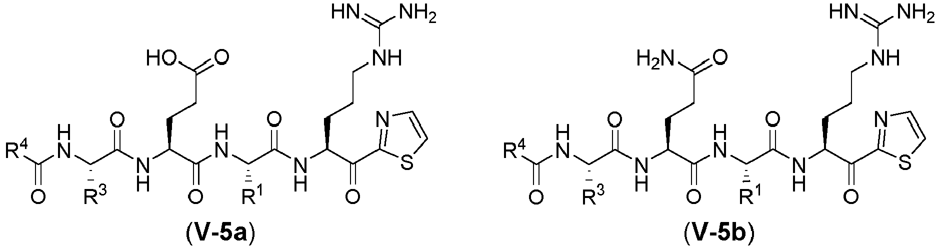 wherein **R¹**, **R³**, **R⁴**, and **R⁵** have the same meanings as defined in the formula (**III-5**).

Still more preferred is the compound having any one of the formulae (**VI-1a**) - (**VI-1g**): 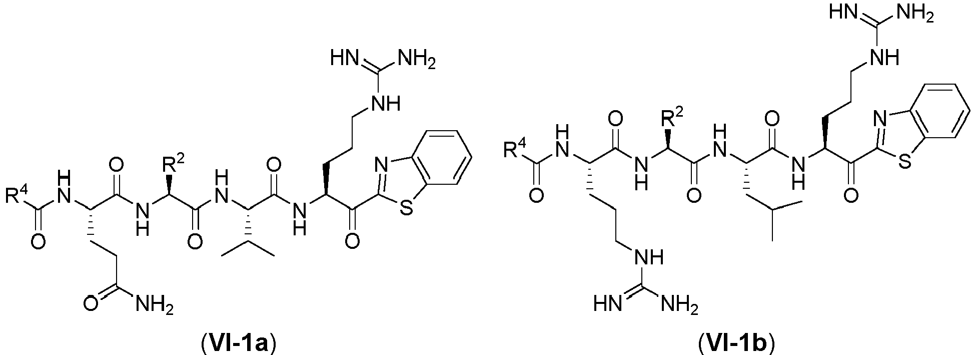 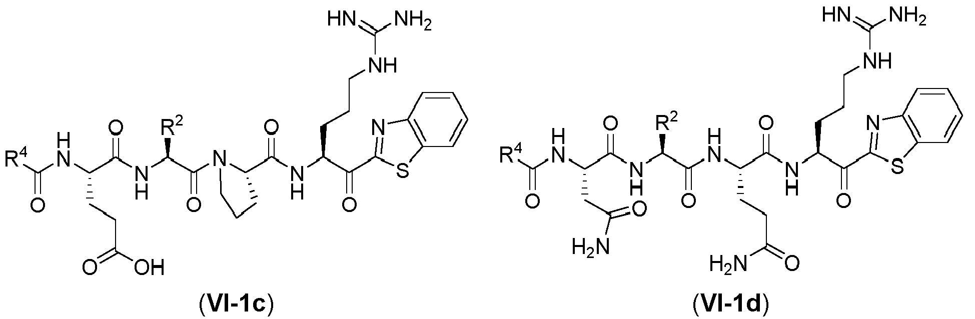 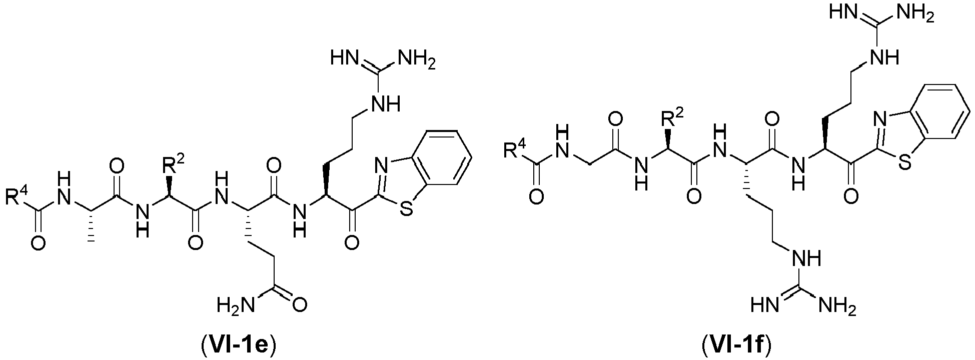 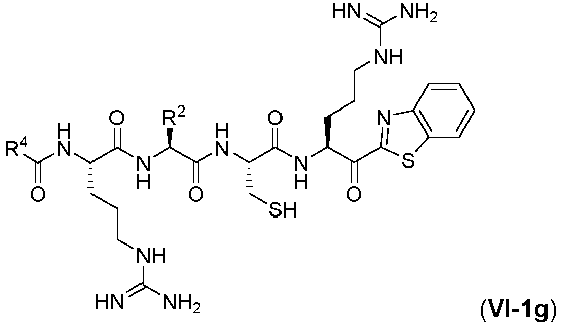 wherein **R²** and **R⁴** have the same meanings as defined in formula (**III-1**).

Still more preferred is the compound having any one of the formulae (**VI-2a**) - (**VI-2g**): 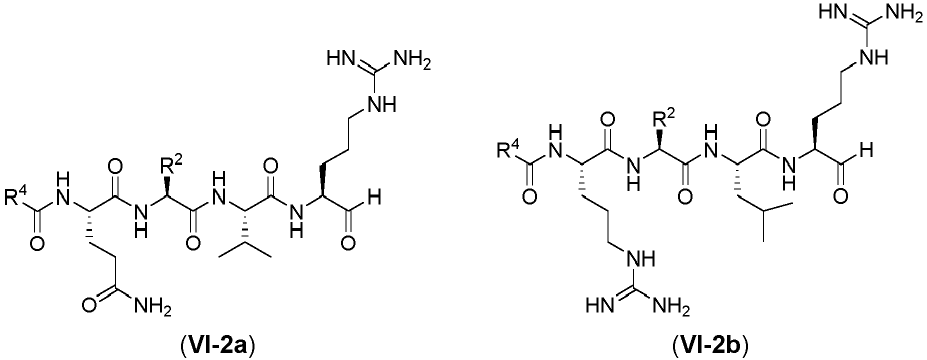 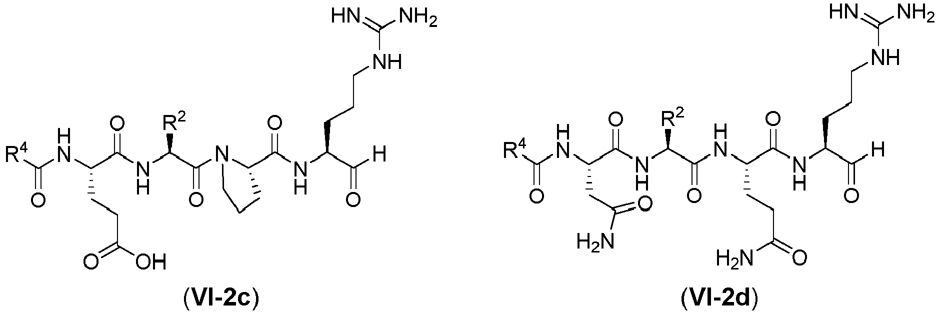 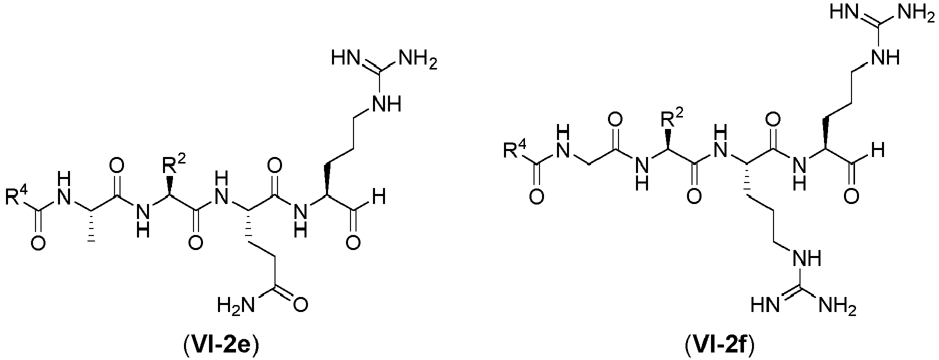 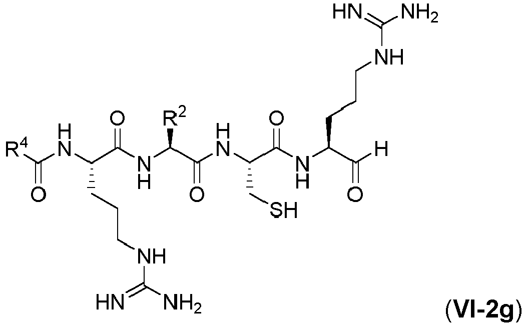 wherein **R²** and **R⁴** have the same meanings as defined in formula (**III-2**).

Still more preferred is the compound having any one of the formulae (**VI-3a**) - (**VI-3g**): 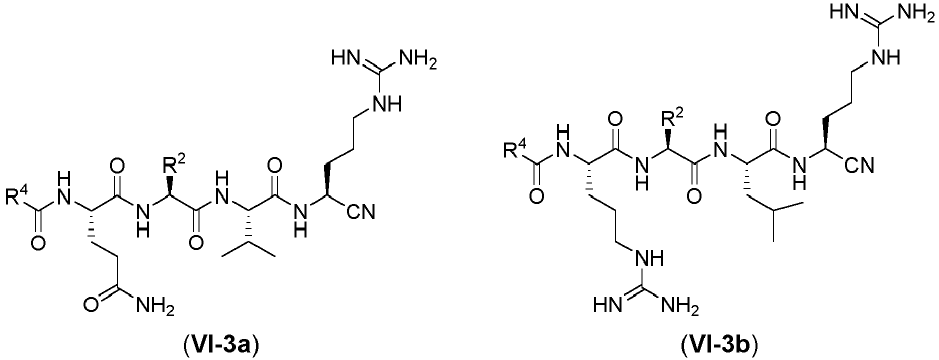 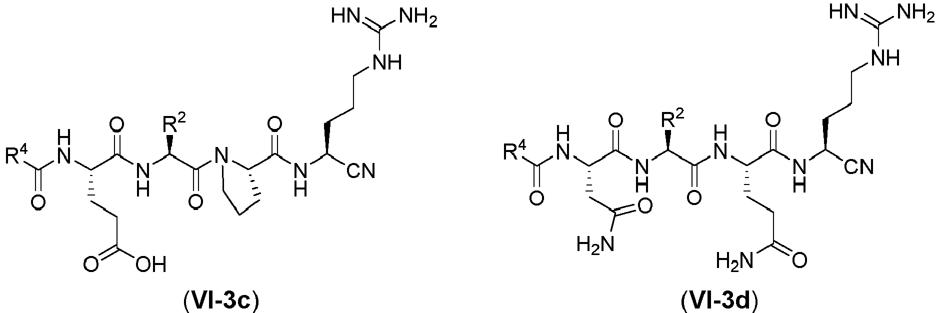 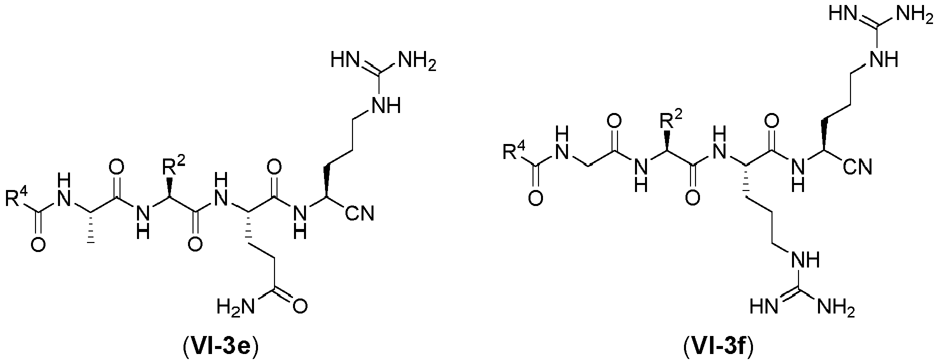 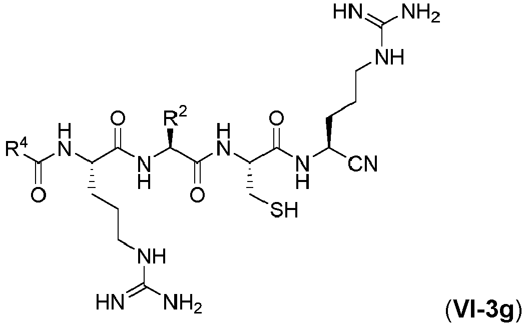 wherein **R²** and **R⁴** have the same meanings as defined in formula (**III-3**).

Still more preferred is the compound having any one of the formulae (**VI-4a**) - (**VI-4g**): 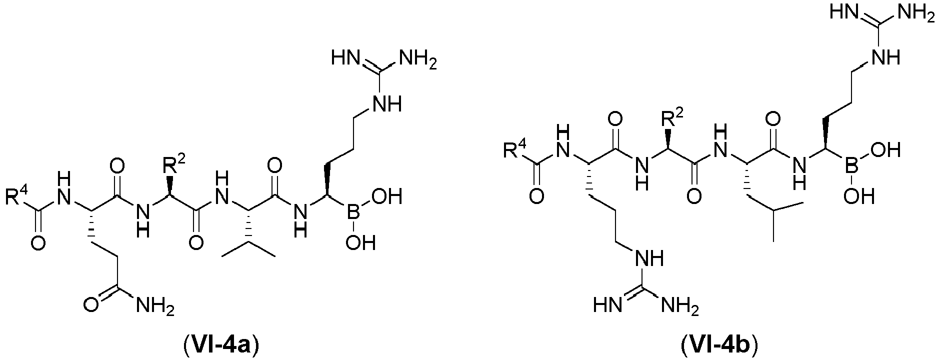 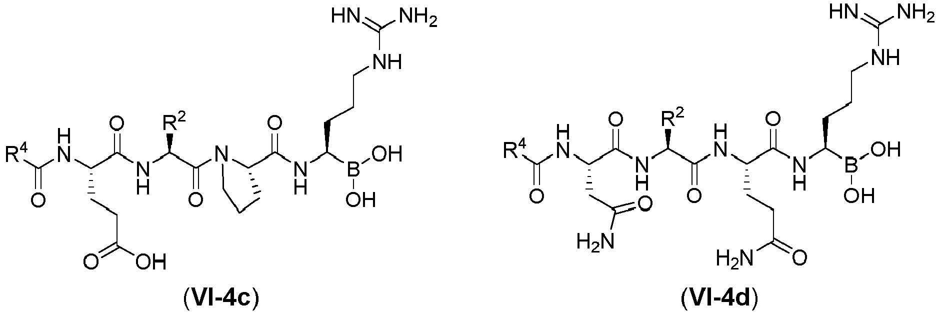 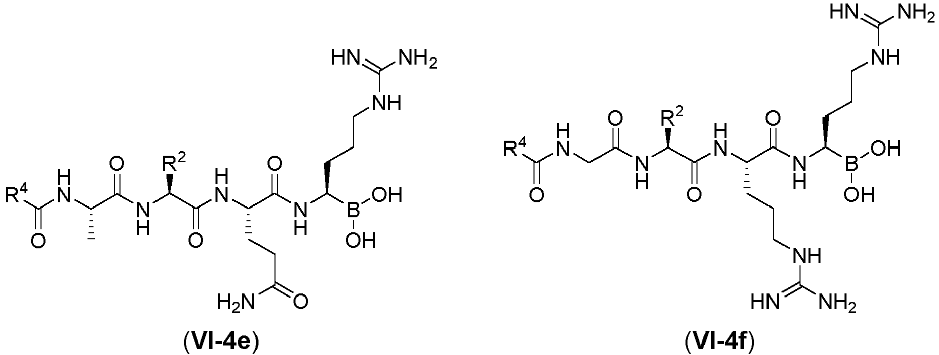 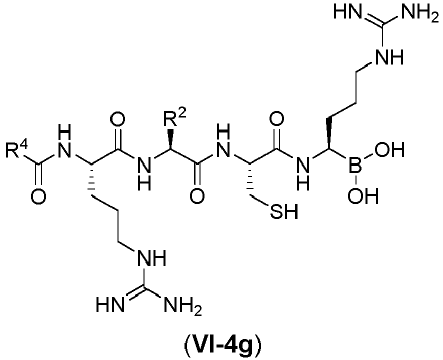 wherein **R²** and **R⁴** have the same meanings as defined in formula (**III-4**).

Still more preferred is the compound having any one of the formulae (**VI-5a**) - (**VI-5g**): 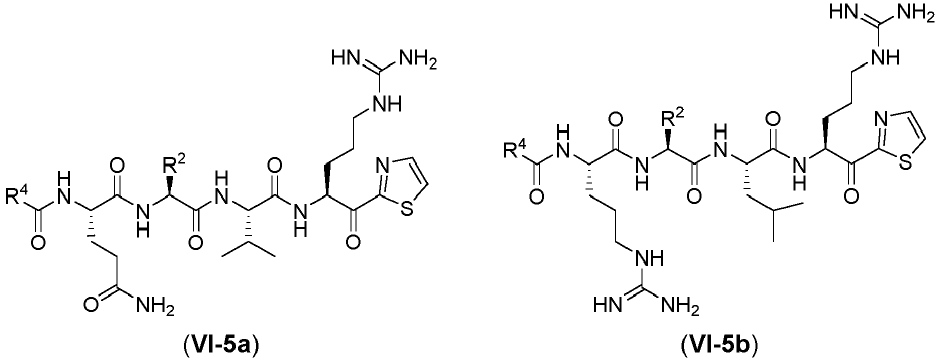 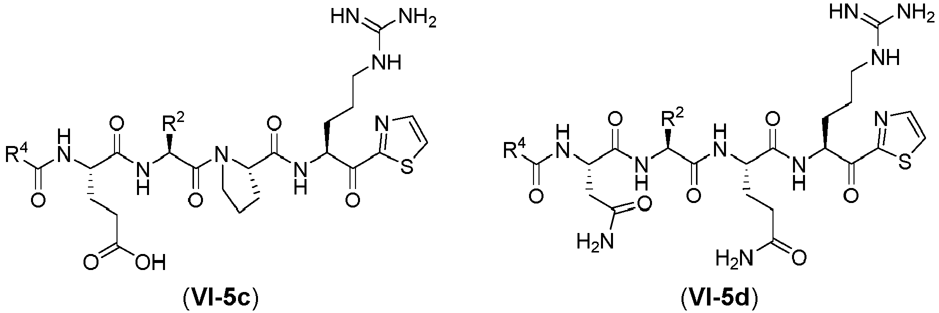 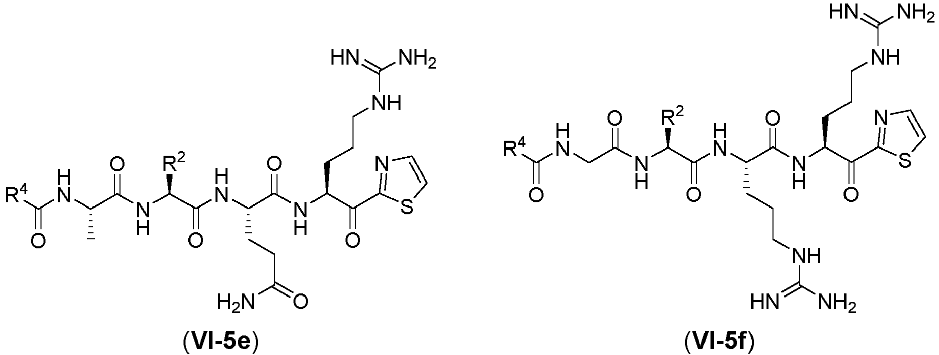 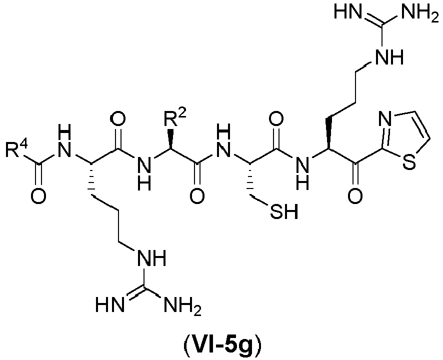 wherein **R²** and **R⁴** have the same meanings as defined in formula (**III-5**).

Still more preferred are compounds of any one of the formulae (**VII-1a**) - (**VII-1h**): 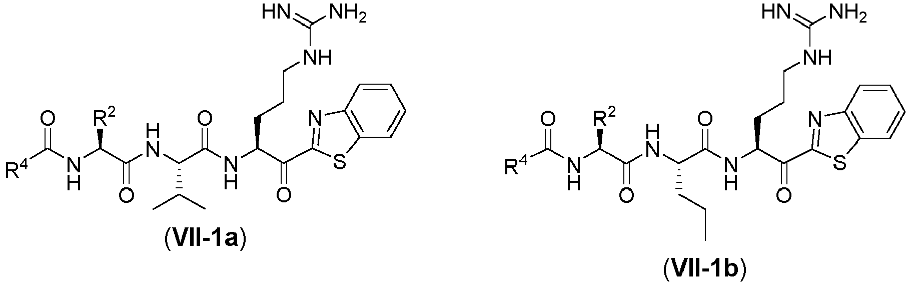 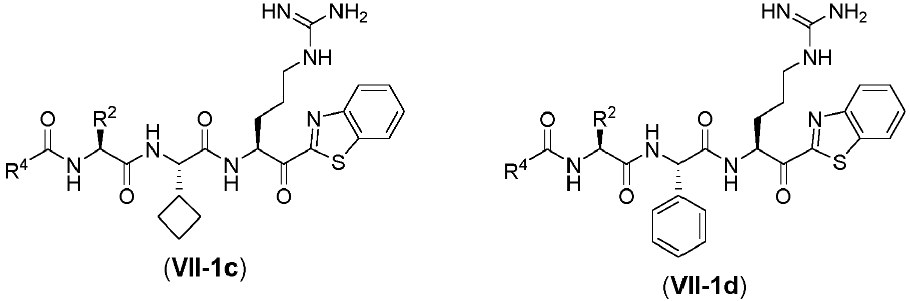 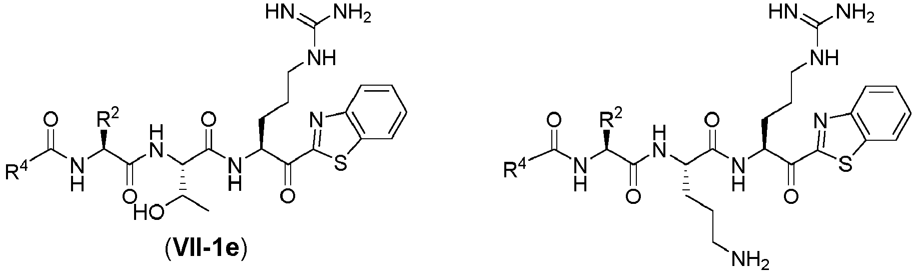 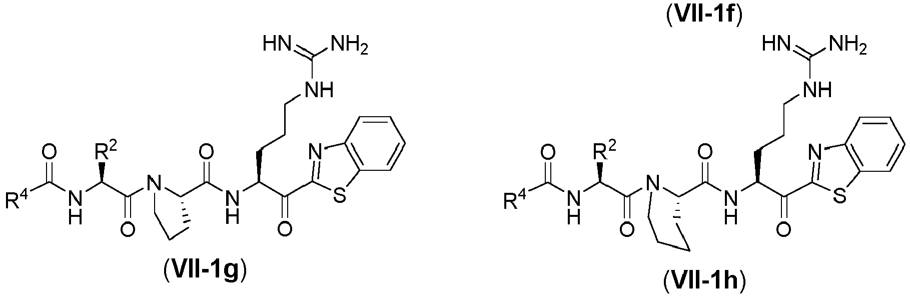
wherein **R²** represents 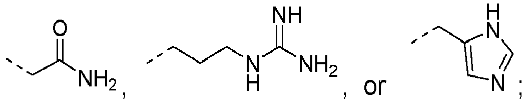
**R⁴** represents -CH₃, or -O-*tert*-Bu.

Still more preferred are compounds of any one of the formulae (**VII-2a**) - (**VII-2h**): 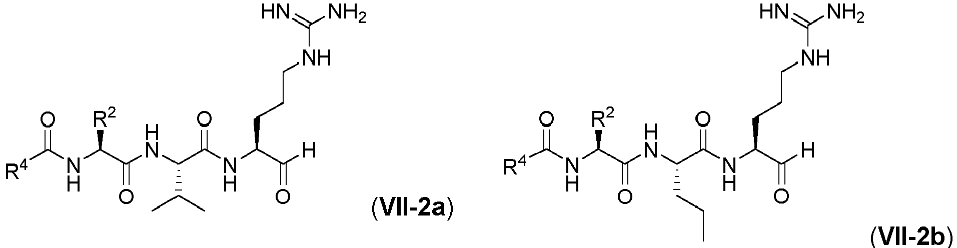 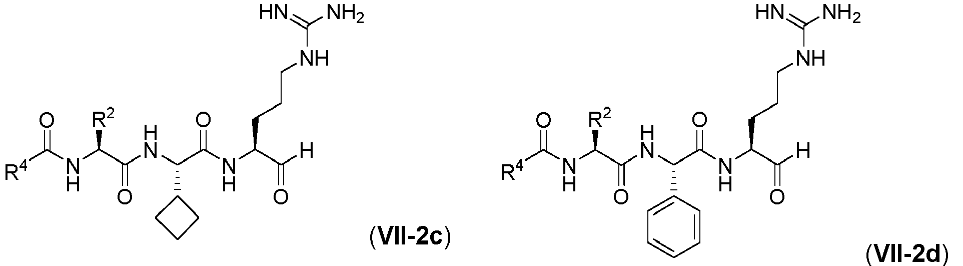 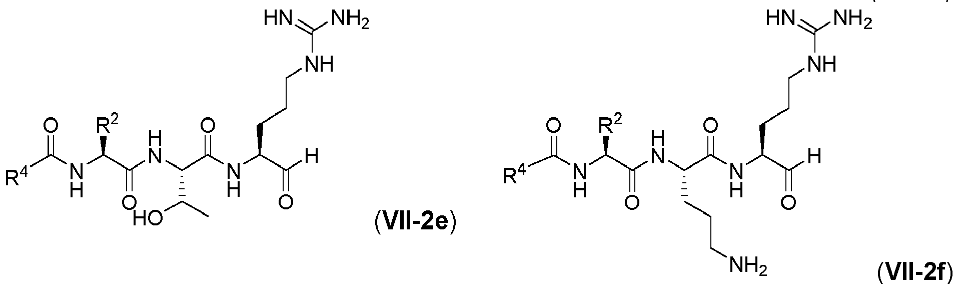 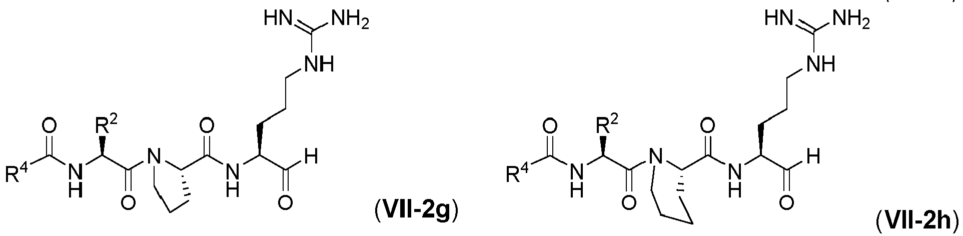
wherein **R²** represents 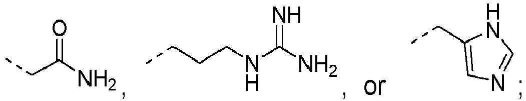
**R⁴** represents -CH₃, or -O-*tert*-Bu.

Still more preferred are the compounds of any one of the formulae (**VII-3a**) - (**VII-3h**): 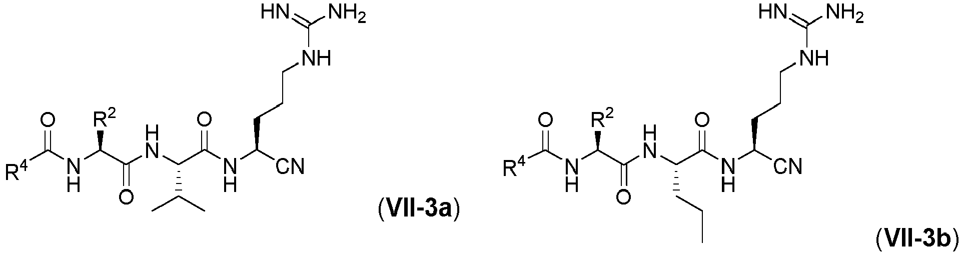 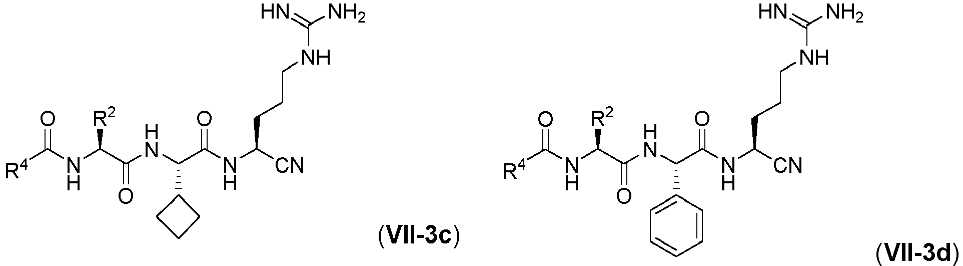 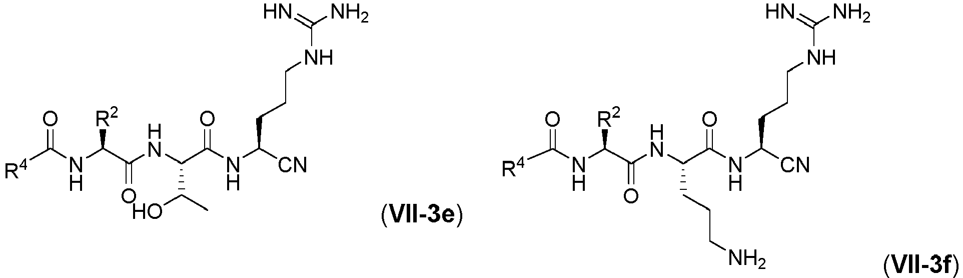 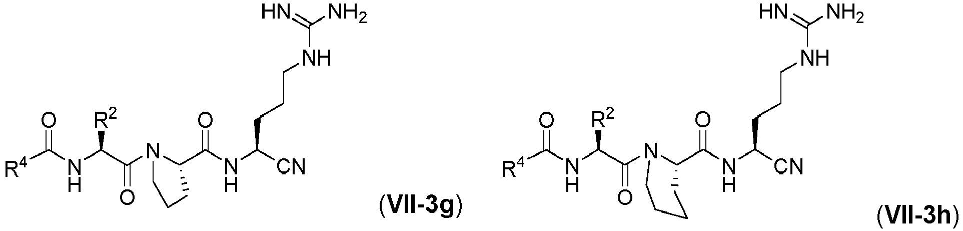
wherein **R²** represents 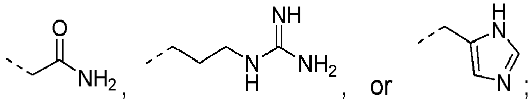
**R⁴** represents -CH₃ or -O-*tert*-Bu.

Still more preferred are compounds of any one of the formulae (**VII-4a**) - (**VII-4h**): 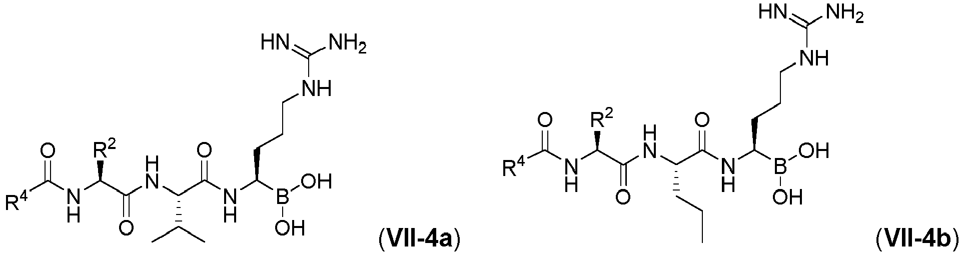 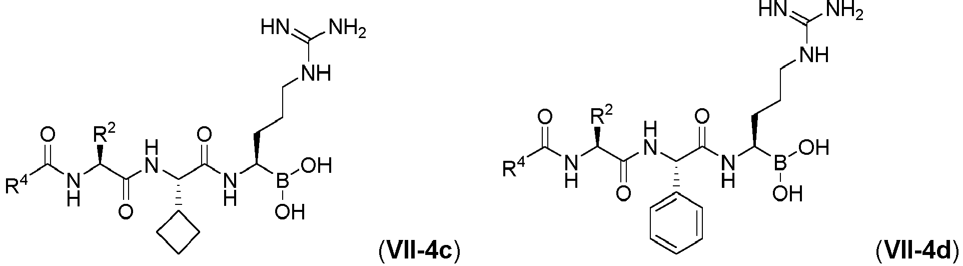 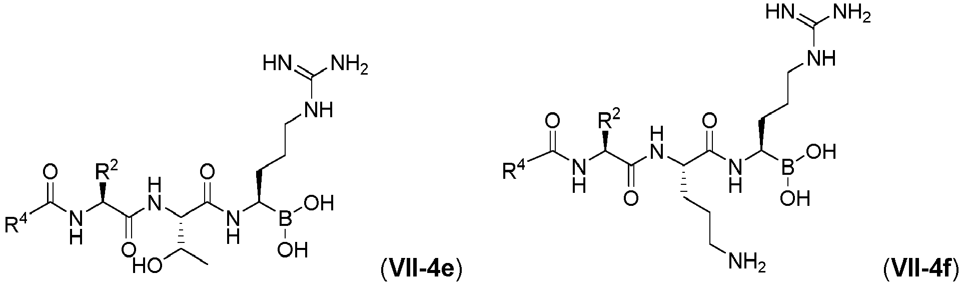 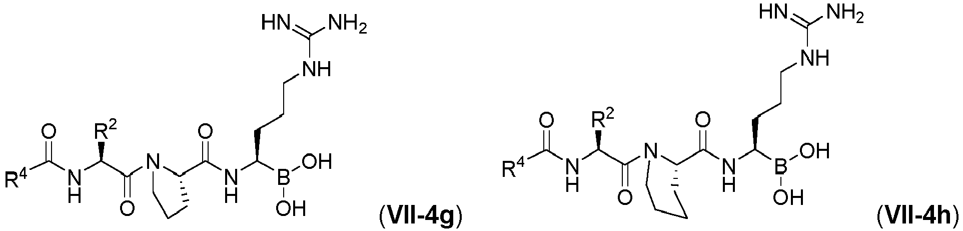
wherein **R²** represents 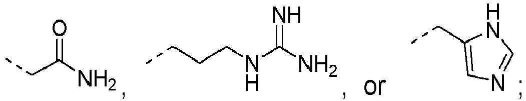
**R⁴** represents -CH₃ or -O-*tert*-Bu.

Still more preferred are compounds of any one of the formulae (**VII-5a**) - (**VII-5h**): 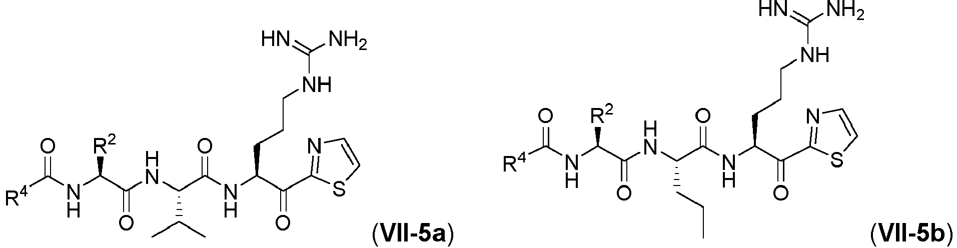 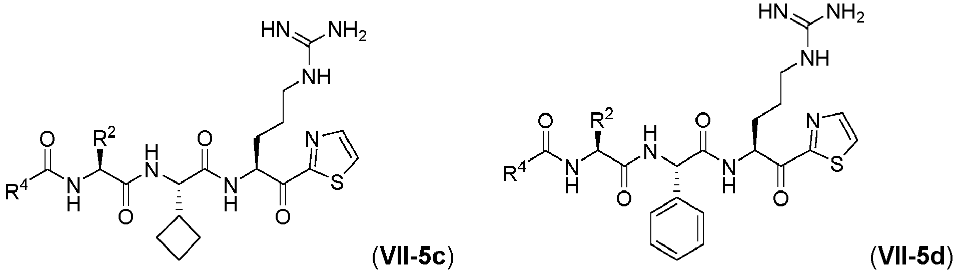 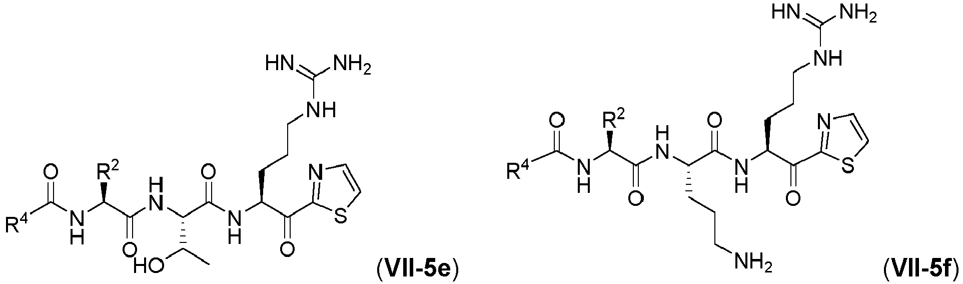 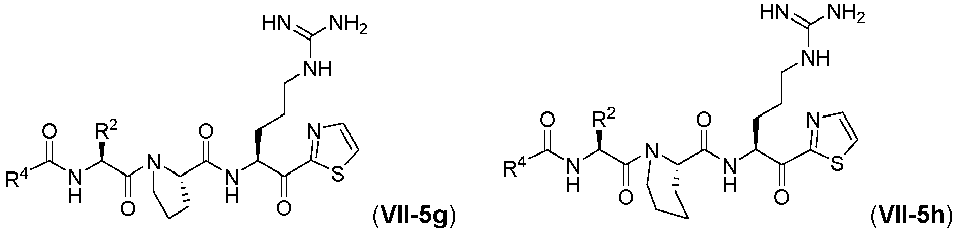
wherein **R²** represents 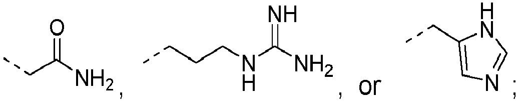
**R⁴** represents -CH₃ or -O-*tert*-Bu.

Still more preferred are compounds of any one of the formulae (**VII-6a**) - (**VII-6h**): 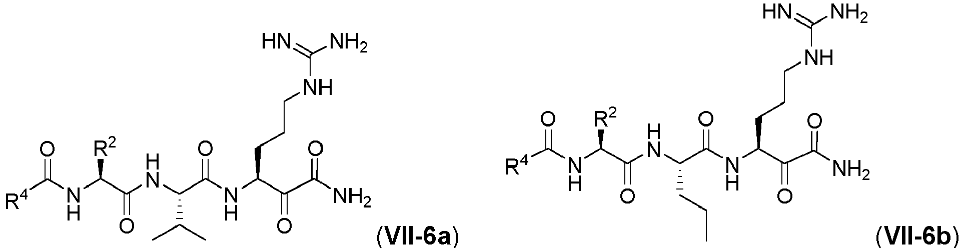 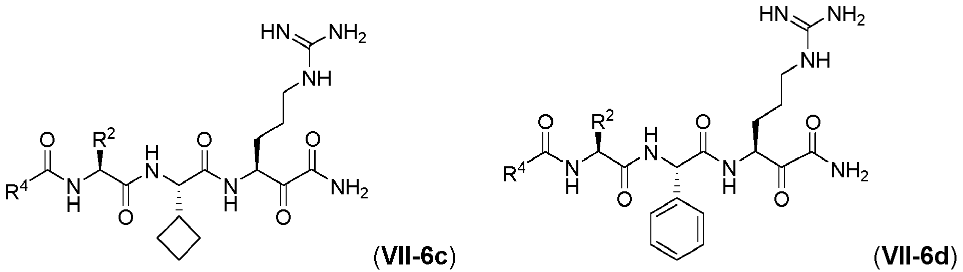 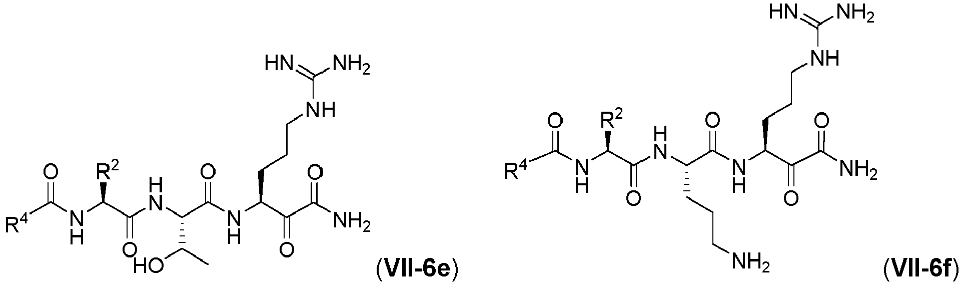 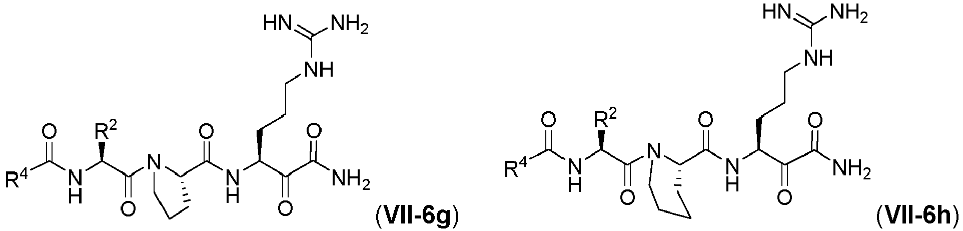
wherein **R²** represents 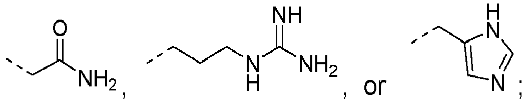
**R⁴** represents -CH₃ or -O-*tert*-Bu.

Still more preferred are compounds of any one of the formulae (**VIII-1a**) - (**VIII-1h**): 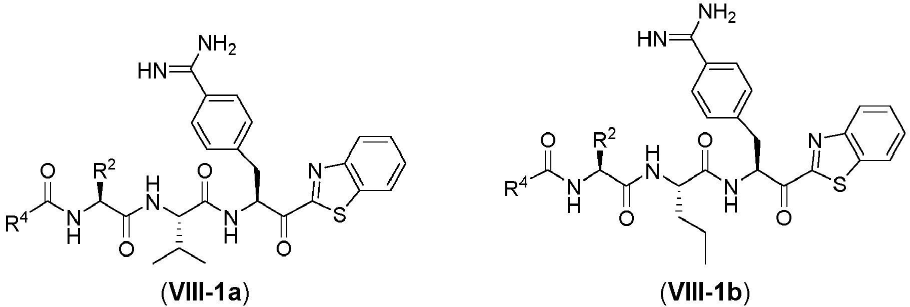 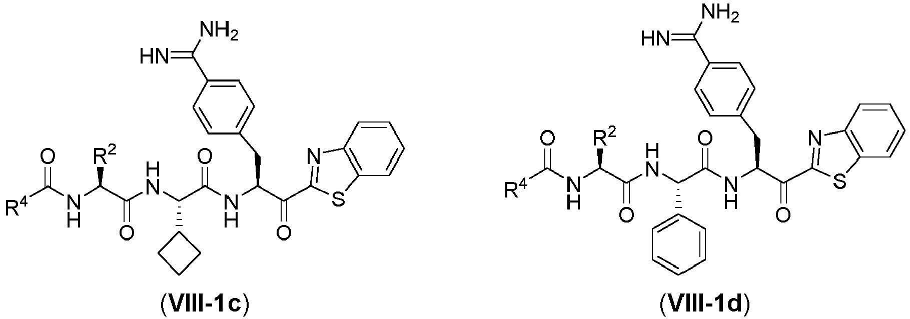 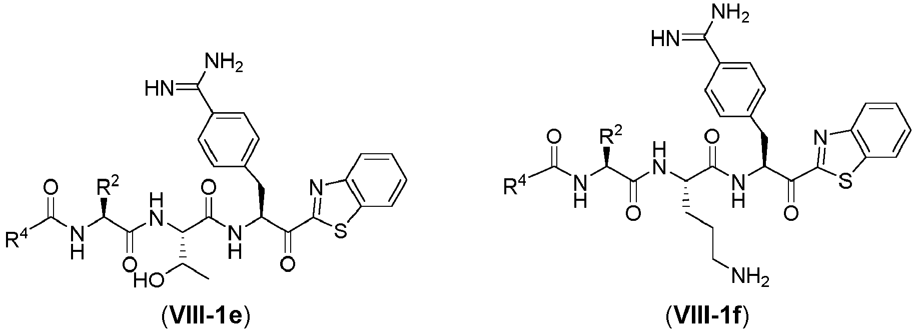 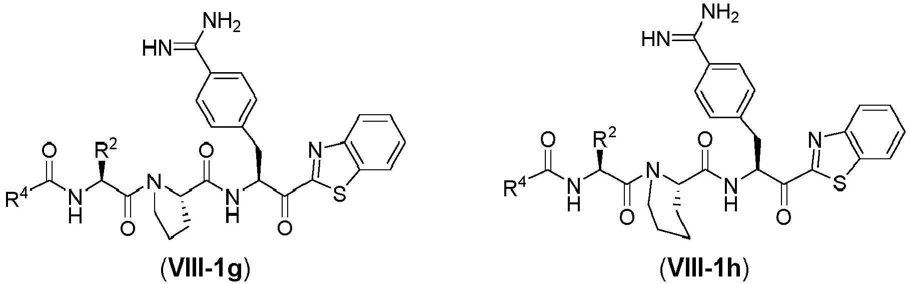
wherein **R²** represents 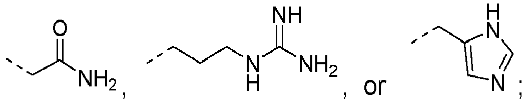
**R⁴** represents -CH₃ or -O-*tert*-Bu.

Still more preferred are compounds of any one of the formulae (**VIII-2a**) - (**VIII-2h**): 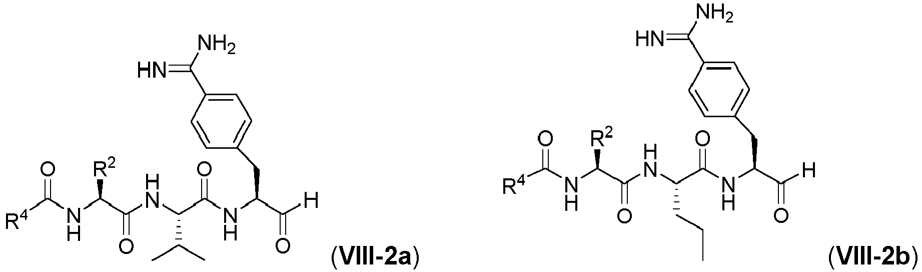 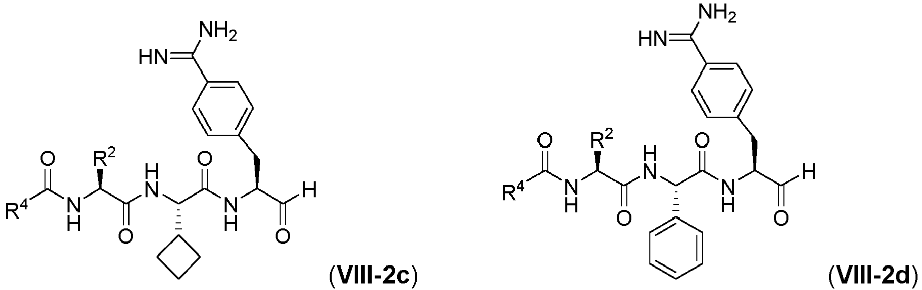 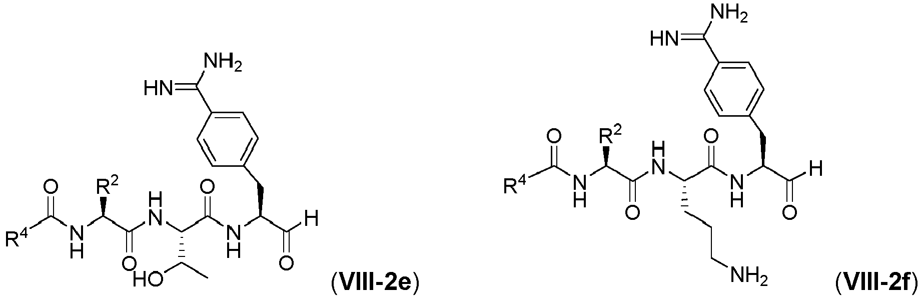 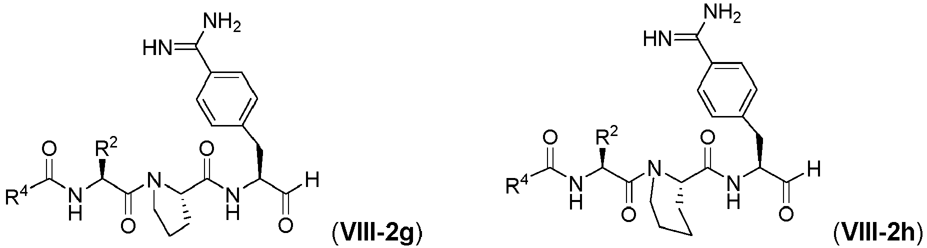
wherein **R²** represents 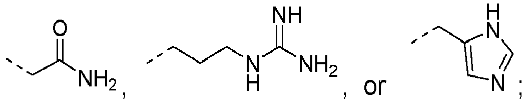
**R⁴** represents -CH₃ or -O-*tert*-Bu.

Still more preferred are compounds of any one of the formulae (**VIII-3a**) - (**VIII-3h**): 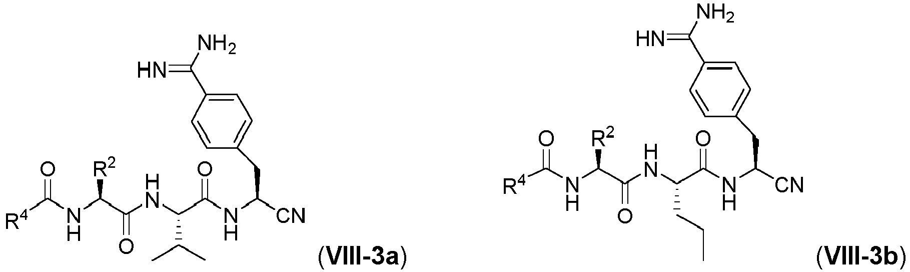 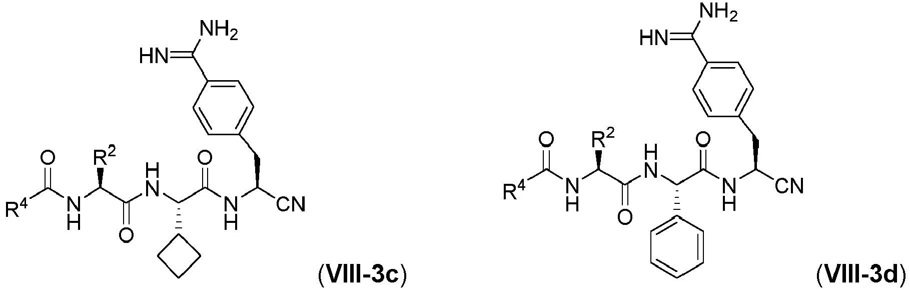 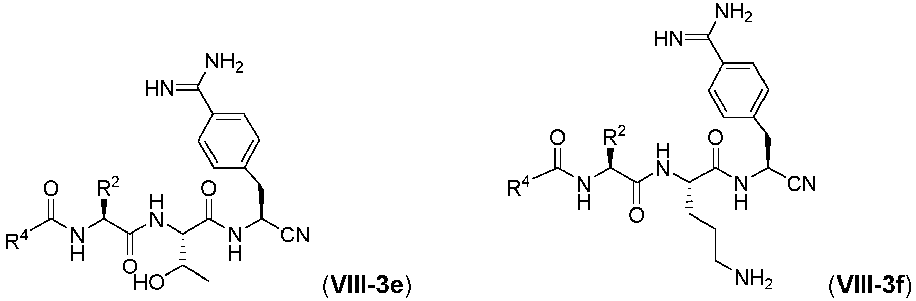 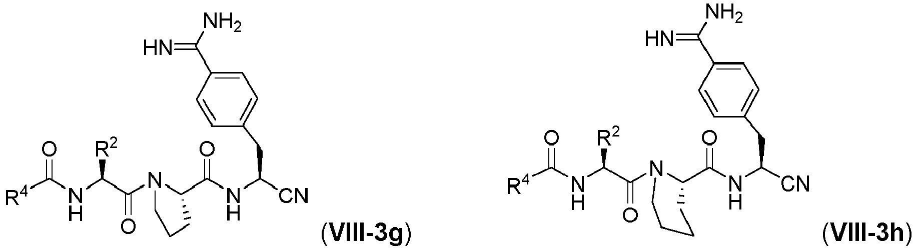
wherein **R²** represents 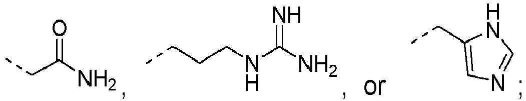
**R⁴** represents -CH₃ or -O-*tert*-Bu.

Still more preferred are compounds of any one of the formulae (**VIII-4a**) - (**VIII-4h**): 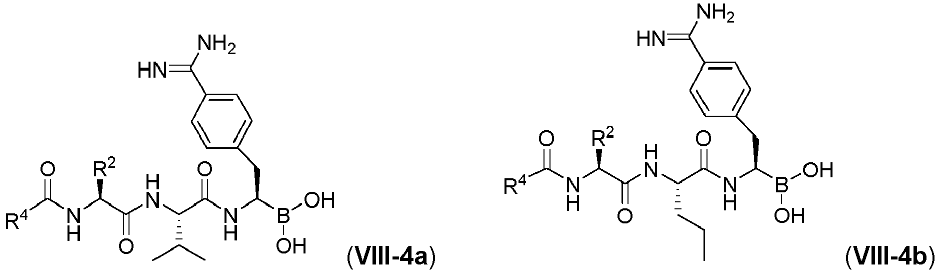 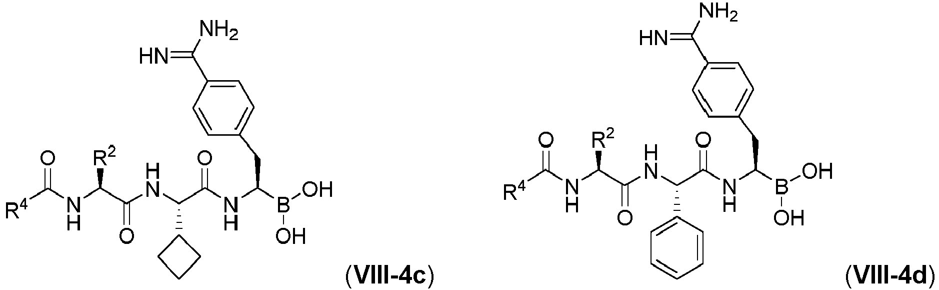 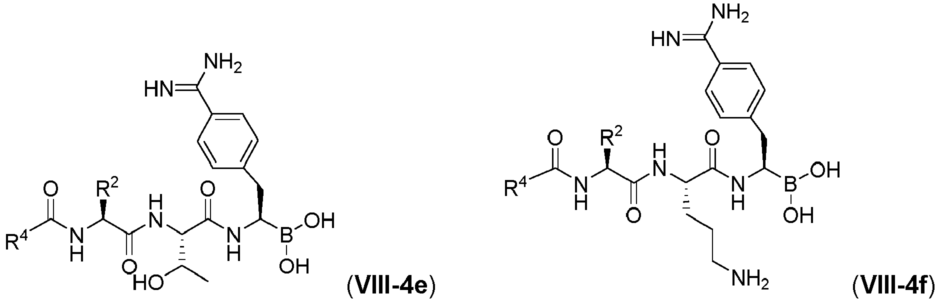 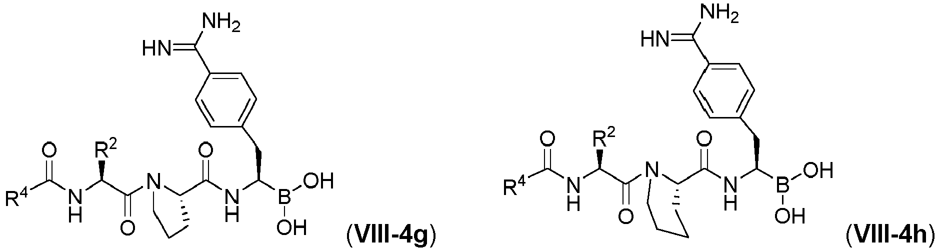
wherein **R²** represents 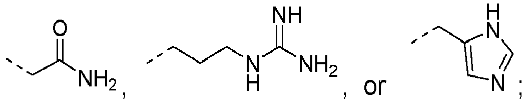
**R⁴** represents -CH₃ or -O-*tert*-Bu.

Still more preferred are compounds of any one of the formulae (**VIII-5a**) - (**VIII-5h**): 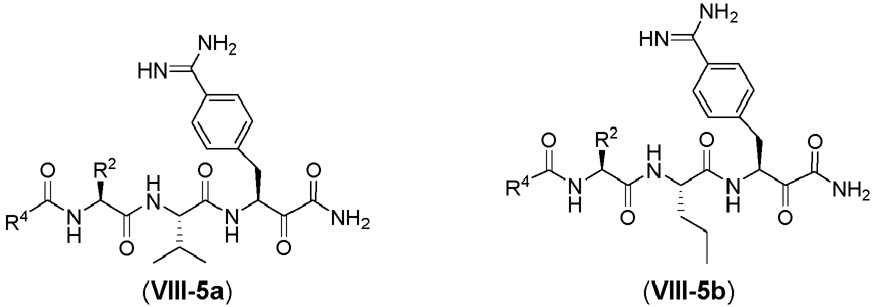 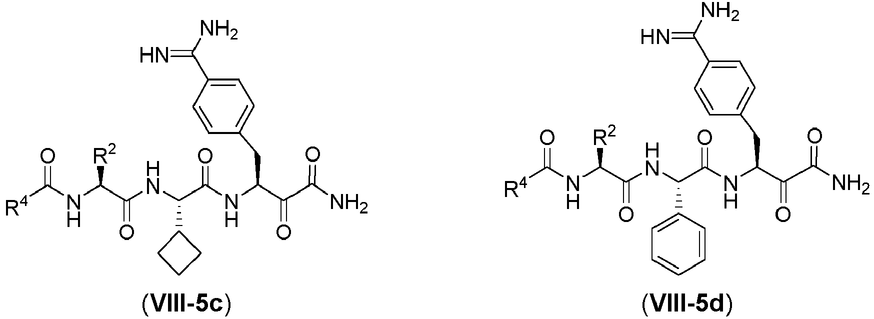 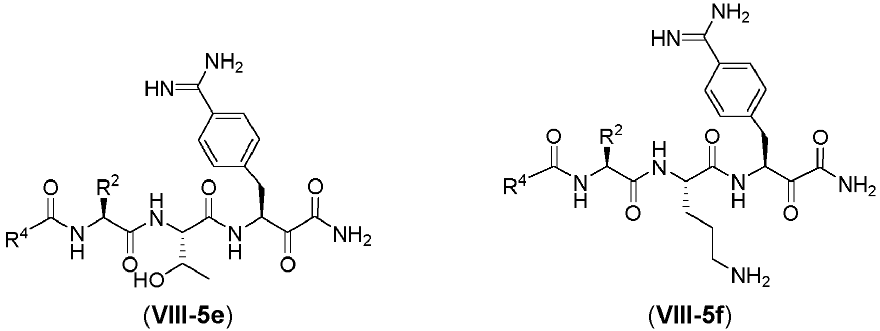 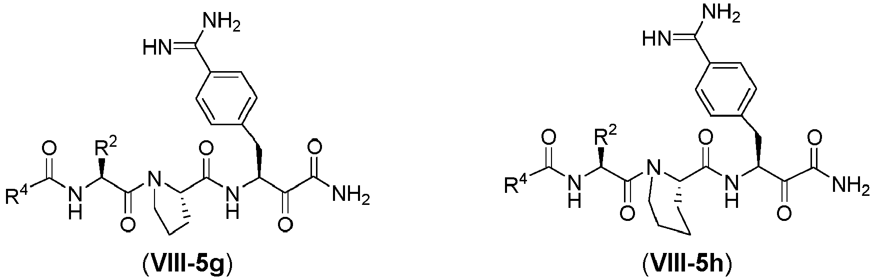
wherein **R²** represents 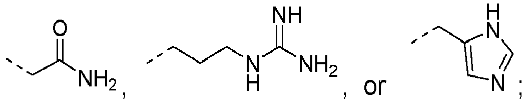
**R⁴** represents -CH₃ or -O-*tert*-Bu.

Still more preferred are compounds of any one of the formulae (**VIII-6a**) - (**VIII-6h**): 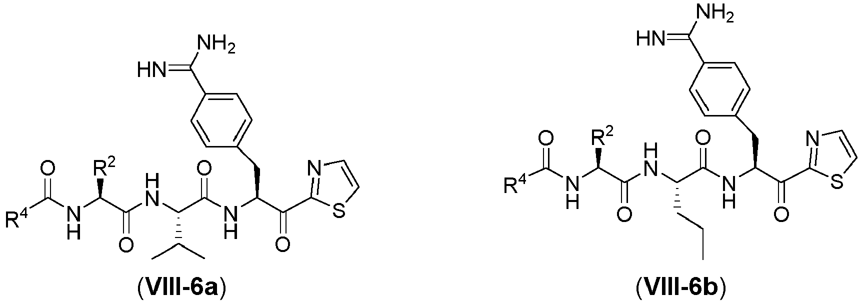 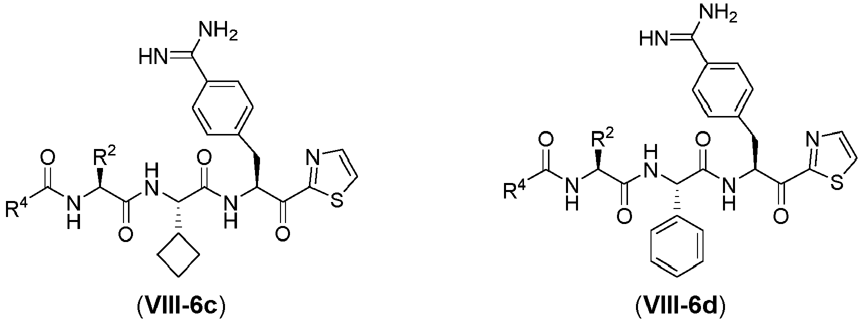 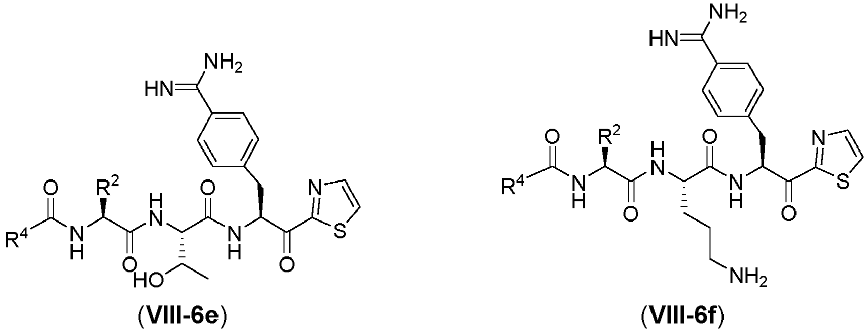 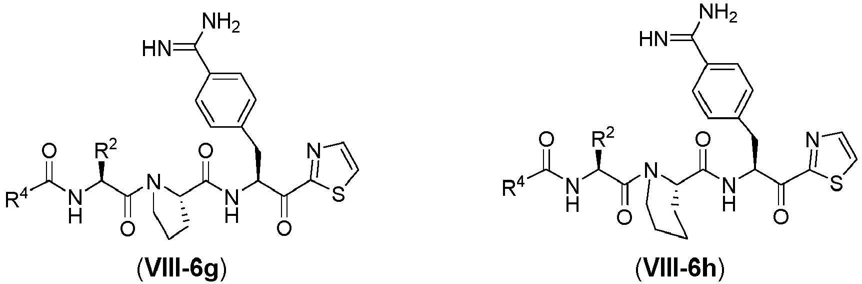
wherein **R²** represents 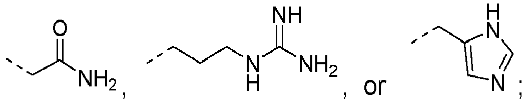
**R⁴** represents -CH₃ or -O-*tert*-Bu.

It is well-known for a person skilled in the art that the proline moiety of the compounds of any of the above-mentioned formulae (**IV-1c**), (**IV-2c**), (**IV-3c**), (**IV-4c**), (**VI-1c**), (**VI-2c**), (**VI-3c**), (**VI-4c**), (**VI-5c**), (**VII-1g**), (**VII-2g**), (**VII-3g**), (**VII-4g**), (**VII-5g**), (**VII-6g**), (**VIII-1g**), (**VIII-2g**), (**VIII-3g**), (**VIII-4g**), (**VIII-5g**) and (**VIII-6g**) can be equivalently replaced with any of the following groups: 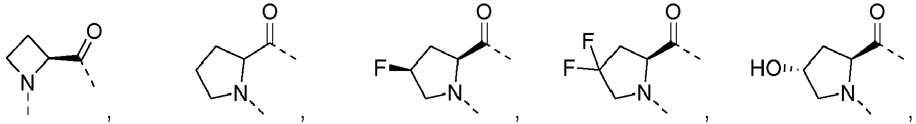 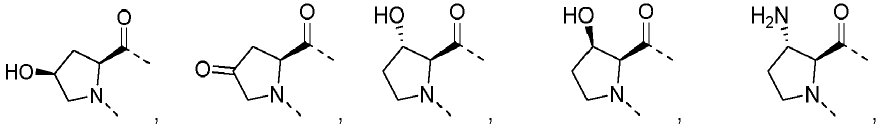 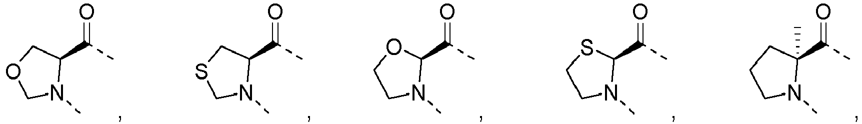 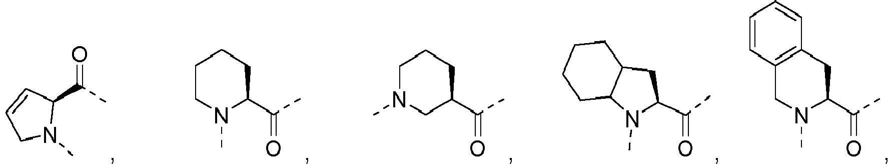 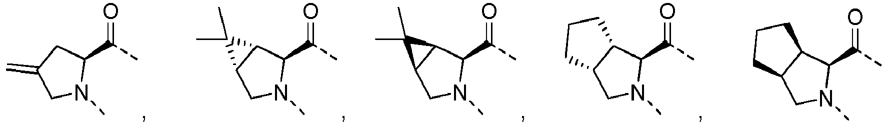 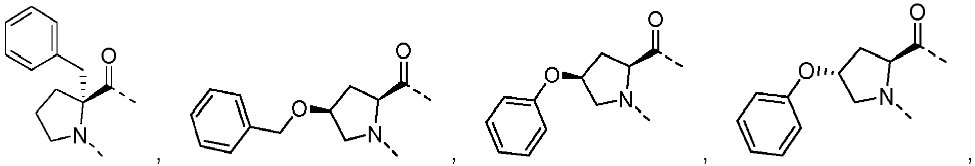 and

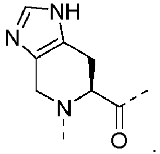

Most preferred are the compounds selected from the group consisting of:

| | |
|---|---|
| **Compound 1:** | 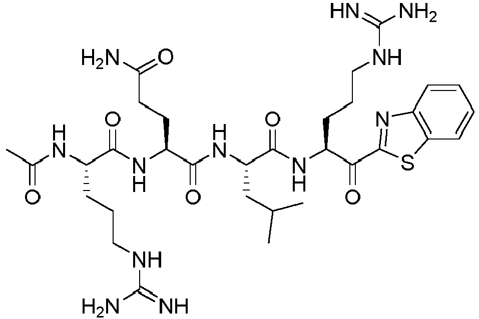 |
| **Compound 2:** | 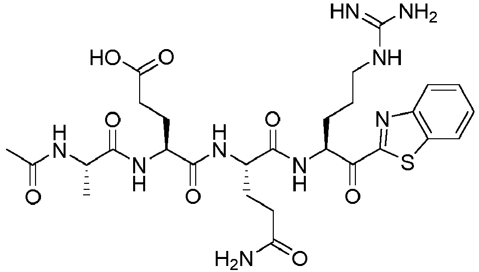 |
| **Compound 3:** | 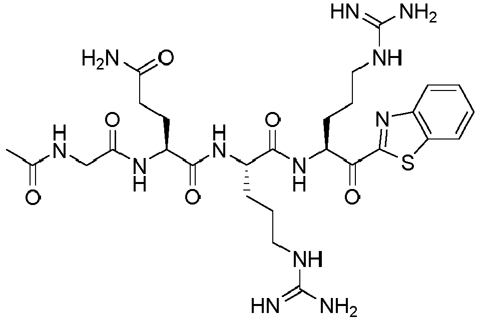 |
| **Compound 4:** | 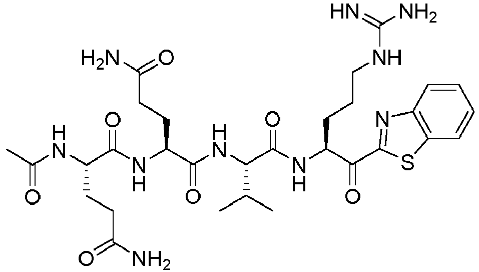 |
| **Compound 5:** | 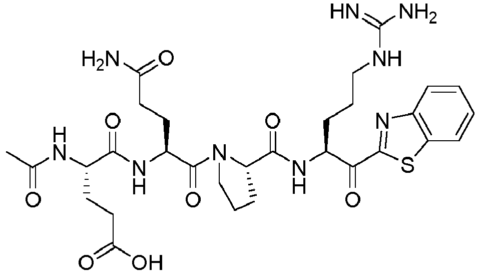 |
| **Compound 6:** | 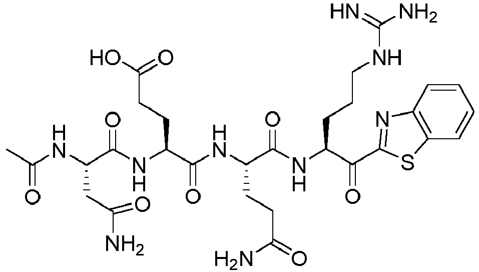 |
| **Compound 7:** | 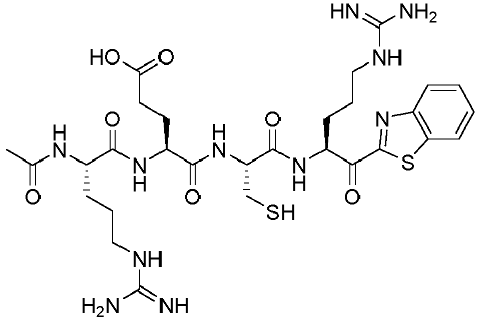 |
| **Compound ST1:** | 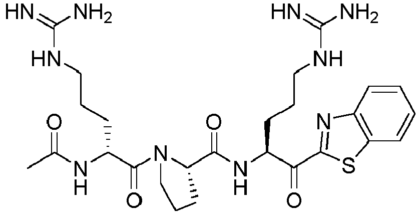 |
| **Compound ST2:** | 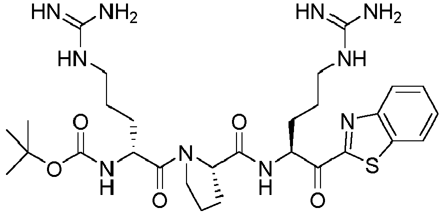 |
| **Compound ST3:** | 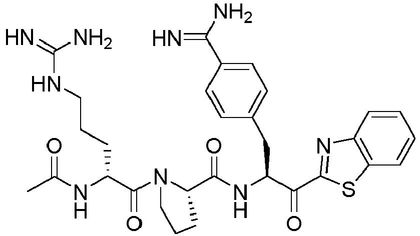 |
| **Compound ST3b:** | 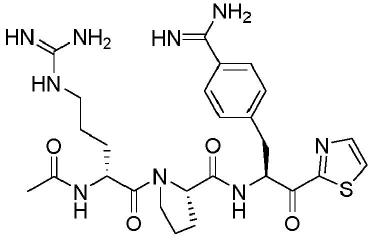 |
| **Compound ST3c:** | 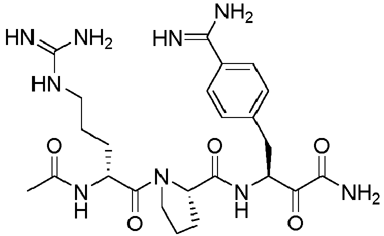 |
| **Compound ST3d:** | 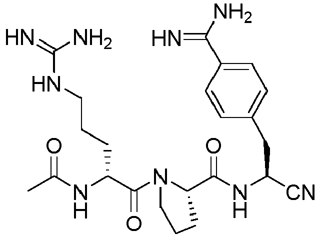 |
| **Compound ST3e:** | 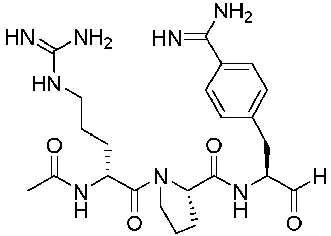 |
| **Compound ST4:** | 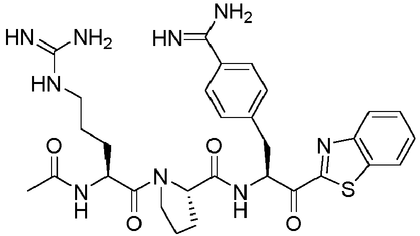 |
| **Compound ST5:** | 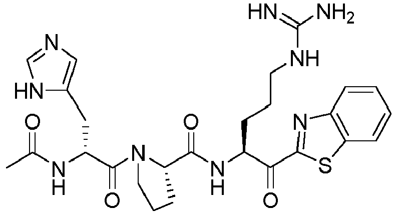 |
| **Compound ST6:** | 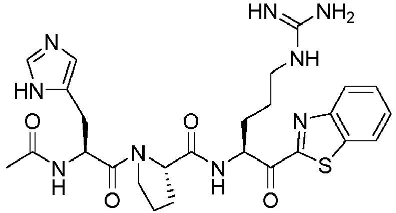 |
| **Compound ST7:** | 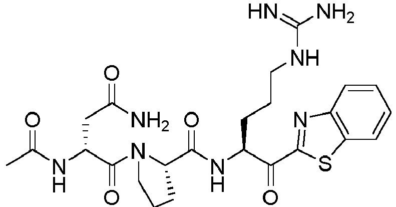 |
| **Compound ST8:** | 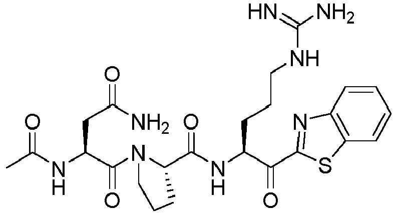 |
| **Compound ST9:** | 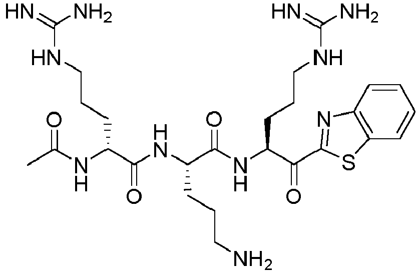 |
| **Compound ST10:** | 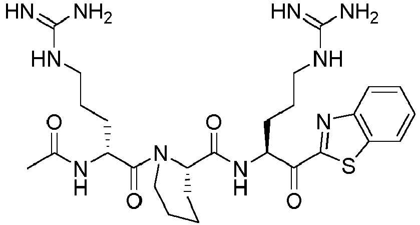 |
| **Compound ST11:** | 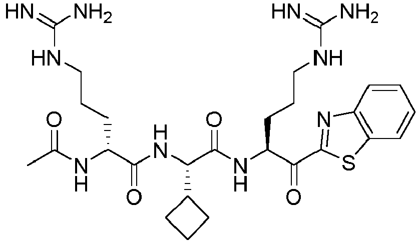 |
| **Compound ST12:** | 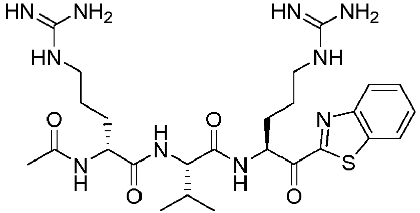 |
| **Compound ST13:** | 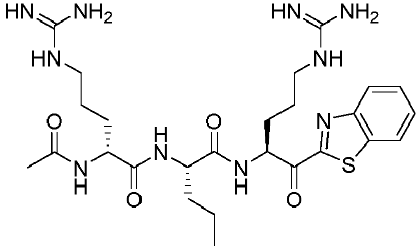 |
| **Compound ST14:** | 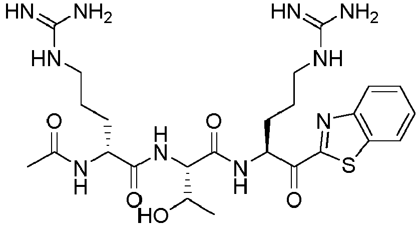 |
| **Compound ST15:** | 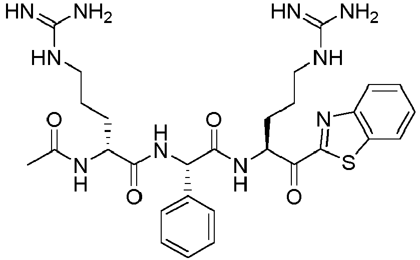 |

A further aspect of the present invention relates to the production of compounds of the general formula (**III-1**).

As shown in Scheme 1, a method for producing the compound of the present invention comprises the steps:
**Step A**: providing a benzothiazole precursor **1*** and a tripeptide precursor **2***; 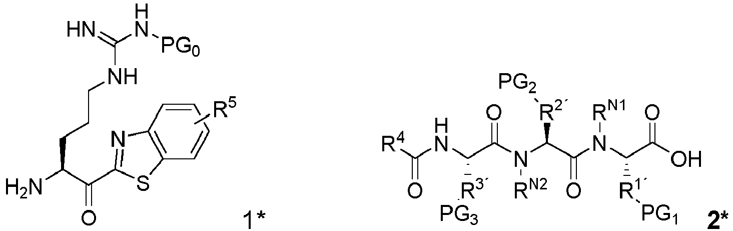
   wherein PG₀, PG₁, PG₂ and PG₃ are protecting groups,
   R^{N1}, R^{N2}, R⁴ and R⁵ have the same meanings as defined herein,
**Step B**: performing a coupling reaction with the benzothiazole precursor **1*** with the tripeptide precursor **2*** to obtain an intermediate compound **3***; 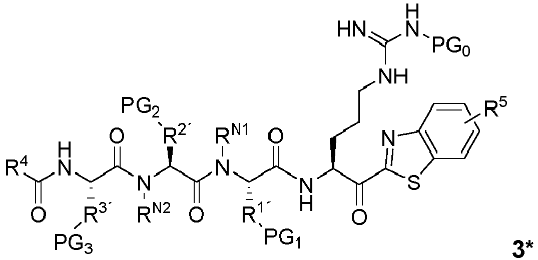 and
**Step C:** removing protecting groups PG₀, PG₁, PG₂ and PG₃ of the intermediate compound to produce the compound of the formular (III-1)

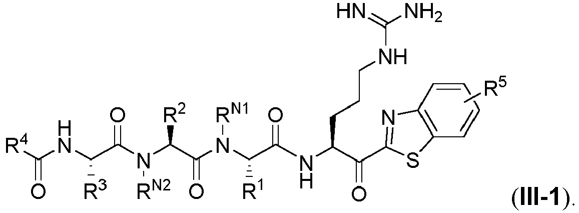

Optionally, purifying and isolsating step may be performed after the Step C as follows:
**Step D:** purifiying and isolating the compound of the formula (III-1) obtained by the Step C.

Herein the tripeptide precursor is obtained by solid phase peptide synthesis.

All protecting groups PG₀, PG₁, PG₂ and PG₃ are selectively or simultaneously removed.

The term "protecting groups" as used herein refers to commonly used protection groups in organic synthesis, preferably for amino and carboxyl groups. Amino protecting gorups may be selected from the group consisting of or comprising: acetyl, benzoyl, benzyloxycarbonyl (Cbz), tert-butylcarbonyl, tert-butyloxycarbonyl (Boc), trimethylbenzenesulfonyl(Mtr) and fluorenylmethylenoxy group (Fmoc). Carboxyl protecting groups may be selected from the group consisting of or comprising: methoxy, ethoxy, isobutoxy, tert-butoxy, benzyloxy; preferably, tert-butoxy group. PG₀ preferably is suitable amino protecting group.

In coupling reaction in Step B, carboxyl acid is activated and promote the coupling reaction with amino group of intermediate compound. Preferably, carboxyl acid is activated in situ and it is well-known in peptide chemistry. Any of the following coupling reagent can be used to introduce activating group AG1: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP.

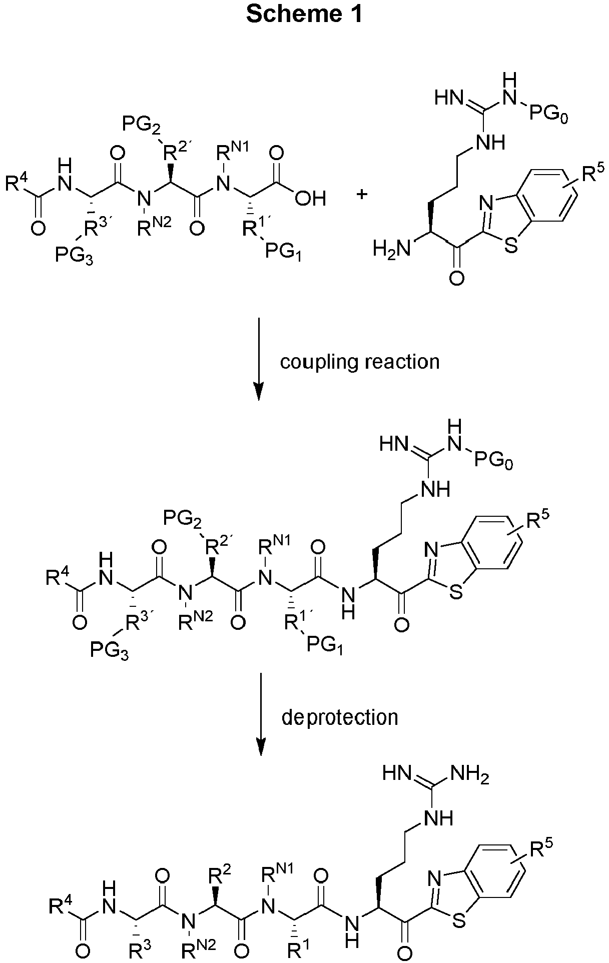

### Medical Use

The particular suitability of the inventive compounds of the general formula (I) is connected to the sterical and electronical properties which result from the molecule structure. The electrophilic benzothiazole warhead group is an essential unit of the reversible TMPRSS2 inhibitors, and, especially in combination with the certain peptidomimetic backbone, the arginine backbone for the P1 pocket and the certain specfic amino acids at the P2 postion which result in potent TMPRSS2 Selectivity is obtained by implementing said components at selected positions within the backbone. Thus, the inventive compound inhibit selectively TMPRSS2 over thrombin, and coagulation factor Xa and also inhibit effectively the replication of influenza virus, or coronavirus, in particular SARS-CoV2.

It is known from the literature on proteases that certain warheads form covalent but reversible complexes with the active site cysteine or serin. This is particularly relevant to provide affinity to the target while forming a thiohemiacetal or hemiacetal respectively.

In the biological example B-1 and B-2, it is proven that the inventive compounds as reversible serine protease inhibitor effectively inhibit the activity of TTSP family, especially TMPRSS2 (see Table 1).

Furthermore, the biological example B-3 demonstrate that the inventive compounds effectively inhibit the infection of CaCo-2 cells casued by the LV(Luc)-CoV2 which is based on an infection-deficient lentiviral backbone that bears SARS-CoV2 spike protein (see Table 2). All of the inventive compound as TMPRSS2 inhibitors effectively LV(Luc)-CoV2-S infection in a dose-dependent manner. In particular Compound 2p2i shows similar activity to camostatmesylate. Furthermore, by microscopy: no apparent cytotoxicity of the inventive compound was observed.

Most of all, the biological example B-4 demonstrate that the inventive compounds effectively inhibit SARS-CoV2 infection (see Table 3).

Some human coronaviruses including the deadly SARS, COVID-19 (SARS-Cov-2) and MERS variants, TMPRSS2 cleavage activates the viral spike (S)-protein at the cell surface enabling cathepsin-independent host cell entry. SARS and SARS-Cov2 uses the receptor ACE2 for attachement and the serine protease TMPRSS2 for S protein priming. Thus, the SARS and COVID-19 (SARS-CoV-2) can be inhibited by TMPRSS2 inhibitors.

Therefore, another aspect of the present invention relates to compounds according to the general formula (I) as medicament as well as their use in medicine. Especially preferred is the use as selective inhibitors of TMPRSS2 over thrombin, and coagulation factor Xa and also inhibit effectively the replication of virus such as influenza virus or coronavirus.

The compounds according to general formula (I) described herein are especially suitable for the treatment and prophylaxis of for use in the treatment or prophylaxis of virus infection, preferred, wherein virus is influenza virus, parainfluenza virus or coronavirus.

Influenza virus is preferably influenza A virus or influenza B virus, more preferably influenza A. Influenza A virus includes H1N1, H3N2, and H7N9.

In a preferred embodiment, the inventive compound is used for the treatment or prophylaxis of of virus infection, preferred, wherein virus is coronavirus, preferably, coronavirus is coronavirus 229E, SARS, MERS, or SARS-CoV2, more preferably, SARS-CoV2 causing COVID-19.

In a preferred embodiment, the inventive compound is used for the treatment and prophylaxis of the above-mentioned virus infection, preferrably, coronavirus in combination with at least one active agent selected from antiviral agent, anti-HIV agent, anti-malarial agent, antibaterial agent, and monoclonal antibody.

Examples for antiviral drugs are: remdesivir, favipiravir, arbidol (umnifenovir), arbidol salts (hydrochloride), Oseltamivir(Tamiflu), DASS181, galidesivir, Virazole^{®} (ribavirin for inhalation solution), Camostat mesylate, Selinexor, emtricitabine/tenofovir.

Examples for anti-HIV agents are: I (opinavir/ ritonavir (Kaletra), Darunavir, Cobicistat, ASC09F. For example, Kaletra lopinavir / ritonavir) is expected to interact with SARS-CoV-2 proteases. The therapeutic effect of ritonavir and lopinavir could be mainly due to its inhibitory effect on coronavirus endopeptidase C30.

Examples for antiparasite and anti-malarial agents are: chloroquine, chloroquine salts (phosphate) hydroxychloroquine, hydroxychloroquine salts (sulfate), atovaquone, nitazoxanide and artemisinin.

Examples for antibacterial agents are: azithromycin (Zithromax), cararimycin, clindamycin, and telemedicine.

Preferably, the inventive compound is used in combination with at least one active agent, wherein the at least one active agent is selected from remdesivir, favipiravir (avigan), osteltamivir (tamiflu) lopinavir/ritonavir (Kaletra), darunavir, cobicistat, chlororquine, hydroxychloroquine, atovaquone, azithromycin, cararimycin, and clindamycin.

Furthermore, the inventive compound can be used further in combination with at least one further active agent as the follows to to obtain a synergistic effect or reduce the other symptoms caused by the virus, in particular coronavirus.

Examples for such further active agents are: ACE inhibitor, angiotensin receptor blaocker, janus knase inhibitor, anti-interleukin durgs, and anti-fibrosis drugs.

The monoclonal antibodies include: monoclonal antibody specific for spike protein of COVID-19, PD-1 blocking antibody and monoclonal antibody against CD14 - IC14 such as , pembrolizumab, nivolumab.

For the treatment against the cytokine storm, ruxolitinib, Lenzilumab TJ003234 (anti-GM-CSF mAb), BMS-986253, leronlimab, Canakinumab can be used.

Further active agents which can be combined with the inventive compounds are: ACE inhibitor, Angiotensin receptor blocker, Angiotensin-converting enzyme inhibitors (ACE-I) and Angiotensin II Receoptor Blockers (ARB) - losartan, losartan potassium, recombinant human angiotensin-converting Enzyme 2 (rhACE2) APN01; Anti-inflammatory drugs such as: indomethacin, Piclidenoson, prednisone; TLR agonist - PUL-042.

For treatment of acute respiratory distress syndrome or failure caused by COVID-19 the following active agents can be administered together with a compound of the present application: Chlorpromazine, sirolimus, dexamethasone, Bromhexine, Nitazoxanide, Levamisole, methylprednisolone (glucocorticoid therapy), Tacrolimus, colchicine, captopril,
and/or
Anti-interleukin (IL-6) drugs such as: bevacizumab, clazakizumab, sarilumab, siltuximab, tocilizumab, Gimsilumab, eculizumab, Anakinra, trimethoprim / sulfamethoxazole,
and/or
immune modulating drugs such as: fingolimod or methotrexate.

For the treatment of the cytokine storm the following active agents could be used together with a compound of the present application: ruxolitinib, Lenzilumab TJ003234 (anti-GM-CSF mAb), BMS-986253, leronlimab, Canakinumab.

Still further active agents could be administered in combination with at least one compound of the present application. Examples for such further active agents are: Pyridostigmine bromide (for the treatment of myasthenia gravis), Kerecis (as oral or nasal spray), chlorhexidine (as oral or nasal spray), Hydrocortisone (for the treatment of sever hypoxia), Vazegepant, Tetrandrine, Tranexamic acid, Isotretinoin.

Still further Kinase inhibitors as active agents could be administered in combination with at least one compound of the present application. Kinase inhibitors: baricitinib, imatinib, Tofacitinib (JAK inhibitor), Acalabrutinib (BTK inhibitor). Baricitinib, Janus kinase inhibitor, showing high affinity for AAK1. Disruption of AAK1 (one of the known regulators of viral endocytosis) could block passage of SARS-CoV-2 to cells and also the intracellular assembly of virus particles. Furthermore, it has the capacity to bind cyclin G-associated kinase, another regulator of endocytosis. Systemic inflammatory response and cytokine production can be limited by inhibiting the JAK-STAT3 pathway. Antiviral properties of Imatinib have been shown in early stages of infection against SARS-CoV and MERS-CoV, phylogenetically related to SARS-CoV2. In addition, it has been linked to reduced inflammation and improved endothelial barrier and pulmonary edema.

Still further active agents could be administered in combination with at least one compound of the present application. Anti-thrombosis agents and anti-fibrosis agents such as: nintedanib, defibrotide (pneumonia); Anticoagulation agents such as: Enoxaparin, heparine, angiotensin-(1,7); PD-1 blocking antibodies such as: monoclonal antibody against CD14 - IC14, Interferon beta-1A, 1B, peginterferon lambda-1a; Colchicine (for reducing myocardinal injury); Valsartan (Diovan), simvastatin.

As supplements vitamin C, vitamin D (cholecalciferol), folic acid, zinc gluconate, 25-OH cholecalciferol, Melatonin, N-acetylcysteine could be present in the pharmaceutical composition.

The pharmaceutical compositions according to the present invention comprise at least one compound according to the present invention. Preferably, the pharmaceutical compositions according to the present invention comprise at least one compound according to the present invention as an active ingredient together with at least one pharmaceutically acceptable (i.e. non-toxic) carrier, excipient and/or diluent.

Optionally, the pharmaceutical composition according to the present invention further comprises at least one active agent selected from antiviral agent, anti-HIV agent, anti-malarial agent, antibacterial agent, and monoclonal antibody as mentioned above.

The pharmaceutical composition according to the present invention is useful for the treatment or prophylaxis of virus infection, preferred, wherein virus is influenza virus, parainfluenza virus or coronavirus. Influenza virus is preferably influenza A virus or influenza B virus, more preferably influenza A. Influenza A virus includes H1N1, H3N2, and H7N9.

Preferred, the pharmaceutical composition according to the present invention is used for the treatment or prophylaxis of of virus infection, preferred, wherein virus is coronavirus, preferrably, coronavirus is coronavirus 229E, SARS, MERS, or COVID-19, more preferrably, COVID-19.

The pharmaceutical compositions of the present invention can be prepared in a conventional solid or liquid carrier or diluent and a conventional pharmaceutically made adjuvant at suitable dosage level in a known way. The preferred preparations are adapted for inhalation or oral application or intraveuous or subcutaneous injections. These administration forms include, for example, pills, tablets, film tablets, coated tablets, capsules, powders and deposits, solutions, microparticles or other inhalative forms using an inhalator or spray or any other device for applying the compound into the airways and preferably deep into the lungs.

The present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

The preferred formulation of the inhalation application for the treatment of respiratory diseases such as COVID-19 are often formulated as dry powders and are delivered using a dry-powder inhaler (DPI). The medicaments are micronized such as to have a respirable aerodynamic diameter which is typically in the region of 0.5 to 10 µm. Such micronized particles tend to be cohesive and have poor flow properties. To increase flowability and dosing accuracy the fine drug particles of respirable size are typically mixed with coarser excipient particles to form an ordered mixture, wherein fine drug particles are attached to the coarser excipient particles.

Thus, the inventive compound as the active ingredient may be in micronized form, i.e. having particle size lower than about 10 µm, for example in the range from about 0.5 to about 10 µm, particularly in the range from about 1 to about 6 µm, such as to be able to deposit target areas in the lungs. Conventional methods, such as milling, can be used to provide the active ingredient in micronized form.

The amount of the active ingredient in the dry powder inhalation composition can vary depending e.g. on the active ingredient and the type of dry powder inhaler used. Generally, the amount of the active ingredient in the dry powder inhalation composition is within the range of 0.02 to 30 %, typically from 0.05 to 10 , more typically from 0.1 to 5 %, per weight of the composition.

The excipient used in the dry powder inhalation composition is a mono- or disaccharide, particularly lactose or mannitol, for example alpha lactose monohydrate. In general, the particle size of the excipient is preferably such that it can be entrained in the air stream but not enter deeply into the lung. However, a small proportion of particles with respirable size (< 10 µm) can be present in the excipient as such fine particles of the excipient may help in attaining higher fine particle dose (FPD) values. The volume median diameter (VMD) of the excipient, such as lactose, to be used in the composition is suitably in the range of, for example, from about 30 to about 150 µm. The excipient of desired VMD can be obtained from commercial sources or can be prepared using methods known in the art such as by blending together excipient powders of known particle size or by sieving.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anticancer activity or activity against cancer metastases and the like or antiviral activity or activity against the spreading of the virus. Suitable dosage forms for sustained release include tablets or inhalative forms having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semisolid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to ca. 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 wgt %.

### Description of Figures

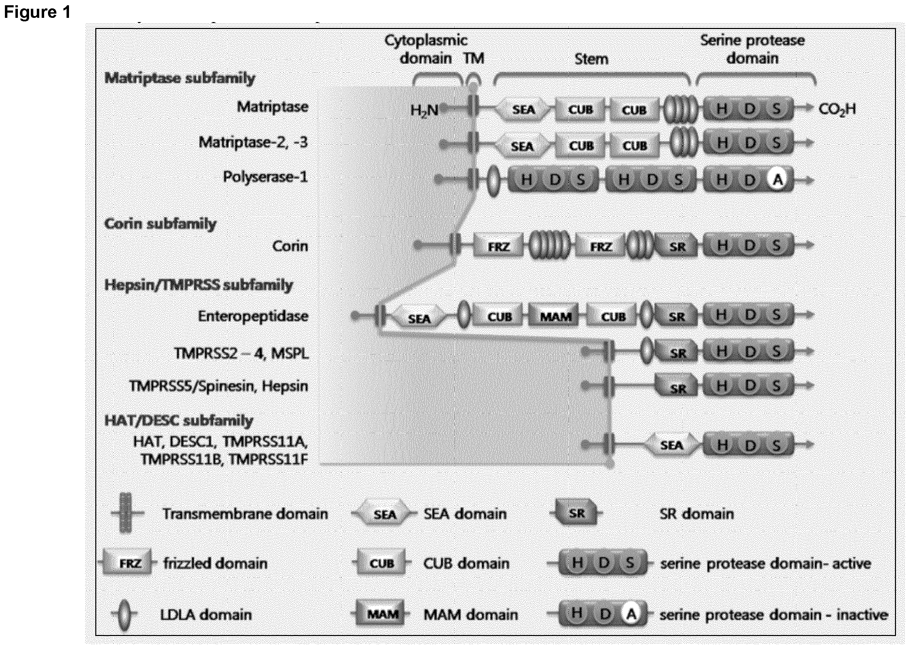

**Figure 1** shows 4 subfamilies of transmembrane serine proteases (17 proteases in total). This Figure was taken from the Atlas of genetics and cytogenetics in oncology and haematology in 2013; Huret JL, Ahmad M, Arsaban M, Bernheim A, Cigna J, Desangles F, Guignard JC, Jacquemot-Perbal MC, Labarussias M, Leberre V, Malo A, Morel-Pair C, Mossafa H, Potier JC, Texier G, Viguié F, Yau Chun Wan-Senon S, Zasadzinski A, Dessen P; Nucleic Acids Res. 2013 Jan;41(Database issue):D920-4; PMID:23161685.

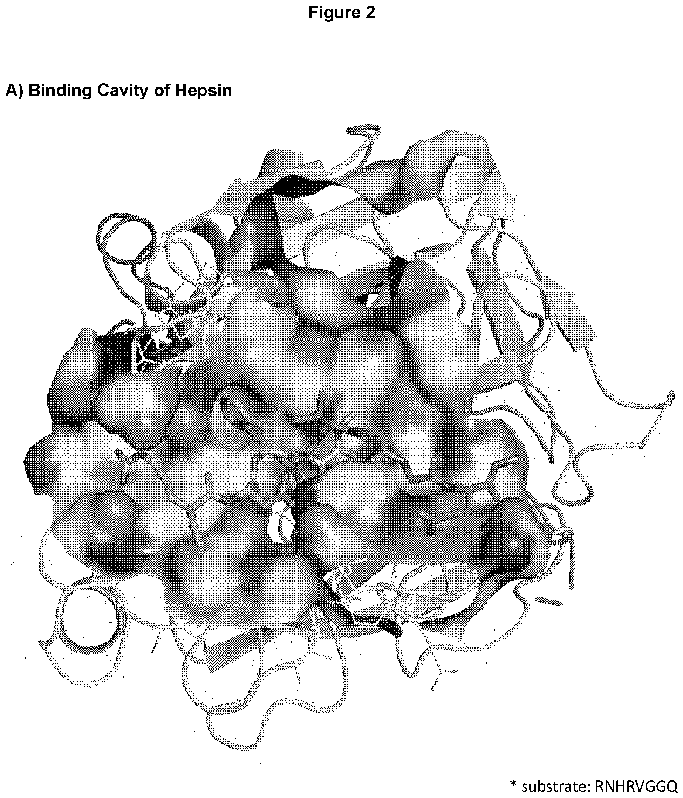

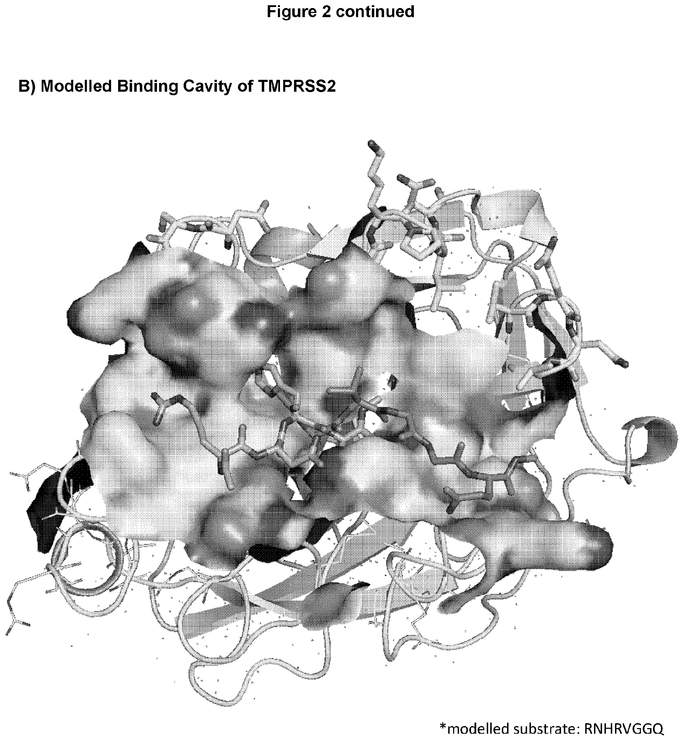

**Figure 2****: A)** a crstal structure of hepsin; **B)** TMPRSS2 model structure. TMPRSS2 belongs to Hepsin/TMPRSS subfamily and thus crystal strucutre of hepsin can provides good templates for TMPRSS2 modelling.

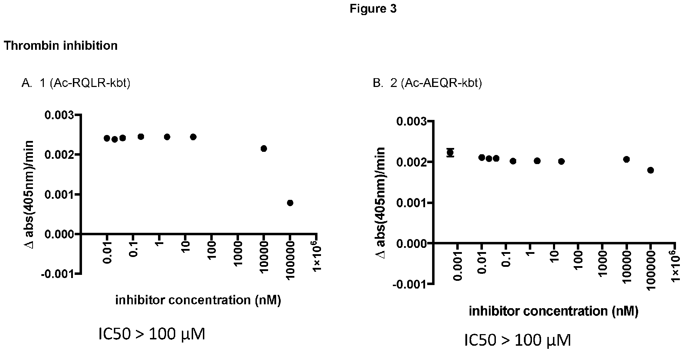

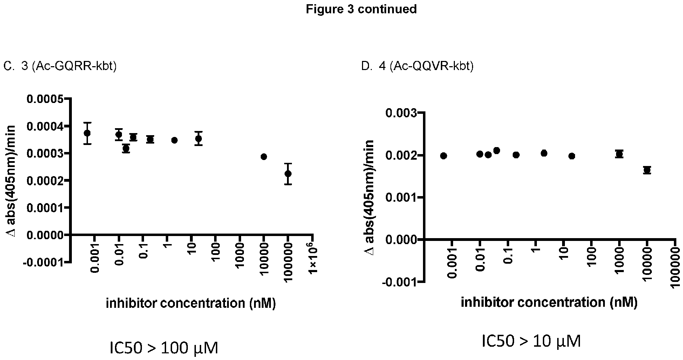

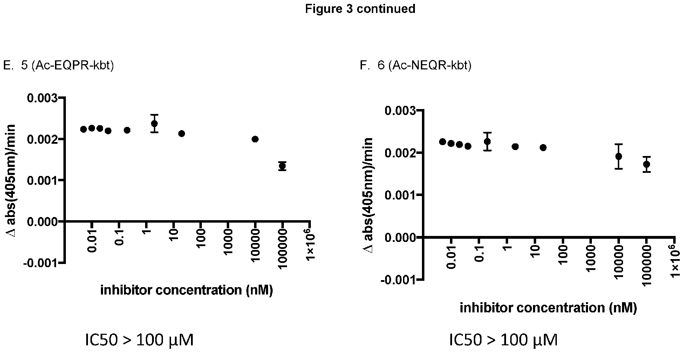

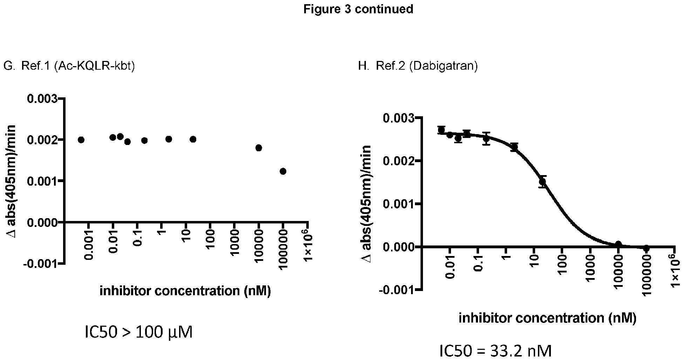

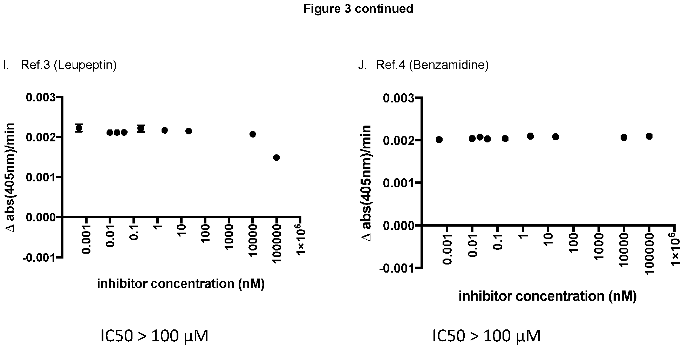

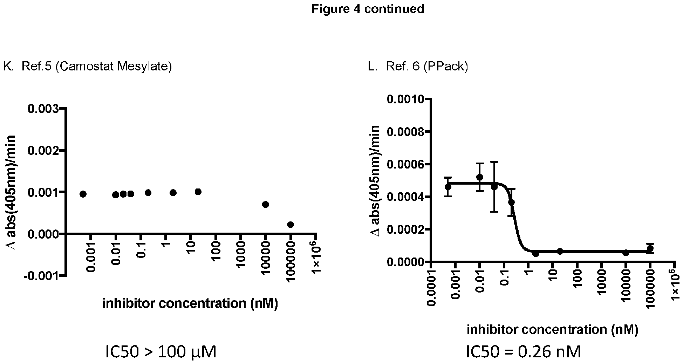

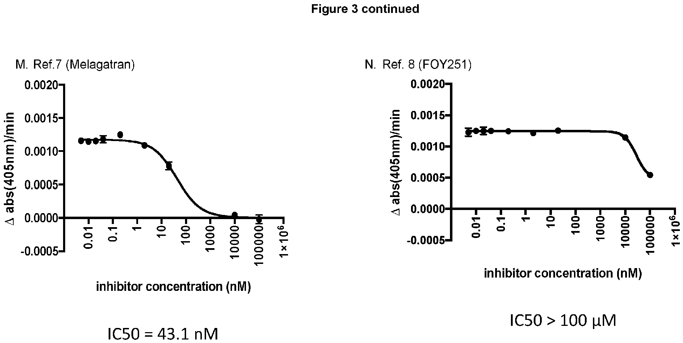

**Figure 3** shows IC₅₀ vaules of the compounds 1 to 6 and reference compound against thrombin.

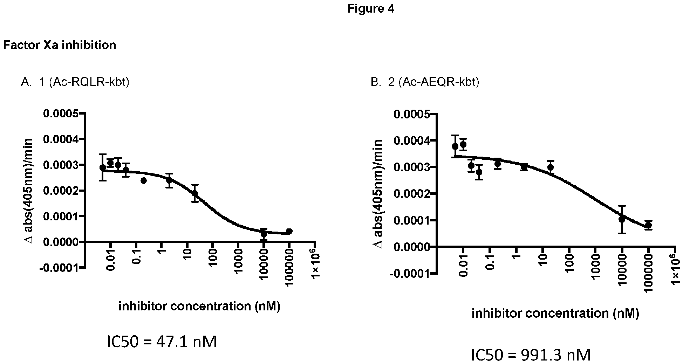

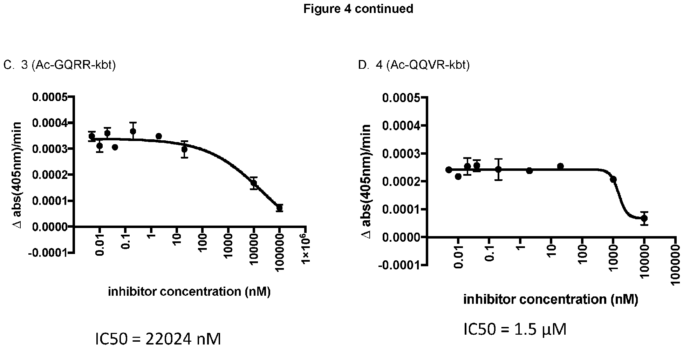

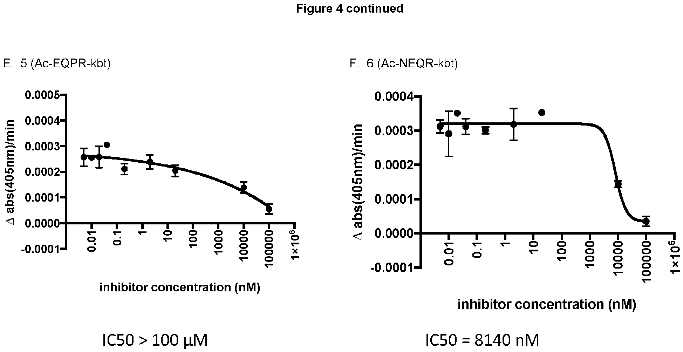

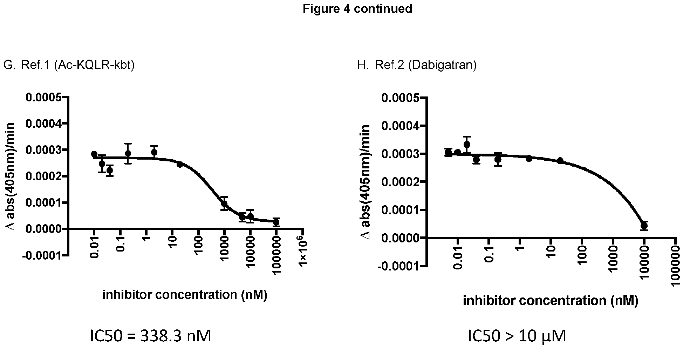

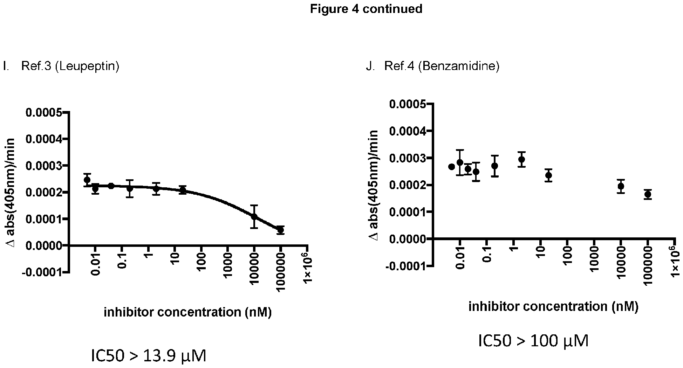

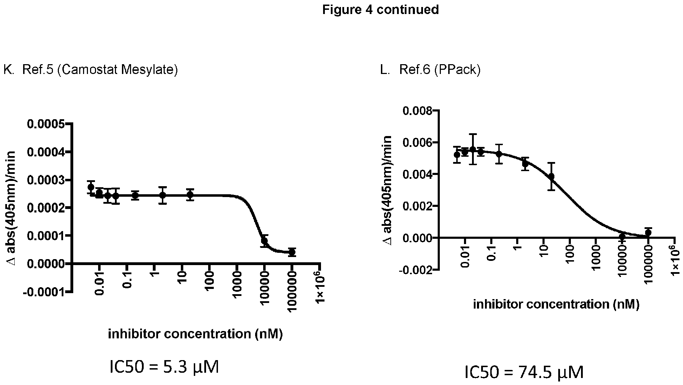

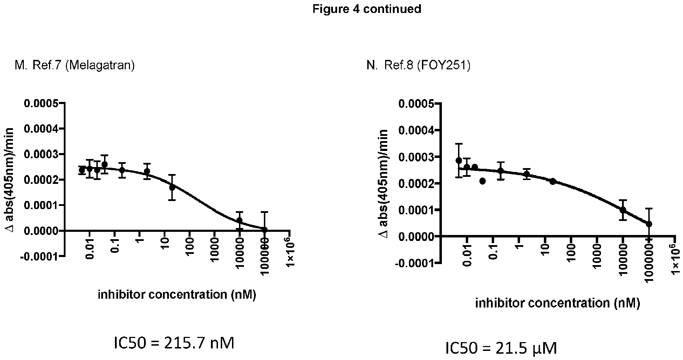

**Figure 4** shows IC₅₀ vaules of the compounds 1 to 6 and reference compound against factor Xa.

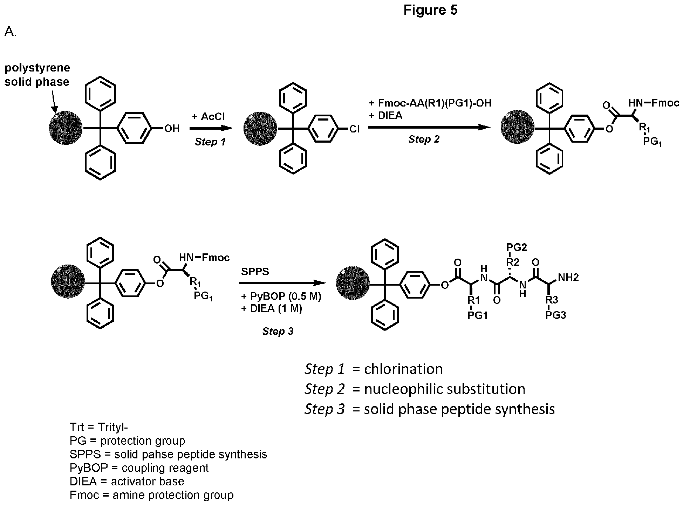

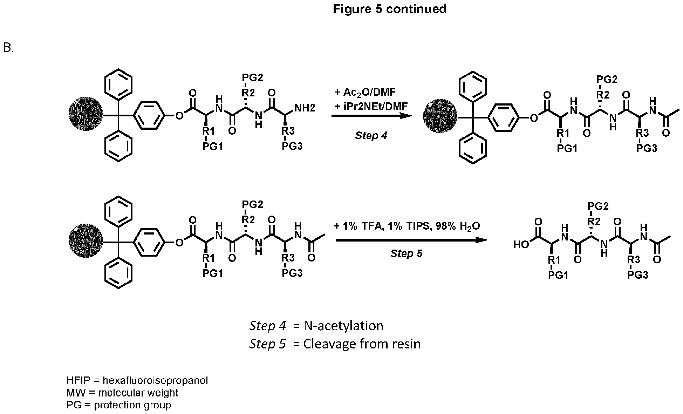

**Figure 5** shows the general synthetic method of the tetrapeptidomimetic compounds.

### Examples

Following abbreviations used in the examples have the following meaning.
- BOC: tert-butyloxycarbonyl
- DIEA: diisopropylethyl amide
- EDCI: ethylcarbodiimide hydrochloride
- HATU: 1-[Bis(dimethylamino)methylene]-1H-1,2,3-triazolo[4,5-b]pyridinium 3-oxid hexafluorophosphate
- Mtr: trimethylbenzenesulfonyl
- Pbf: 2,2,4,6,7-pentamethyldlhydrobenzofuran-5-sulfonyl
- PyBOP: benzotriazol-1-yl-oxytripyrrolidinophosphonium hexafluorophosphate

### Chemical Examples

The following examples are intended to illustrate the invention with selected compounds without limiting the protecting scope of the present intellectual property right on these concrete examples. It is clear for a person skilled in the art that analogous compounds and compounds produced according to analogous synthetic ways fall under the protecting scope of the present intellectual property right.

### Example 1. Synthesis of arginine modified serine trap with mtr protected guanidino group (serine trap precursor)

### 1.1 Synthesis of arginine weinreb amide*

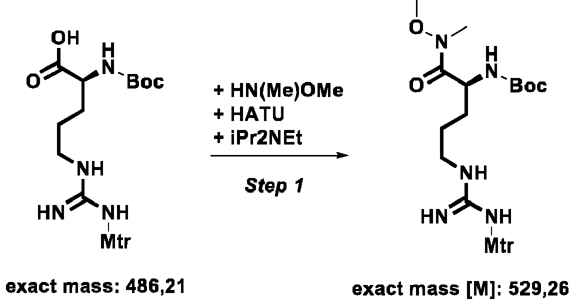

Boc-NH-Arg(Mtr)-OH (1.14g, 2.34 mmol, 1.0 equiv), N,O-dimethylhydroxyl-amine hydrochloride (460 mg, 4.72 mmol, 2.0 equiv), and 1-hydroxybenzotriazole [HOBt] (350 mg, 2.59 mmol, 1.1 equiv) were dissolved in 25 mL THF at room temperature. N, N-diisopropylethylamine [DIEA] (1.22 mL, 7.00 mmol, 3 equiv) was added via syringe and the solution was stirred until it was homogeneous. 1-(3-dimethylaminopropyl)-3-ethylcarbodiimide hydrochloride [EDCI] (470 mg, 2.45 mmol, 1.05 equiv) was added in one portion and the reaction was allowed to stir at room temperature for 4.5 h. THF may be first removed in vacuo, then the reaction mixture is diluted with ethyl acetate (150 mL), washed with 5% aqueous acetic acid (1 × 75 mL), sat. aqueous NaHCO₃ (1 × 75 mL), water (1 × 75 mL), and brine (1 × 75 mL). The organic layer was dried over MgSO4, filtered and concentrated in vacuo to afford the title Weinreb amide. Mass = 858 mg, yield: 69%, purity > 95%
Scientific name: (S)-tert-butyl-1-(methoxy(methyl)amino)-5-(3-(4-methoxy-2,5 dimethylphenylsulfonyl)guanidino)-1-oxopentan-2-ylcarbamate.
**1H NMR** (300 MHz, CD3OD): d=1.43 (s, 9 H) 1.45-1.67 (m, 4 H) 1.87 (s, 1 H) 2.13 (s, 3 H) 2.61 (s, 3 H) 2.67 (s, 3 H) 3.17 (m, 3 H) 3.74 (s, 3 H) 3.90 (s, 3 H) 6.67 ppm (s, 1 H);
**MS(ESI):** found: [M+H]⁺ = 530.2, [M+H+DIEA]⁺ = 659.4, [2M+H]⁺ = 1059.4

### 1.2 Synthesis of Boc-Arg(Mtr) ketobenzothiazole Weinreb-Nahm ketone synthesis*

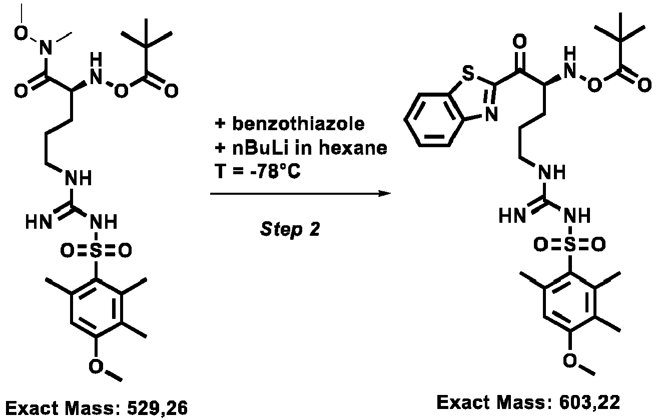

At -78,8°C, nBuLi in hexane (1.6 M) (21.88 mL) was added dropwise into the solution of benzothiazole (0.8 g, 1.52 mmol) in THF (50 mL) over 15 min. After the mixture was stirred for an additional 30 min, the solution of Boc-Arg(Mtr) Weinreb amide (0.8 g, 1.52 mmol) in THF (15 mL) was added slowly over 50 min. The mixture was stirred at - 78,8°C for 3h. The reaction was quenched with aqueous NH₄Cl and the aqueous layer was extracted with EtOAc. The organic phase was collected, dried with Na₂SO₄, and then concentrated. The resulting residue was purified by silica gel chromatography with CHCl₂/MeOH combination as eluent to give the title compound.
**1H NMR** (300 MHz, CD3OD): d=1.43 (s, 9 H) 1.53-1.78 (m, 4 H) 2.05 (s, 3 H) 2.55 (s, 3 H) 2.61 (s, 3 H) 3.17-3.29 (m, 2 H) 3.82 (s, 3 H) 5.19-5.43 (m, 1 H) 6.57 (s, 1 H) 7.53-7.71 (m, 2 H) 8.06-8.28 ppm (m, 2 H).
**MS(ESI):** found: [M+H]⁺, 603.8, [2M+H]⁺ = 1206.5

### 1.3 Synthesis of HCl·H-Arg(Mtr) ketobenzothiazole

To 100 mg of Boc-Arg(Mtr) ketobenzothiazole, a solution of 5 ml (1.5 M HCl/Dioxane) was added. The mixture was stirred at room temperature for 18h. The solvent was removed the resulting residue was dried in vacuo. 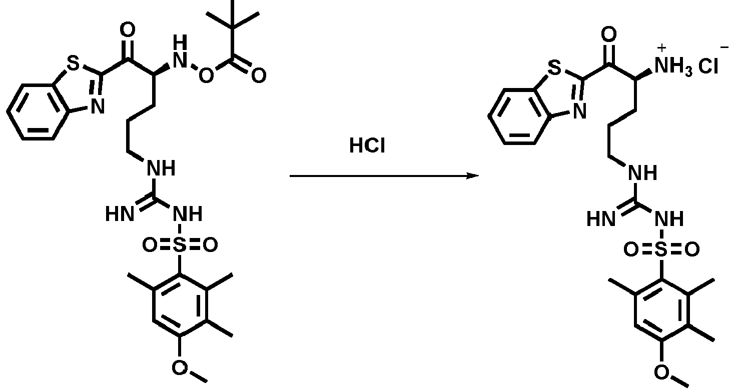 **1H NMR** (300 MHz, CD3OD): d=1.60-1.86 (m, 2 H) 2.00-2.20 (m, 4 H) 2.24-2.36 (m, 1 H) 2.53 (s, 3 H) 2.61 (s, 3 H) 3.84 (s, 3 H) 5.14-5.40 (m, 1 H) 6.65 (s, 1 H) 7.59-7.78 (m, 2 H) 8.10-8.34 ppm (m, 2 H).
**MS(ESI):** found: [M +H]⁺ = 502.6, [2M+H]⁺ = 1006.5

### 1.4 Purification and lyophilization of serine trap precursor

All compounds were purified with semi-preparative HPLC. The following gradient was applied: 95%H₂O/5%ACN to 5%H₂O/95%ACN in 30 minutes. Column used: Zobrax Eclipse XDB C-18 9.4x250 mm 5µm, company: Agilent Technologies. Detector: UV Vis detector model S-3702, company: Soma. For the detection of the compounds a wavelength of 220 nm was used. After purification with HPLC, the fractions were collected and freeze dried overnight. The purified and lyophilized compounds were stored in the freezer at -20°C. The mass of the purified compounds was determined with MS-ESI. Model used: expression-L compact mass spectrometer, company Advion.

Peptides were dissolved to a concentration of c = 0.01 mg/ml in MeOH + 0.1% formic acid. Injection was done by a syringe pump with a flow rate of 10 µl/min.

### Data analysis

- MS ESI data were analyzed with the program Advion Mass/Data express
- HPLC data were analyzed with Graph Pad Prism 7

### Example 2. Synthesis of tripeptides with protected side chains, acetylated N-terminus and free carboxyl group (tripeptide precursors)

### 2.1 Chlorination of tritylhydroxid resin (Trt-OH)*

A 20 ml glass vial was rinsed out with dry DCM. 5 g of Trt-OH (1.33 mmol/g, mesh 100-200, company: Iris Biotech) was weighed and put into glas vial. The resin was suspended in a mixture of 50% DCM and 50% toluene, just enough to double the resin volume and 2 ml acetyl chloride (30 eq) was added. The glass vial was sealed and agitated for 24 h. The next day, the resin was dried and thoroughly washed with DCM (4 x 5 ml). Resin was stored in freezer.

### 2.2 Addition of first amino acid to Trt-CI

10 ml of dry DCM were added to 200 mg Trt-CI (subst. 1.33 mmol/g) and shaken for 10 min. 10 eq. (2.66 mmol) of amino acid was dissolved and 20 eq. (5.32 mmol) of activator base was added. The coupling reaction was shaken for 4 h at room temperature. After the reaction, the resin was washed with DMF (2 x 5 ml) and DCM (2 x 5 ml). The resin was swollen in 10 ml DMF for 1h prior to use for solid phase peptide synthesis.

### 2.3 Solid phase peptide synthesis

### Fmoc protected amino acids:

All fmoc protected amino acids were purchased from Carl Roth / Novabiochem / Merck and dissolved in DMF to a concentration of c = 0.2 M.

### Deprotection solution for fmoc group:

20% Piperidine in DMF (peptide synthesis grade)

### Coupling reagents:

Activator PyBOP (Company: Merck) was dissolved in DMF to a concentration of 1 M and the activator base diisopropylethyl amide (DIEA) was dissolved in DMF to a concentration of 0.5M.

### Peptide synthesizer:

Microwave assisted peptide synthesizer from the company CEM. Model: Liberty.
The standard procedure for solid phase peptide synthesis was used with slight modifications to couple two additional amino acids to the loaded resin. Please see the attached synthesis protocols for the respective experiment.

### Conditions for Fmoc deprotection:

| **step** | **temperature** | **power (microwave)** | **time** |
|---|---|---|---|
| **1.** | 75 °C | 35 Watt | 30 s |
| **2.** | 75 °C | 40 Watt | 180 s |

### Conditions for coupling reaction for all amino acids except Fmoc-His(Trt)-OH, Fmoc-Cys(Trt)-OH and Fmoc-Arg(Pbf)-OH:

| **temperature** | **power (microwave)** | **time** |
|---|---|---|
| **75 °C** | 23 Watt | 600 s |

### Conditions for coupling reaction Fmoc-HisTrt-OH and Fmoc-Cys(Trt)-OH:

| **step** | **temperature** | **power (microwave)** | **time** |
|---|---|---|---|
| **1.** | 50 °C | 0 Watt | 120 s |
| **2.** | 50 °C | 25 Watt | 600 s |

### Conditions for coupling reaction Fmoc-Arg(Pbf)-OH:

| **step** | **temperature** | **power (microwave)** | **time** |
|---|---|---|---|
| **1** | 75 °C | 0 Watt | 25 min |
| **2** | 75 °C | 25 Watt | 5 min |

### 2.4 Acetyl capping of the tripeptides*

Acetyl capping solution: 0.5 M Ac₂O/DMF and 1 M iPr2NEt/DMF
200 mg of tripeptide resin was suspended in 20 ml acetyl capping solution. The mixture was shaken at RT for 1 h. The resin was filtered and washed with DMF (3 x 5 ml) followed by DCM (3 x 5 ml) *(*ChemMedChem, 2016, 11(6), 685)

### 2.5 Cleavage of tripeptide from resin**

Cleavage solution: 20% Hexafluoroisopropanol (HFIP) in CH₂Cl₂) 20 ml of cleavage solution was freshly prepared (6 ml HFIP / 24 ml DCM) and added to 200 mg resin. Suspension was put on orbital shaker for 3h and then filtered. The solvent was taken off with rotovap.

### 2.6 Purification and lyophilization of tripeptide precursors

All tripeptide precursors were purified with semi-preparative HPLC. The following gradient was applied: 95%H₂O/5%ACN to 5%H₂O/95%ACN in 30 minutes. Column used: Zobrax Eclipse XDB C-18 9.4x250 mm 5µm, company: Agilent Technologies. Detector: UV Vis detector model S-3702, company: Soma. For the detection of the peptides a wavelength of 220 nm was used. After purification with HPLC, the fractions were collected and freeze dried overnight. The purified and lyophilized tripeptide precursors were stored in the freezer at -20°C. The mass of the purified compounds was determined with MS-ESI. Model used: expression-L compact mass spectrometer, company Advion. Peptides were dissolved to a concentration of c = 0.01 mg/ml in MeOH + 0.1% formic acid. Injection was done by a syringe pump with a flow rate of 10 µl/min.

### Data analysis

- MS ESI data were analyzed with the program Advion Mass/Data express
- HPLC data were analyzed with Graph Pad Prism 7

**Table A. Synthesized tripeptide precursors with highest calculated score values of P-sid library**

| compound | | P4 | P3 | P2 | | score of related tetrapeptides [kJ/mol] (Rank) | MS data |
|---|---|---|---|---|---|---|---|
| **1a** RSi precursor | Ac-N | R (Pbf) | Q (Trt) | L | -CO₂H | -60.1 (175) | m/z = 952 [M+H]⁺ |
| P1ip precursor | Ac-N | R (Pbf) | E (OtBu) | C (Trt) | -CO₂H | -73.1 (1) | m/z = 999 [M+H]⁺ |
| **2a** P2ip precursor | Ac-N | A | E (OtBu) | Q (Trt) | -CO₂H | -72.7 (2) | m/z = 709 [M+Na]⁺ |
| **3a** P3ip precursor | Ac-N | G | Q (Trt) | R (Pbf) | -CO₂H | -72.7 (3) | m/z = 897 [M+H]⁺ |
| P4ip precursor | Ac-N | R (Pbf) | N (Trt) | H (Trt) | -CO₂H | -71.6 (4) | m/z = 1204 |
| | | | | | | | [M+H]⁺ |
| **4a** P5ip precursor | Ac-N | Q (Trt) | Q (Trt) | V | -CO₂H | -69.5 (5) | m/z = 889 [M+H]⁺ |
| P6ip precursor | Ac-N | R (Pbf) | H (Trt) | Q (Trt) | -CO₂H | -69.1 (6) | m/z = 633 [M+H]⁺ |
| **5a** P7ip precursor | Ac-N | E (OtBu) | Q (Trt) | P | -CO₂H | -68.9 (7) | m/z = 713 [M+H]+ |
| **6a** P8ip precursor | Ac-N | N (Trt) | E (OtBu) | Q (Trt) | -CO₂H | -67.6 (8) | m/z = 972 [M+H]⁺ |
| **NSip^{a}** precursor | Ac-N | K (Boc) | Q (Trt) | L | -CO₂H | - | m/z = 769 [M-H]⁻ |
| **Rsip^{b}** precursor | Ac-N | R (Pbf) | Q (Trt) | L | -CO₂H | -60.1 (175) | m/z = 952 [M+H]⁺ |
| **NCip^{c}** precursor | Ac-N | A | A | A | -CO₂H | -45.0 (2475) | |
| **SAip^{d}** precursor | Ac-N | S (OtBu) | P | R (Pbf) | -CO₂H | | m/z = 709 [M+H]⁺ |

| | | | | | | | |
|---|---|---|---|---|---|---|---|
| *^{a}* natural substrate of hepsin, *^{b}* reference sequence of known substrate, *^{c}* negative control. ^{d} Spike protein recognition sequence of TMPRSS2 * purity >99%, lyophilized | | | | | | | |

**Table B. MS data of the compounds of the invention**

| compound | sequence | structure | MS data |
|---|---|---|---|
| **1** RSip peptide | Ac-RQLR-kbt | 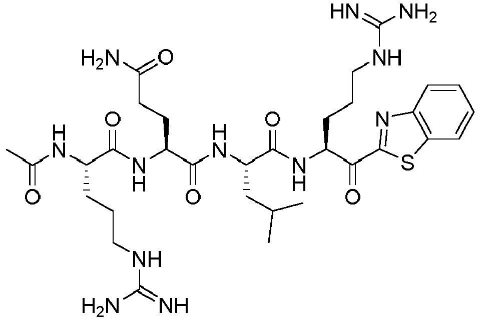 | m/z = 366 [M+2H]²⁺ |
| **2** P2ip peptide | Ac-AEQR-kbt | 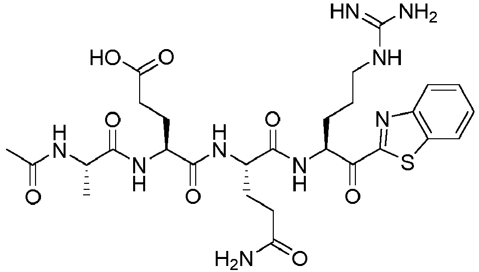 | m/z = 662 [M+H]+ |
| **3** P3ip peptide | Ac-GQRR-kbt | 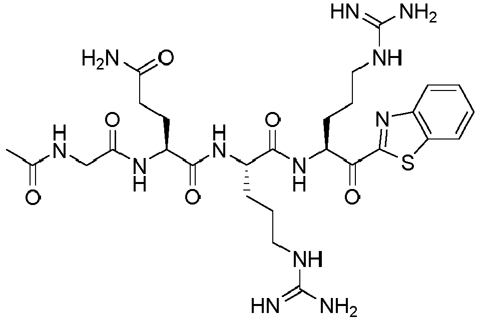 | m/z = 338 [M+2H]²⁺ |
| **4** P5ip peptide | Ac-QQVR-kbt | 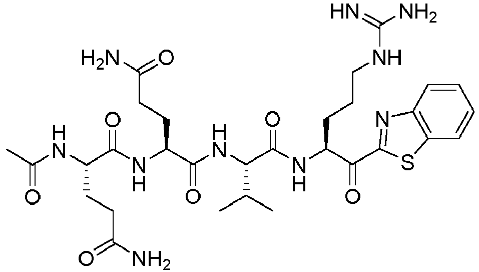 | m/z = 689 [M+H]+ |
| **5** P7ip peptide | Ac-EQPR-kbt | 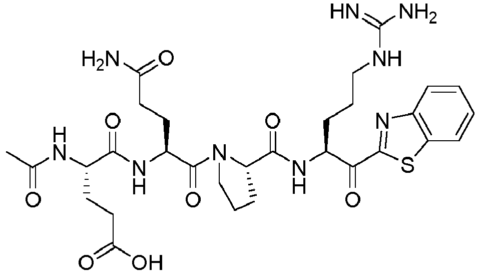 | m/z = 688 [M+H]+ |
| **6** P8ip peptide | Ac-NEQR-kbt | 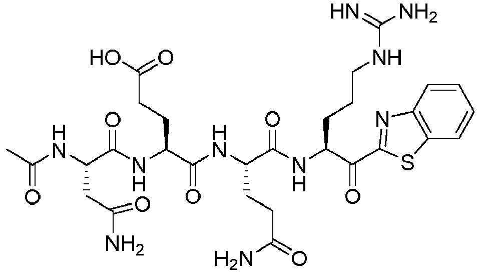 | m/z = 705 [M+H]+ |
| **Ref. 1** NSi peptide | Ac-KQLR-kbt | 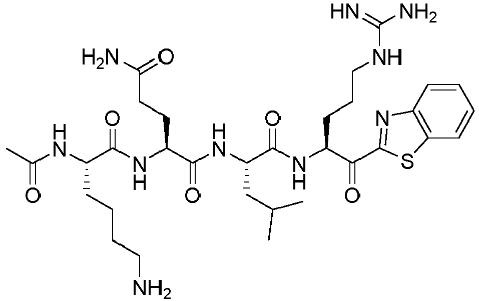 | m/z = 702 [M+H]+ |

### Biological Examples

### Example B-1. Inhibitory activity of the inventive compounds against thrombin

Protease Thrombin inhibiton assay - Calculation of IC₅₀ values

### Materials

Protease: Recomb. Human Thrombin, Bought 30.01.2020; Art. 1473-SE-010;
Company: R&D systems
Reference substrate: D-Phe-Homopro-Arg-pNA; Company: Bachem; Art no.: 4008145.0050

### Plates for plate reader:

Greiner 96 well plates, PS, F-Bottom (Chimney well), µclear, black, cellstar. Cat. No.: 655090/655096/655097
Tubes: Eppendorf, Protein LoBind Tube 1.5ml/2ml. Cat. No.: 022431081

### Platereader:

Device: Infinite M1000
Application: Tecan i-control
Firmware: V_2.09_04/2011_S3LCE (May 2 2011/09.25.56)

### Preparation of solutions

### 1. Preparation of Thrombin solution:

- Reconstitute at 100 µg/ml (1428.57 nM) in sterile 50 mM HEPES and 640 mM NaCl
- Diluted to a stock solution c = 0.04 µg/µl with TNC buffer

### 2. Preparation of reference substrate solution:

- Dilute and aliquote 2.1 mg of reference substrate to a concentration of c = 2000 µM in TNC. Dilute and aliquote further to a concentration of c = 200 µM in TNC buffer. Endconcentration in well: c = 100 µM.

### 3. Preparation of inhibitor solutions:

• Inhibitor was diluted with pure DMSO to a concentration of 10 mM
• This 10 mM stock solution was further diluted:

| concentration of inhibitor (stock solution) | concentration of inhibitor in well* |
|---|---|
| c = 10 mM | c = 100 µM |
| c = 1 mM | c = 10 µM |
| c = 2 µM | c = 20 nM |
| c = 200 nM | c = 2 nM |
| c = 20 nM | c = 0.2 nM |
| c = 4 nM | c = 40 pM |
| c = 2 nM | c = 20 pM |
| c = 1 nM | c = 10 pM |
| c = 0.5 nM | c = 5 pM |

| | |
|---|---|
| *Total volume per well = 100 µl, so the dilution factor of the stock solutions is 100x | |

### 2. Preparation of TNC buffer:

- 25 mM Tris, 150 mM NaCl, 5 mM CaCl₂, 0,01% Triton X-100, pH = 8 (stored at 8°C)

### Platereader setup

- Total volume per well: 100µl
- Temperature: 25°C
- Kinetic interval: every 2 minutes
- Absorption: 405 nm
- Number of flashes: 25
- Shaking before measurememt: 3s
- Shaking amplitude: 5 mm

### Experimental setup

- Per well: add 50 µl of Thrombin/TNC solution (c = 0.04 µg/µl), add 1 µl of inhibitor.
- Incubate for 30 minutes at room temperature
- After incubation, add 1 µl of c = 200 µM reference substrate
Endconcentration of substances per well: c = 100 µM of reference substrate, c = 0.002 µg of Factor Xa solution, 1% DMSO

### Data analysis

- Graph pad prism 7 was used to analyze the data. The velocity (change of absorbance over time) was plotted against the concentration. A non linear regression was performed with the following equation: [inhibitor] vs. response - Variable slope (four parameters)

### Example B-2. Inhibitory activity of the inventive compounds against Factor Xa

Protease Factor Xa inhibiton assay - Calculation of IC₅₀ values

### Materials

### Protease:

Recomb. Human Coag. Factor Xa protein, CF: Bought 09.12.2020; Art. 1063-SE-010;
Company: Bio-Techne GmbH

### Reference substrate:

Bz-Ile-Glu-Gly-Arg-pNA; Company: Bachem; Art no.: 4003874.00051000025708

### Plates for plate reader:

Greiner 96 well plates, PS, F-Bottom (Chimney well), µclear, black, cellstar. Cat. No.: 655090/655096/655097
Tubes: Eppendorf, Protein LoBind Tube 1.5ml/2ml. Cat. No.: 022431081

### Platereader:

Device: Infinite M1000
Application: Tecan i-control
Firmware: V_2.09_04/2011_S3LCE (May 2 2011/09.25.56)

### Preparation of solutions

### 1. Preparation of Factor Xa solution:

- Reconstitute at 100 µg/ml in sterile 25 mM MES, 150 mM NaCl, 5 mM CaCl2, pH6
- Diluted to a stock solution of 1000 nM with TNC buffer
- This stock solution was further diluted with TNC buffer to a concentration of 35 nM and aliquoted. Stored at -20°C. Endconcentration in well: c = 0.35 nM

### 2. Preparation of reference substrate solution:

- Delivered: 5mg. Dilute and aliquote reference substrate to a concentration of 10 mM in pure DMSO. Endconcentration in well: c = 200 µM (= K_{M} for Factor Xa)

### 3. Preparation of inhibitor solutions:

• Inhibitor was diluted with pure DMSO to a concentration of 10 mM
• This 10 mM stock solution was further diluted:

| concentration of inhibitor (stock solution) | concentration of inhibitor in well* |
|---|---|
| c = 10 mM | c = 100 µM |
| c = 1 mM | c = 10 µM |
| c = 2 µM | c = 20 nM |
| c = 200 nM | c = 2 nM |
| c = 20 nM | c = 0.2 nM |
| c = 4 nM | c = 40 pM |
| c = 2 nM | c = 20 pM |
| c = 1 nM | c = 10 pM |
| c = 0.5 nM | c = 5 pM |

| | |
|---|---|
| *Total volume per well = 100 µl so the dilution factor of the stock solutions is 100x | |

### 2. Preparation of TNC buffer:

- 25 mM Tris, 150 mM NaCl, 5 mM CaCl₂, 0,01% Triton X-100, pH = 8 (stored at 8°C)

### Platereader setup

- Total volume per well: 100µl
- Temperature: 25°C
- Kinetic interval: every 2 minutes
- Absorption: 405 nm
- Number of flashes: 25
- Shaking before measurememt: 3s
- Shaking amplitude: 5 mm

### Experimental setup

- Per well: add 97 µl TNC buffer, add 1 µl of c = 35 nM solution of Factor Xa, add 1 µl of inhibitor
- Incubate for 30 minutes at room temperature
- After incubation, add 1 µl of c = 10 mM reference substrate
Endconcentration of substances per well: c = 200 µM of reference substrate, c = 0.35 nM of Factor Xa solution, 2% DMSO

### Data analysis

Graph pad prism 7 was used to analyze the data. The velocity (change of absorbance over time) was plotted against the concentration. A non linear regression was performed with the following equation: [inhibitor] vs. response - Variable slope (four parameters)

**Table 1. IC₅₀ values of inventive inhibitors with respect to TMPRSS2, thrombin and Factor Xa**

| compound | sequence | IC₅₀ TMPRSS2 [nM] | IC₅₀ Thrombin [nM] | IC₅₀ FXa [nM] |
|---|---|---|---|---|
| **1** RSi peptide** | Ac-RQLR-kbt | 1,263 | >100,000 | 47.1 |
| **2** P2i peptide | Ac-AEQR-kbt | 1,670 | >100,000 | 991.3 |
| **3** P3i peptide | Ac-GQRR-kbt | 332.5 | >100,000 | 22,024 |
| **4** P5i peptide | Ac-QQVR-kbt | 500.3 | >10,000 | 1,500 |
| **5** P7i peptide | Ac-EQPR-kbt | 2,871 | >100,000 | >100,000 |
| **6** P8i peptide | Ac-NEQR-kbt | 1,186 | >100,000 | 8140 |
| **7** P1 i peptide | Ac-RECR-kbt | 835.1 | >100,000 | 611 |
| **Ref.1** NSi peptide* | Ac-KQLR-kbt | 3430 | >100,000 | 338.3 |
| **Ref.2** Dabigatran | - | >100,000 | 33.2 | >10,000 |
| **Ref.3** | - | >27,600 | >100,000 | >13,900 |
| Leupeptin | | | | |
| **Ref.4** Benzamidine | - | >100,000 | >100,000 | >100,000 |
| **Ref.5** Camostat mesylate | - | 943.6 | >100,000 | 5,300 |
| **Ref.6** PPack | - | >100,000 | 0.268 | 74.5 |
| **Ref.7** Melagatran | - | - | 43.1 | 215.7 |
| **Ref.8** FOY 251**** | - | - | >100,000 | 21,500 |

In vitro results of synthesized tetrapeptide inhibitors with ketobenzothiazol warhead. with highest calculated score values of P-side library. Inhibitors were screened against TMPRSS2, thrombin and factor Xa. IC₅₀ values were calculated from nonlinear regressions with the following equation: [Inhibitor] vs. Response - Variable slope (four parameters) usig the program Prism 7
* NS : natural substrate (P-site from hepatocyte growth factor)
** RS : reference substrate (P-site from preferred substrate for hepsin)
*** SA : SARS CoV-2 S Protein-TMPRSS2 recognition sequence
**** FOY251 : active form of Camostate mesylate

### Example B-3. Inhibitory activity against a pseudotyped particle LV(Luc)-CoV2 in CaCo-2 cells

**Generation of lentiviral SARS-CoV-2 pseudoparticles** All cells were cultured in a humidified incubator at 37 °C, 5 % CO₂. To generate lentiviral pseudoparticles harboring the SARS-CoV-2 Spike (termed LV(Luc)-CoV-2-S), 900 000 HEK293T (human embryonal kidney) cells were seeded in 2 ml of Dulbecco's Modified Eagle Medium (DMEM, Gibco) supplemented with 10 % FCS (Gibco), 2 mM glutamine, 100 U/ml Penicillin and 100 µg/ml Streptomycin (Pan Biotech) in 6-well plates. The next day, medium was refreshed and cells were transfected using Polyethyleneimine (PEI) transfection reagent. A total of 1 µg DNA comprising 2 % pCG1-SARS-2-S (encoding SARS-CoV-2 Spike of isolate Wuhan-HU-1, NCBI Reference Sequence: YP_009724390.1) (Hoffmann M, et. al., Cell, 2020, 181:271-280.e8), pSEW-luc2 (a crippled lentiviral vector expressing luciferase (Abel T, et. al., Blood, 2013, 122:2030-2038; Demaison C, et. al., Hum Gene Ther., 2002, 13:803-813; Münch RC, et. al., Mol Ther, 2011, 19:686-693.) and pCMVdR8.91 (a Gag-Pol expression plasmid) (Romain Zufferey, et. al., Nat. Biotechnol., 1997, 3:3-8) at a 1:1 ratio was mixed in 1.5 ml serum reduced medium (Opti-MEM, Gibco). PEI was added to the DNA at a PEI:DNA ratio of 3:1. The mixture was briefly vortexed, incubated for 20 min at RT and added dropwise to the cells. At 8 h post transfection, cells were washed with PBS and 2 ml of DMEM supplemented with 2.5 % FCS, 2 mM glutamine, 100 U/ml Penicillin and 100 µg/ml Streptomycin were added. At 48 h post transfection, pseudoparticle containing supernatants were collected and clarified by centrifugation at 1500 rpm for 5 min.

For transduction experiments in presence of TMPRSS2 inhibitors, 10 000 Caco2 (human colon) cells were seeded in 100 µl of DMEM supplemented with 10 % FCS (Gibco), 2 mM glutamine, 100 U/ml Penicillin, 100 µg/ml Streptomycin, 1x non-essential amino acids (NEAA) and 1 mM sodium-pyruvate (ThermoFischer) in 96-well flat-bottom plates. The next day medium was removed and replaced by 60 µl fresh medium in the presence of 20 µl TMPRSS2 inhibitors, EK1 peptide inhibitor control (Xia S, *et. al.*, 2019, A pan-coronavirus fusion inhibitor targeting the HR1 domain of human coronavirus spike. *Sci Adv* 5:eaav4580.) or equivalent volumes of DMSO solvent control. After 2 h of incubation, cells were transduced with 20 µl of fresh, infectivity normalized LV(Luc)-CoV-2-S. At 48 h post transduction, transduction rates were assessed by measuring luciferase activity of cell lysates using a commercially available kit (Promega). Briefly, cells were washed with PBS and incubated with 40 µl cell culture lysis reagent for 10 min at RT. 30 µl of lysates were transferred to opaque 96-well plates and mixed with 50 µl of Luciferase assay substrate. Luminescence was recorded immediately for 0.1 s/well in an Orion II Microplate luminometer (Berthold). Luciferase activities in absence of inhibitors were set to 100 % and IC₅₀s were determined by linear regression using GraphPad Prism version 8.4.2.

LV(Luc)-CoV2 is based on an infection-deficient lentiviral backbone that bears SARS-CoV2 spike protein, furthermore, it harbors a luciferase reporter gene as readout of infection .

### Protocol:

- day 0: seed 10 000 Caco2 cells per well in 100 µl Caco medium in a 96 F-well plate
- day 1: remove medium from cells and add 60 µl of Caco medium
- dilute inhibitors (stock 10 mM) to 100 µM and titrate in 1:5 steps
- add 20 µl inhibitors to cells in duplicates and incubate for 2 h at 37 °C
- add 20 µl of LV(Luc)-CoV2 pseudoparticle
- at 4 h post infection: measure luciferase activity

### Caco Medium:

| | ml |
|---|---|
| DMEM | |
| 10 % FCS | 50 |
| 1 % P/S | 5 |
| 2 mM Gln | 5 |
| 1 x NEAA | 5 |
| 1 x NaPyruvate | 5 |

The inventive compounds effectively inhibit the infection of CaCo-2 cells casued by the LV(Luc)-CoV2 which is based on an infection-deficient lentiviral backbone that bears SARS-CoV2 spike protein (see Table 2).

All of the inventive compound as TMPRSS2 inhibitors effectively LV(Luc)-CoV2-S infection in a dose-dependent manner. In particular Compound 2p2i shows similar activity to camostatmesylate. Furthermore, by microscopy: no apparent cytotoxicity of the inventive compound was observed.

**Table 2. IC₅₀ values of the inventive compounds against a pseudotyped particle LV(Luc)-CoV2 in CaCo-2 cells**

| compound | sequence | IC₅₀ [nM] |
|---|---|---|
| **1** (RSi peptide)** | Ac-RQLR-kbt | 803.5 |
| **2** (P2i peptide) | Ac-AEQR-kbt | 470.3 |
| **3** (P3i peptide) | Ac-GQRR-kbt | 1,299 |
| **4** (P5i peptide) | Ac-QQVR-kbt | 2,046 |
| **5** (P7i peptide) | Ac-EQPR-kbt | 2,101 |
| **6** (P8i peptide) | Ac-NEQR-kbt | 1,401 |
| **7** (P1i peptide) | Ac-RECR-kbt | - |
| **Ref.1** (NSi peptide) | Ac-KQLR-kbt | 3,012 |
| **Ref.2** (Dabigatran) | - | 385,000 |
| **Ref.3** (Leupeptin) | - | 36,810 |
| **Ref.4** (Benzamidine) | - | >1,000,000 |
| **Ref.5** (Camostat mesylate) | - | 505.2 |
| **Ref.6** (PPack) | - | 189,600 |
| **Ref.9** (EK1) | - | 227.8 |
| **DMSO** | | >1,000,000 |

| | | |
|---|---|---|
| Ref. 2: Dabigatran - thrombin inhibitor Ref. 3: Leupeptin - naturally occruing inhibitor of Ser, Cys and Thr protease Ref. 4: Benzamidine - inhibitor of trypsin, trypsin-like enzyme and Ser protease Ref. 6: PPack: irreversible thrombin inhibitor Ref. 9: EK1 peptide: EK1 protein binds to SARS-COV2 spike protein | | |

### Example B-4. Inhibition of SARS-CoV2 WT infection

### Protocol:

- Seed 30 000 cells/well in 96 well plate
- Serially dilute TMPRSS2 inhibitors and controls in 1:5 steps from 500 to 0.0064 µM
- Add 20 µl of TMPRSS2 inhibitors to 60 µl cells
- Incubate for 2 h at 37 °C
- Infect with 20 µl of SARS-CoV2 at an MOI of 0.005
- Incubate for 48 h at 37 °C
- Check cells in microscope & measure ELISA

Virus isolate used: BetaCoV/France/IDF0372/2020

### Readout: In-cell ELISA

• Immunodetection of viral antigens in cells
• 48 h post infection
• Principle:
- primary CoV2-S Antibody detects Spike protein
- Secondary antibody (labelled) detects primary antibody
- Readout by measuring signal of secondary antibody

**Table 3. IC₅₀ values of the inventive compounds against SARS-COV2 WT infection**

| compound | sequence | IC₅₀ [nM] |
|---|---|---|
| **1** (RSi peptide)** | Ac-RQLR-kbt | 11,140 |
| **2** (P2i peptide) | Ac-AEQR-kbt | 18,970 |
| **3** (P3i peptide) | Ac-GQRR-kbt | 20,050 |
| **4** (P5i peptide) | Ac-QQVR-kbt | 58,480 |
| **6** (P8i peptide) | Ac-NEQR-kbt | 34,220 |
| **7** (P1i peptide) | Ac-RECR-kbt | - |
| **Ref.1** (NSi peptide) | Ac-KQLR-kbt | 15,570 |
| **Ref.2** (Dabigatran) | - | - |
| **Ref.3** (Leupeptin) | - | - |
| **Ref.4** (Benzamidine) | - | - |
| **Ref.5** (Camostat mesylate) | - | 32,400 |

• TMPRSS2 inhibitors are active against SARS-CoV2 WT
• IC₅₀s are higher in all instances, this could be caused by:
- High infection, leading to cell death and therefore lower signal in wells with little/ no inhibitor→ normalization to wells without inhibitor makes it look like infection is enhanced with higher conc of inhibitor before inhibition can be seen
- The higher number of cells seeded (3x more than in Pseudoparticle test)
• IC₅₀ of comopund 4(P5i) appears quite high, however last datapoint of the compound 4 at 10 µM has tremendous SD

## Claims

1. A compound of the general formula (I): 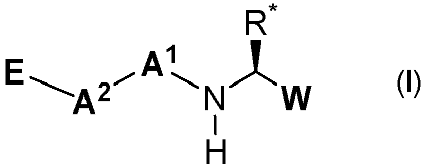 wherein
**W** represents -CN, -B(OH)₂, 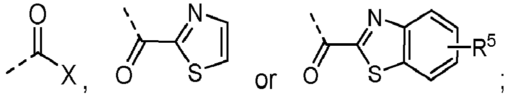
**X** represents -H, -CO-OR⁶, or -CO-NR⁷R⁸;
**R*** represents 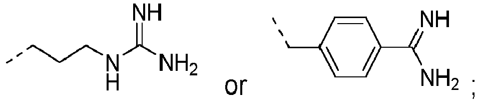
**E** represents -H, -R⁴, -CO**R⁴**, -**A³**-H, -**A³**-R⁴, or -**A³**-CO**R⁴**;
**A¹** represents 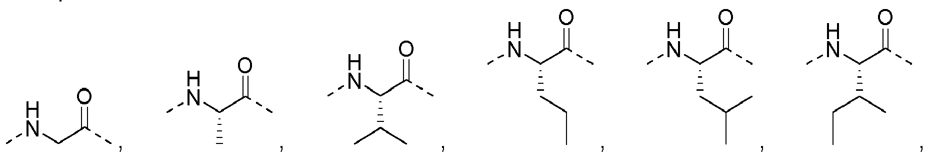 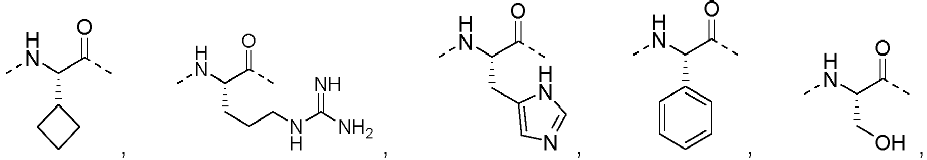 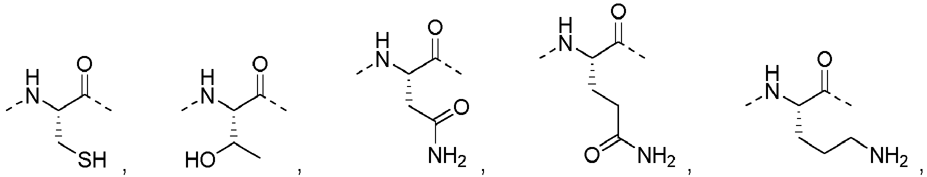 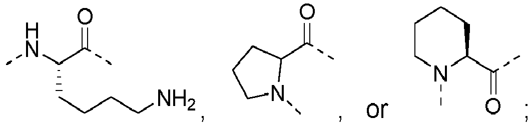
**A²** represents 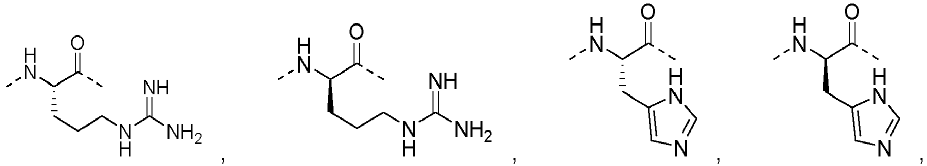 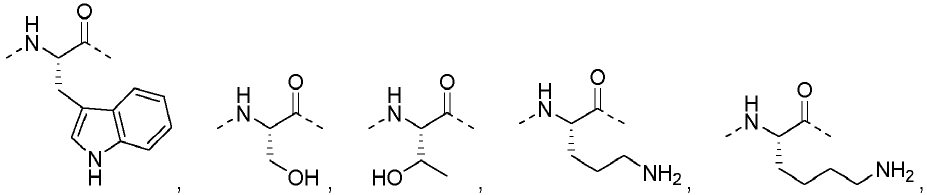 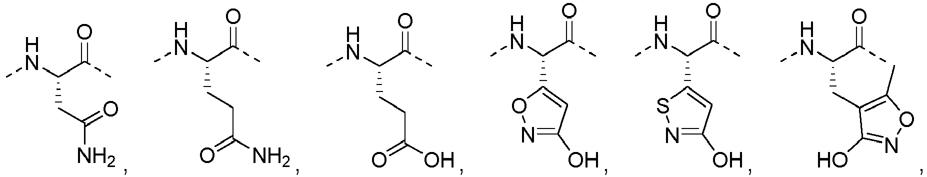 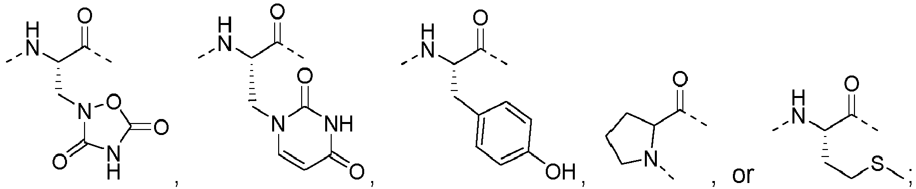
**A³** represents 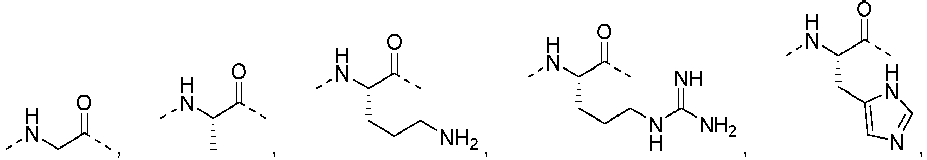 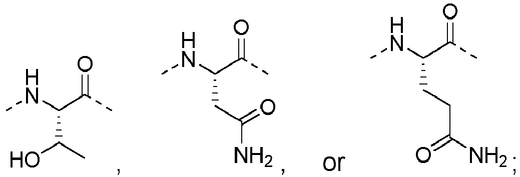
**R⁴** represent -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
**R⁶, R⁷,** and **R⁸** represent independently of each other -H, -CH₃, -CH₂CH₃, -CH₂CH₂CH₃, -CH(CH₃)₂, -C(CH₃)₃, or -cyclo-C₃H₅;
or a diastereomer, an enantiomer, a solvate, a hydrate, a prodrug or a pharmaceutically acceptable salt thereof.

2. The compound according to Claim 1 of the general formula (**II-1**): 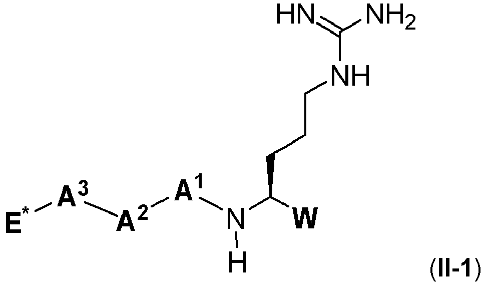 wherein
**W** represents -CN, -B(OH)₂, 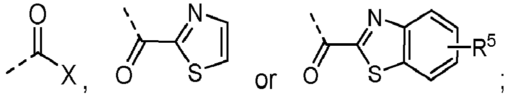
**X** represents -H, -CO-OR⁶, or -CO-NR⁷R⁸;
**E** represents -H, -R⁴, or -CO**R⁴**;
**A¹** represents 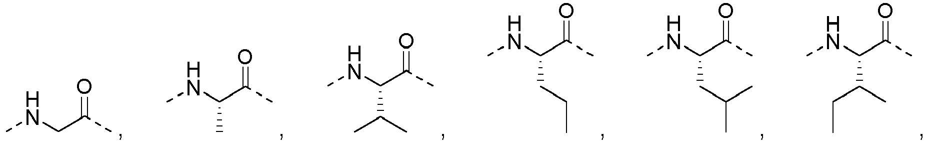 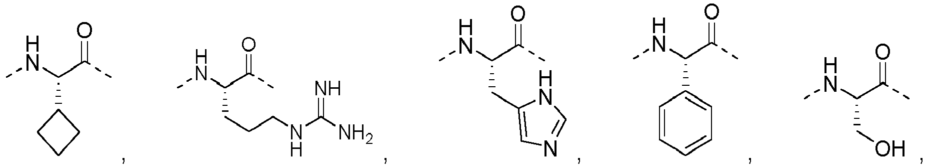 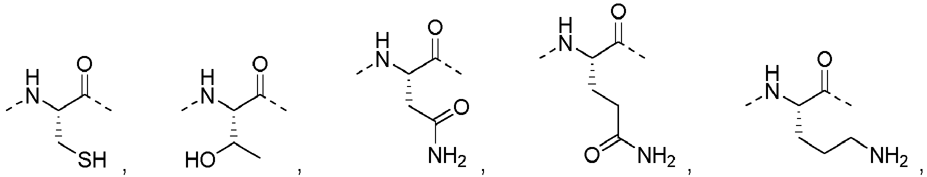 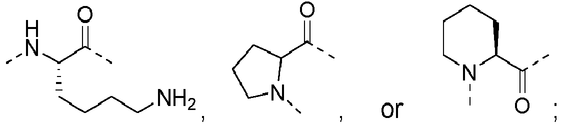
**A²** represents 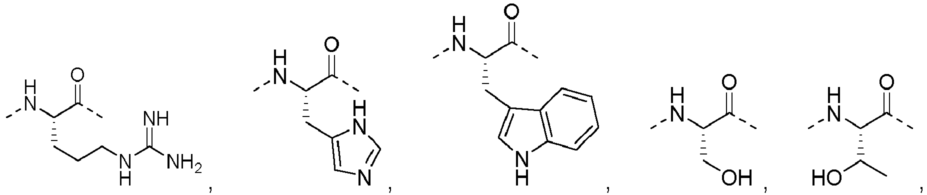 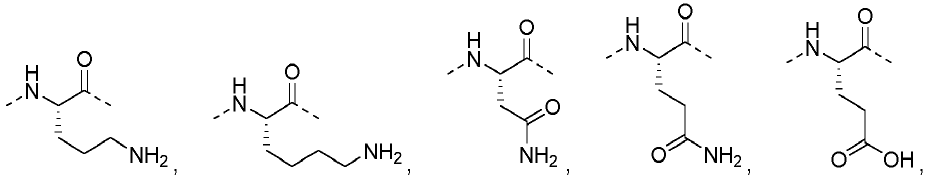 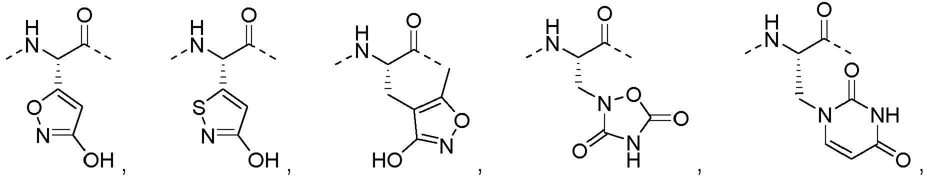 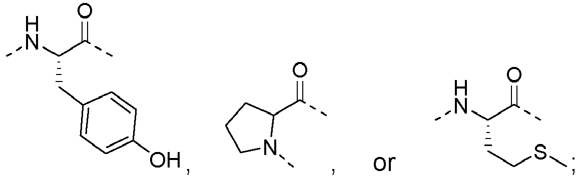
**A³** represents 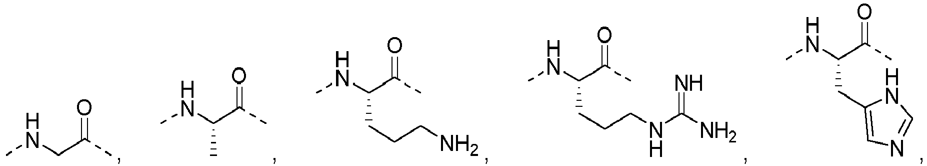 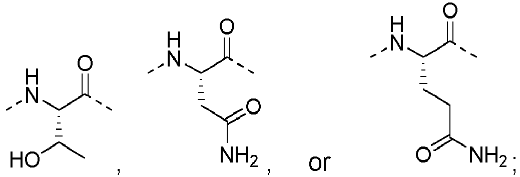
**R⁴** represent -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
or a diastereomer, an enantiomer, a solvate, a hydrate, a prodrug or a pharmaceutically acceptable salt thereof.

3. The compound according to Claim 1 of the formula (**II-2a**) or (**II-2b**): 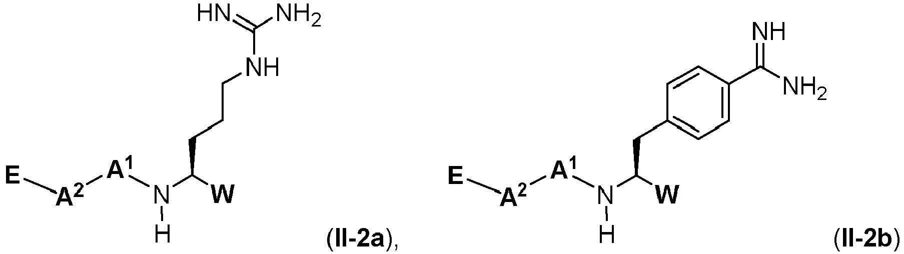 wherein
**A¹** represents 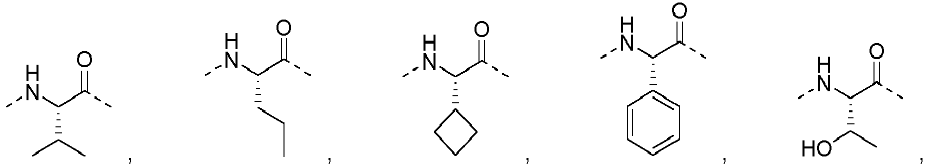 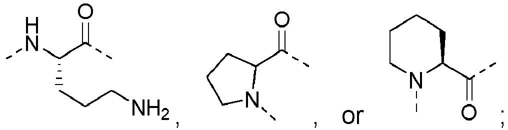
**A²** represents 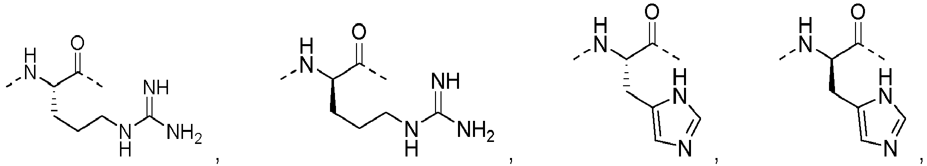 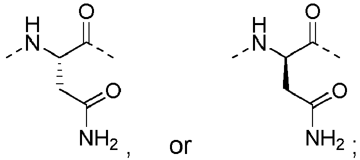
and **W** and **E** have the same meanings as defined in Claim 1.

4. The compound according to Claim 1 of any of the formulae (**III-1**) to (**III-5**): 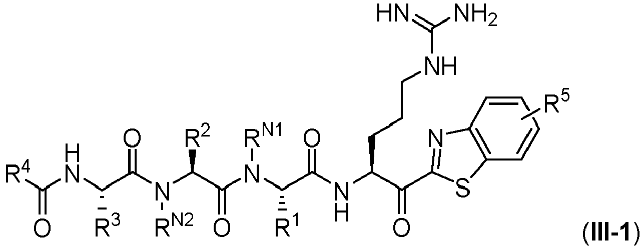 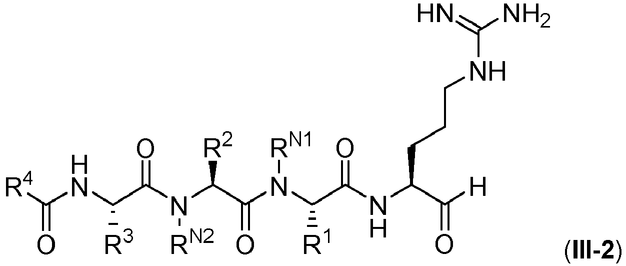 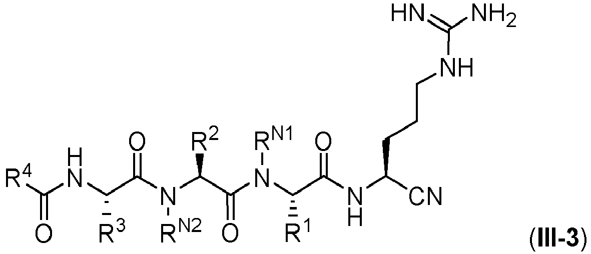 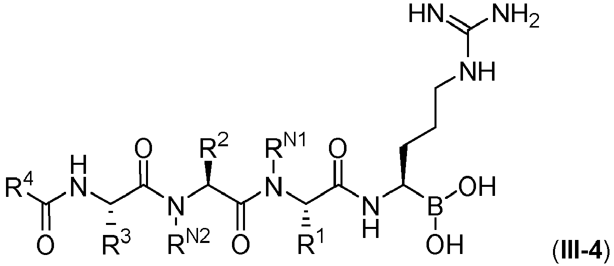 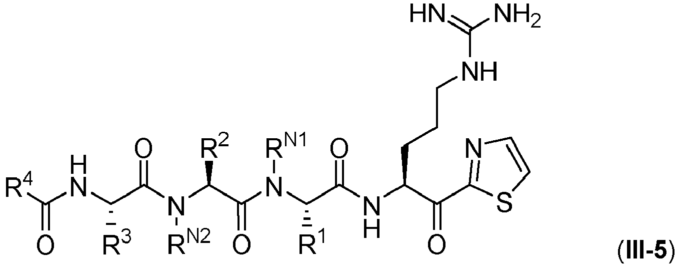 wherein
**R^{N1}** represents -H, or **R¹** and **R^{N1}** form -C₂H₄-;
**R^{N2}** represents -H, or **R²** and **R^{N2}** form -C₂H₄-;
**R¹** represents 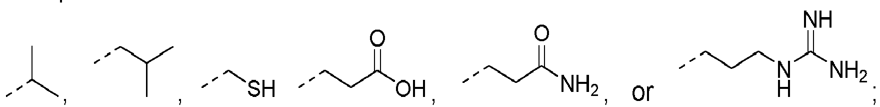
**R²** represents 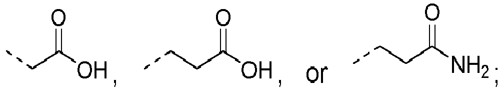
**R³** represents H, -CH₃, 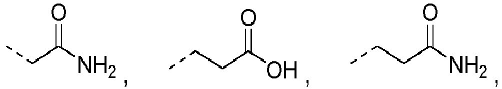 or
**R⁴** represents -CH₃, -CH₂CH₃, -CH₂CH₂CH₃, -CH(CH₃)₂, -O-C(CH₃)₃, or -cyclo-C₃H₅;
**R⁵** represents -H, -COOH, or -CONH₂;
or a solvate, a hydrate, a prodrug or a pharmaceutically acceptable salt thereof.

5. The compound according to any one of the Claims 1, 2 and 4 of any one of the formulae (**IV-1a**) - (**IV-1f**): 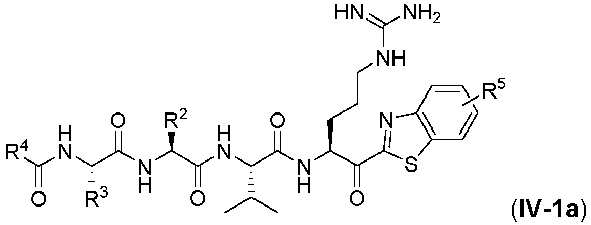 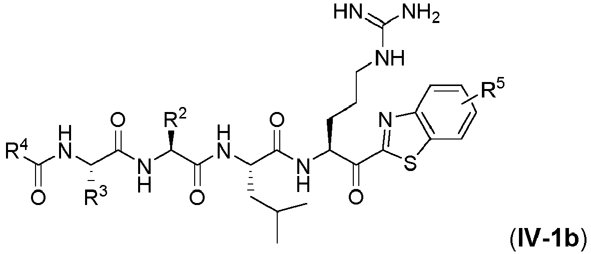 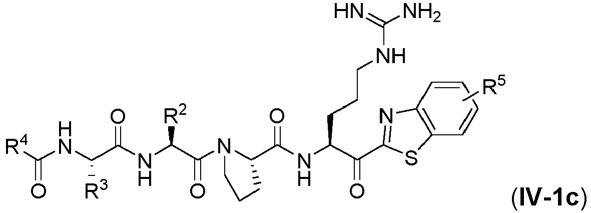 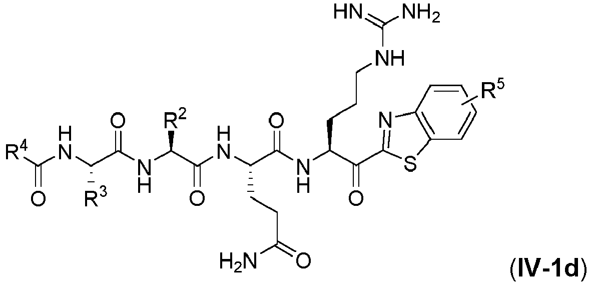 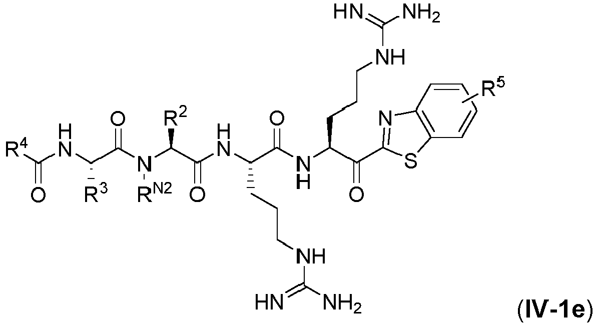 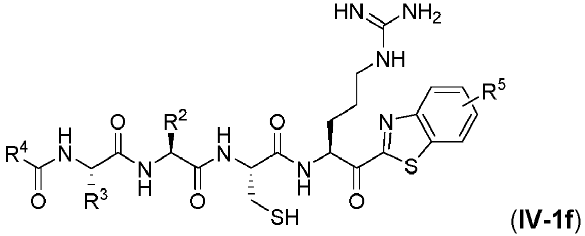 wherein
**R²** - **R⁵** and **R^{N2}** have the same meansings as defined in Claim 4.

6. The compound according to any one of the Claim 1, 2, and 4 of any one of the formulae (**V-1a**) - (**V-1b**): 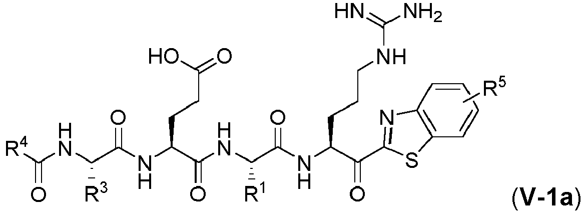 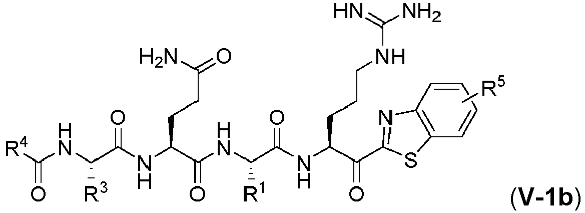 wherein
**R¹, R³, R⁴,** and **R⁵** have the same meanings as defined in Claim 4.

7. The compound according to any one of the Claims 1, 2, 4, 5, and 6 of any one of the formulae (**VI-1a**) - (**VI-1g**): 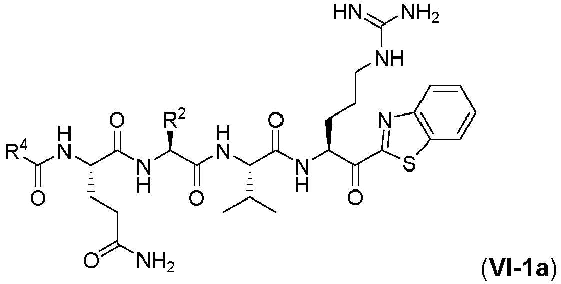 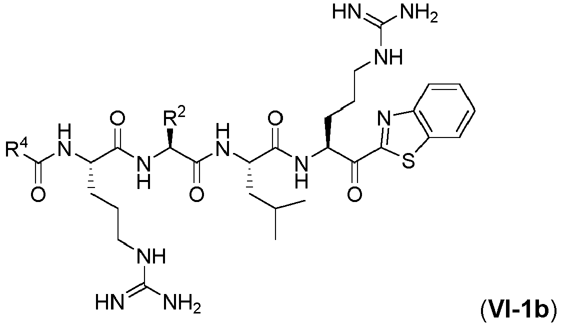 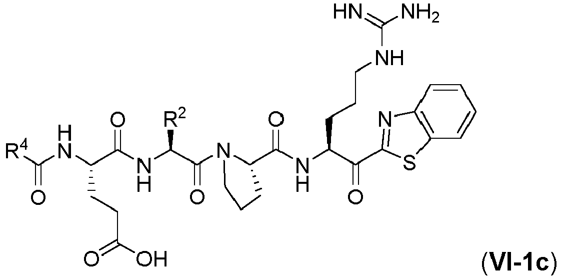 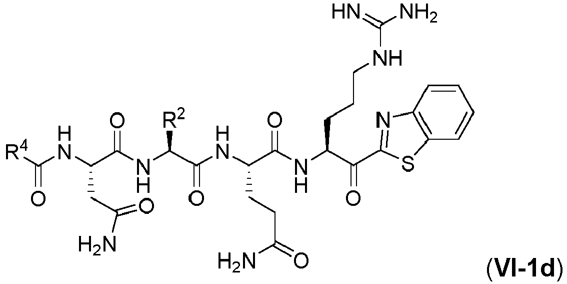 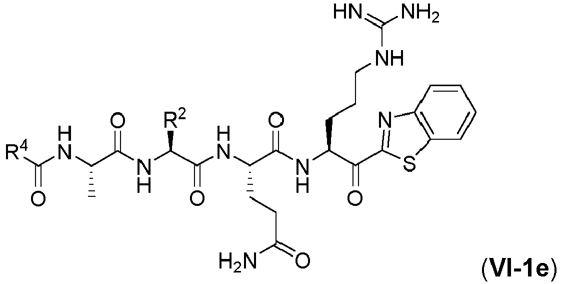 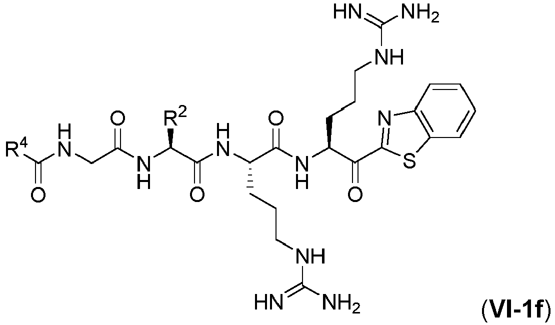 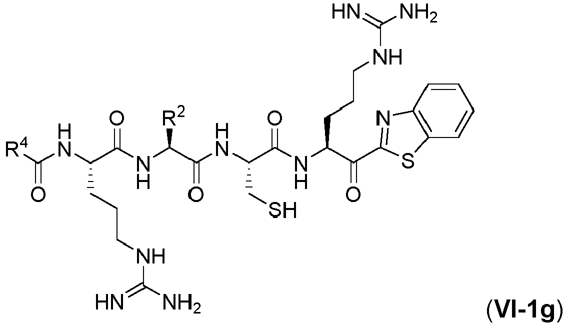 wherein **R²** and **R⁴** have the same meanings as defined in Claim 4.

8. The compound according to Claim 1 selected from the group consisting of:
**Compound 1:** 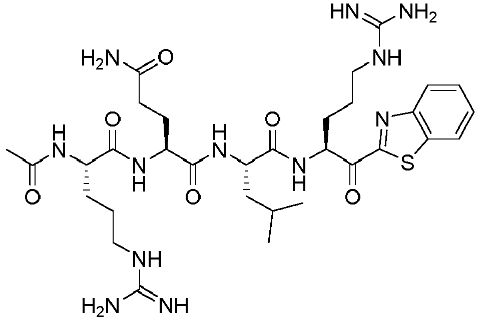
**Compound 2:** 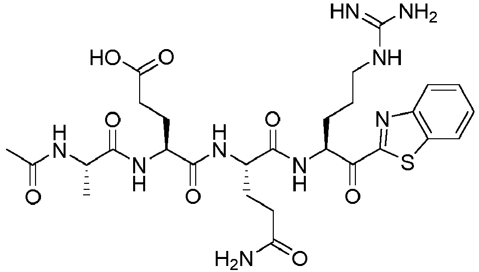
**Compound 3:** 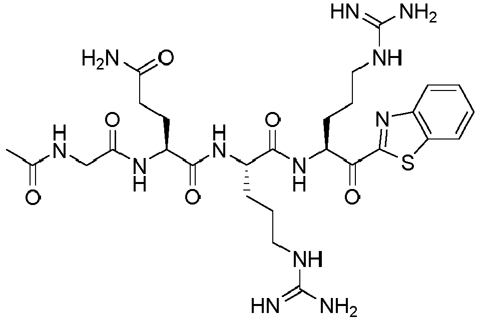
**Compound 4:** 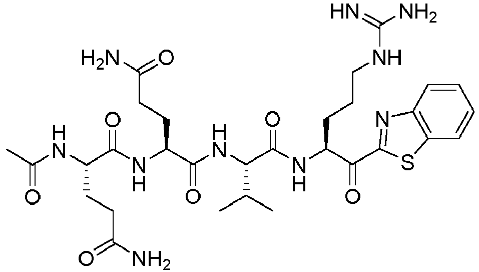
**Compound 5:** 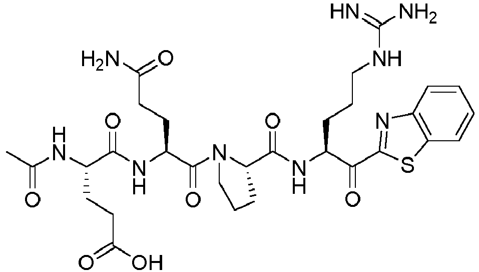
**Compound 6:** 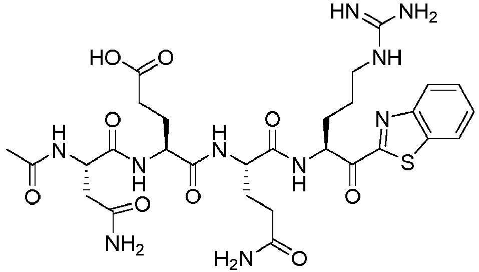
**Compound 7:** 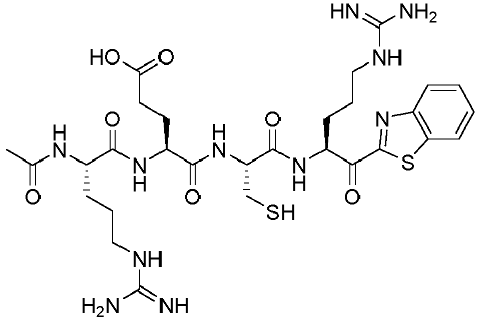
**Compound ST1:** 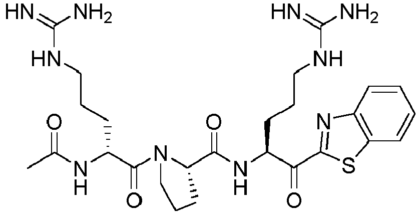
**Compound ST2:** 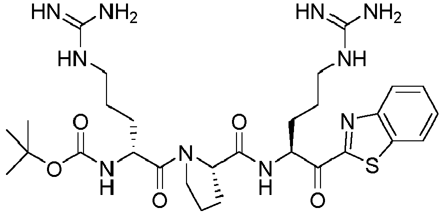
**Compound ST3:** 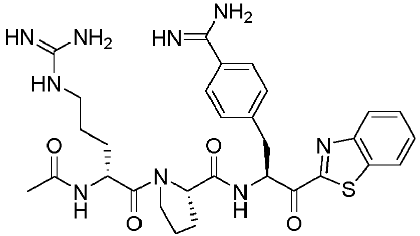
**Compound ST3b:** 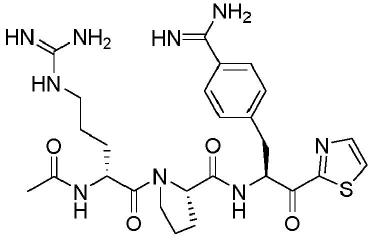
**Compound ST3c:** 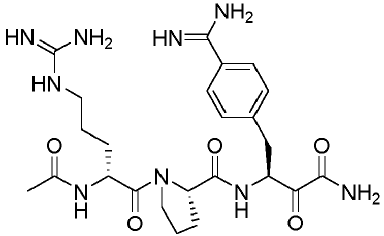
**Compound ST3d:** 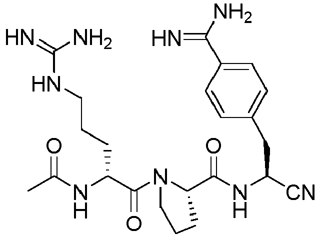
**Compound ST3e:** 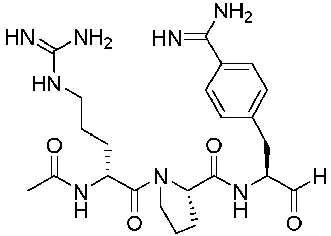
**Compound ST4:** 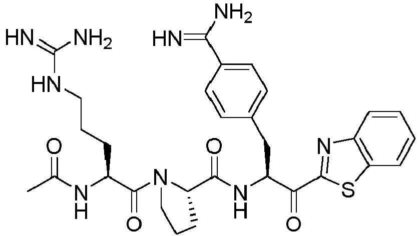
**Compound ST5:** 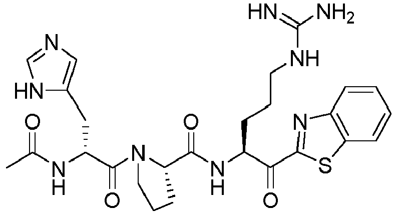
**Compound ST6:** 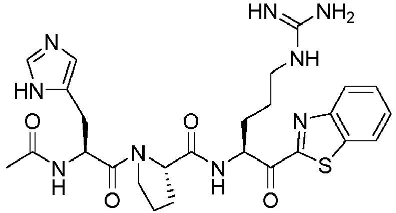
**Compound ST7:** 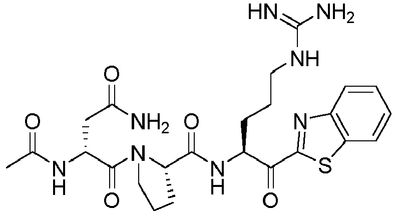
**Compound ST8:** 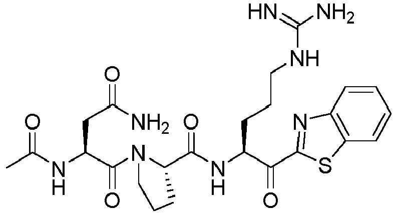
**Compound ST9:** 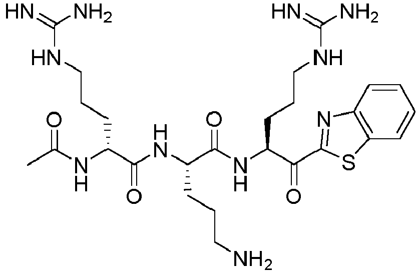
**Compound ST10:** 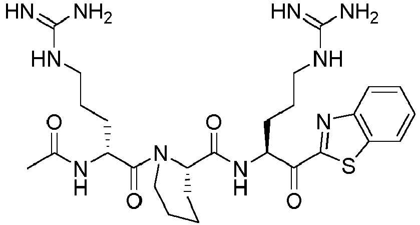
**Compound ST11:** 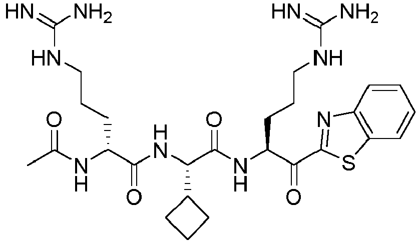
**Compound ST12:** 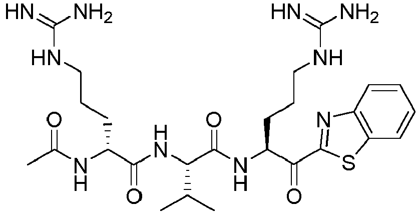
**Compound ST13:** 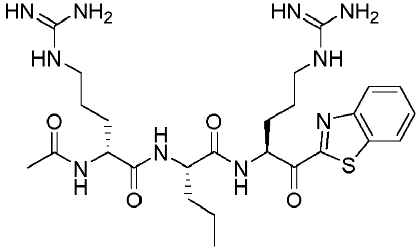
**Compound ST14:** 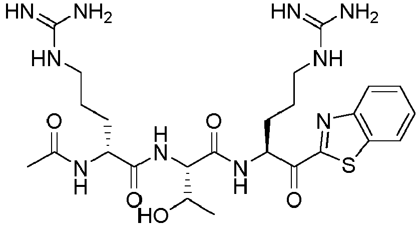
**Compound ST15:**

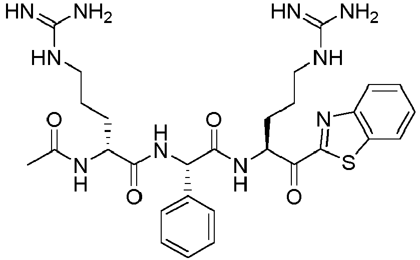

9. The compound according to any one of the Claims 1 - 8 for use in medicine or for use in the treatment or prophylaxis of virus infection .

10. The compound for use according to Claim 9, wherein the virus is influenza virus or coronavirus.

11. The compound for use according to Claim 10, wherein the virus is coronavirus 229E, SARS, MERS, or COVID-19.

12. The compound for use according to Claim 10 or 11 in combination with at least one active agent selected from antiviral agent, anti-HIV agent, anti-malarial agent, antibacterial agent and monoclonal antibodies.

13. Pharmaceutical composition containing at least one compound according to Claim 1 together with at least one pharmaceutically acceptable carrier, excipient and/or diluent.

14. The pharmaceutical composition according to Claim 13 in combination with at least one active agent selected from antiviral agent, anti-HIV agent, anti-malarial agent, antibacterial agent and monoclonal antibodies.

15. The pharmaceutical composition according to Claim 14, wherein the at least one active agent is selected from remdesivir, favipiravir (avigan), osteltamivir (tamiflu) lopinavir/ritonavir (Kaletra), darunavir, cobicistat, chlororquine, hydroxychloroquine, atovaquone, azithromycin, cararimycin, clindamycin.
